# EUROPEAN PATENT SPECIFICATION

(11) **EP 4 298 091 B1**
(45) Date of publication and mention of the grant of the patent: **26.08.2026**
(21) Application number: 22760217.4
(22) Date of filing: 15.02.2022
(51) Int. Cl.: C07D 401/14, C07D 403/14, C07D 413/14, C07D 417/14, A61P 13/12, A61K 31/137, A61K 31/4545, A61K 31/496, A61K 31/497, A61K 31/506

(54) **HETEROARYL DERIVATIVES AS APELIN RECEPTOR AGONISTS FOR THE TREATMENT OF ASTHMA**
HETEROARYLDERIVATE ALS APELINREZEPTORAGONISTEN ZUR BEHANDLUNG VON ASTHMA
DÉRIVÉS HÉTÉROARYLES UTILISÉS EN TANT QU'AGONISTES DU RÉCEPTEUR DE L'APÉLINE POUR LE TRAITEMENT D'ASTHME

(30) Priority: 25.02.2021 US 202163153822 P
(43) Date of publication of application: 03.01.2024
(73) Proprietor: Research Triangle Institute, Research Triangle Park, NC 27709 (US)
(72) Inventor: RUNYON, Scott P., Hillsborough, North Carolina 27278 (US); MAITRA, Rangan, Cary, North Carolina 27513 (US); NARAYANAN, Sanju, Morrisville, North Carolina 27560 (US)
(74) Representative: Cameron Intellectual Property Ltd
(86) International application number: PCT/US2022/016428
(87) International publication number: WO 2022/182547

(56) References cited:
- WO-A1-2015/188073
- WO-A1-2017/100558
- WO-A1-2018/071526
- WO-A1-2021/041791
- HUANG SHIFANG; CHEN LINXI; LU LIQUN; LI LANFANG: "The apelin–APJ axis: A novel potential therapeutic target for organ fibrosis", CLINICA CHIMICA ACTA, ELSEVIER BV, AMSTERDAM, NL, vol. 456, 2 March 2016 (2016-03-02), AMSTERDAM, NL , pages 81 - 88, XP029499592, ISSN: 0009-8981, DOI: 10.1016/j.cca.2016.02.025

## Description

### FIELD OF THE INVENTION

This disclosure relates generally to the discovery of agonists of the apelin receptor (APJ) and to such agonists for use use in methods of medical treatment.

### BACKGROUND

### Introduction: Apelin and the Apelin Receptor (APJ)

The apelin receptor (APJ) was cloned in 1993 as an orphan G-protein coupled receptor (GPCR). The human APJ gene is located on the long arm of chromosome 11 and encodes a 377 amino acid G protein-coupled receptor. The gene for APJ was designated angiotensin-receptor like 1 (AGTRL1) due to sequence similarities between the two receptors. Carpene et al., J Physiol Biochem. 2007; 63(4):359-373. However, none of the known peptidergic ligands for the angiotensin receptors, including angiotensin, activate APJ. APJ remained an orphan GPCR until 1998 when the peptide apelin was identified as its endogenous ligand. Lee et al., J Neurochem. 2000; 74(1):34-41; Habata et al., Biochim Biophys Acta. 1999; 1452(1):25-35.

Over the years, apelin and APJ have emerged as an important regulator of various physiological processes. Both apelin and APJ are expressed in the central nervous system (CNS) and peripherally in a number of tissues. Expression of APJ has been noted within the vasculature of some organs and is a potent regulator of related processes including angiogenesis and vasoconstriction. Cobellis *et al.* report increased of expression levels of both apelin and APJ receptor in preeclampsia-complicated pregnancies. Cobellis et al., Histol Histopathol. 2007; 22(1):1-8. APJ is also expressed in nonvascular cell types in heart, liver, and CNS where its primary role is currently under investigation. Medhurst et al., J Neurochem. 2003; 84(5):1162-1172. Apelin and APJ are often co-localized within the same organ suggesting an autocrine regulation of the receptor by its ligand. However, apelin has since been detected in blood suggesting that concomitant paracrine regulation of the receptor is also possible. The apelin-APJ system has been implicated as a regulator of various physiological functions and is believed to play an important role in thermoregulation, immunity, glucose metabolism, angiogenesis, fluid homeostasis, cardiac function, hepatic function and renal function. Ladeiras-Lopes et al., Arq Bras Cardiol. 2008; 90(5):343-349. APJ also acts as a co-receptor during HIV infection. O'Donnell et al., J Neurochem. 2007; 102(6):1905-1917; Zou et al., FEBS Lett. 2000; 473(1):15-18.

Expression of apelin and APJ are either up- or down-regulated in various pathophysiological conditions. In particular, the APJ appears to be an emerging target for the treatment of cardiovascular failure, idiopathic pulmonary fibrosis, cancer, angiopathies, pancreatitis, and as a prophylactic against HIV infection. In 2011 Andersen *et al.* reviewed apelin and APJ as an opportunity for therapeutic uses for pulmonary hypertension and pulmonary arterial hypertension (PAH). Andersen et al. Pulm. Circ. 2011; 1(3) 334-346.

Unfortunately, small molecule ligands of the APJ having suitable pharmacological properties are lacking. Few nonpeptide ligand systems has been reported to date. Iturrioz *et al.* report compounds that contain polycyclic fluorophores, such as lissamine, which make them ill-suited for pharmaceutical uses. Iturrioz et al., FASEB J. 2010; 24:1506-1517; EP 1903052 (Llorens-Cortes et al.). US Publ. Pat. Appn. 2014/0094450 (Hachtel et al.) discloses benzoimidazole-carboxylic acid amide derivatives as APJ receptor modulators.

Idiopathic Pulmonary Fibrosis ("IPF") is a chronic and progressive lung disease that results in respiratory failure and death. Median survival is about 2 to 4 years from diagnosis. The etiology of IPF remains unknown, but the disease is characterized by fibrotic interstitial infiltrates that are consistent with the histopathologic pattern of usual interstitial pneumonia. Reference is made to Gross TJ et al, NEngl J Med (2001), 345:(7}:517-525. As interstitial fibrosis advances with accompanying distortion of lung architecture, the lung becomes less compliant, increasing the effort associated with breathing, leading to dyspnea. Typically, lung function declines slowly over time, but some patients experience rapid declines that can lead to hospitalization or death, particularly in later stages of the disease. Reference is made to Martinez FJ et al. Ann Intern Med (2005), 142:963-967.

While the pathogenesis of IPF is not clearly defined, the disease is believed to be caused by repetitive cell injury. See, for example, Selman M et al., Ami Intern Med (2001), 134:136 51; and Selman. M. P c Am Thorac Soc (2006) (4):364-372. According to this hypothesis, initial injuries to the lungs are repaired but continuous injury and loss of basement membrane leads to irreversible loss of epithelial cells and chronic inflammation. Endothelial cells line the small airway capillaries and play an important role in preservation of airway architecture and integrity of basement membrane. Loss of endothelial cells or activation of these cells can lead to exaggerated inflammation and sustained injury to alveolar cells. Chronic cell injury and loss of basement membrane initiate a dysregulated wound healing response characterized by exaggerated deposition of extracellular matrix proteins and replacement of lost parenchymal cells with mesenchymal cells that leads to loss of lung function in susceptible individuals. See, Selman M et.al, (2001) supra; and Selman M. (2006) supra. Agents which may block endothelial cell injury or promote regeneration may present a novel treatment strategy for IPF patients.

The apelin receptor (APJ) was cloned in 1993 as an orphan G-protein coupled receptor (GPCR). The human APJ gene is located on the long arm of chromosome 11 and encodes a 377 amino acid G protein-coupled receptor. The gene for APJ was designated angiotensin-receptor like 1 (AGTRL1) due to sequence similarities between the two receptors. Carpene et al., J Physiol Biochem. 2007; 63(4):359-373. However, none of the known peptidergic ligands for the angiotensin receptors, including angiotensin, activate APJ. APJ remained an orphan GPCR until 1998 when the peptide apelin was identified as its endogenous ligand. Lee et al., J Neurochem. 2000; 74(1):34-41; Habata et al., Biochim Biophys Acta. 1999; 1452(1):25-35.

Over the years, apelin and APJ have emerged as an important regulator of various physiological processes. Both apelin and APJ are expressed in the central nervous system (CNS) and peripherally in a number of tissues. Expression of APJ has been noted within the vasculature of some organs and is a potent regulator of related processes including angiogenesis and vasoconstriction. Cobellis *et al.* report increased of expression levels of both apelin and APJ receptor in preeclampsia-complicated pregnancies. Cobellis et al., Histol Histopathol. 2007; 22(1):1-8. APJ is also expressed in nonvascular cell types in heart, liver, and CNS where its primary role is currently under investigation. Medhurst et al., J Neurochem. 2003; 84(5):1162-1172. Apelin and APJ are often co-localized within the same organ suggesting an autocrine regulation of the receptor by its ligand. However, apelin has since been detected in blood suggesting that concomitant paracrine regulation of the receptor is also possible. The apelin-APJ system has been implicated as a regulator of various physiological functions and is believed to play an important role in thermoregulation, immunity, glucose metabolism, angiogenesis, fluid homeostasis, cardiac function, hepatic function and renal function. Ladeiras-Lopes et al., Arq Bras Cardiol. 2008; 90(5):343-349. APJ also acts as a co-receptor during HIV infection. O'Donnell et al., J Neurochem. 2007; 102(6):1905-1917; Zou et al., FEBS Lett. 2000; 473(1):15-18.

Expression of apelin and APJ are either up- or down-regulated in various pathophysiological conditions. In particular, the APJ appears to be an emerging target for the treatment of cardiovascular failure, liver fibrosis, cancer, angiopathies, pancreatitis, and as a prophylactic against HIV infection. In 2011 Andersen *et al.* reviewed apelin and APJ as an opportunity for therapeutic uses for pulmonary hypertension and pulmonary arterial hypertension (PAH). Andersen et al. Pulm. Circ. 2011; 1(3) 334-346.

Compounds useful as APJ agonists are described in international patent applications PCT/US2015/034427, which published as WO 2015/188073, PCT/US2016/065808, which published as WO 2017/100558, and PCT/US2017/056117, which published as WO 2018/071526.

WO2018071526, WO2017100558, and WO2015188073 all disclose agonists of the apelin receptor (APJ) with heterocyclic cores and uses of such agonists.

Nevertheless, there continues to be a need for effective small molecule agonists of apelin.

### SUMMARY OF THE DISCLOSURE

The invention is defined by the appended claims.

One embodiment of the present disclosure includes a compound represented by the Formula I: 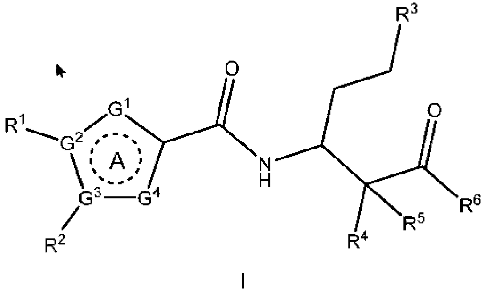
or a pharmaceutically acceptable salt thereof,
wherein
each of G¹, G², G³, and G⁴ is C, CH, or N, wherein:
   G¹ is CH, G² is C, G³ is N, and G⁴ is N;
   G¹ is CH, G² is N, G³ is C, and G⁴ is N;
   G¹ is N, G² is N, G³ is C, and G⁴ is CH; or
   G¹ is N, G² is N, G³ is C, and G⁴ is N; and
the dashed ring A is heteroaromatic;
R¹ is 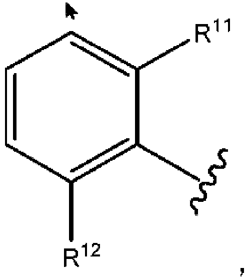
wherein
   R¹¹ is halogen, C₁₋₆ alkoxy, or C₁₋₆ haloalkyl;
   R¹² is H, halogen, C₁₋₆ alkoxy, or C₁₋₆ haloalkyl;
R² is a 5- to 7-membered heteroaromatic ring with one or more heteroatom selected from O, N, or S, wherein R² may be optionally substituted with one or more R²¹,
wherein each R²¹ is halogen, C₁₋₆ alkyl, C₁₋₆ alkoxy, or C₁₋₆ haloalkyl;
R³ is 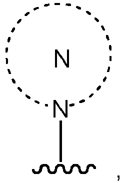
wherein
   ring B is a 5- or 6-membered heterocyclic ring, optionally containing one or more degrees of unsaturation, and substituted with one or more R³¹,
   wherein each R³¹ is halogen, C₁₋₆ alkyl, or oxo;
each of R⁴ and R⁵ is H, CH₃, or R⁴ and R⁵ combine with the atom to which they are attached to form a 3- to 6-membered cycloalkyl ring; and
R⁶ is OH or NH-R⁶¹, wherein R⁶¹ is a 3- to 6-membered cycloalkyl ring.

In one aspect, R¹¹ is C₁₋₆ alkoxy or C₁₋₆ haloalkyl. In one aspect, R¹¹ is methoxy or trifluoromethyl. In one aspect, R¹¹ is trifluoromethyl. In one aspect, R¹² is H, halogen, or C₁₋₆ alkoxy. In one aspect, R¹² is H, F, or methoxy. In one aspect, R¹² is H. In one aspect, R² is pyridine, pyrimidine, pyrazole, pyrazine, thiazole, thiophene, or oxazole. In one aspect, R² is thiazole. In one aspect, R² is unsubstituted. In one aspect, R²¹ is substituted with halogen or C₁₋₆ alkyl. In one aspect, R²¹ is F or methyl. In one aspect, ring B is piperidine, pyrrolidine, or azetidine. In one aspect, ring B is piperdine. In one aspect, ring B is substituted with two R³¹. In one aspect, each R³¹ is halogen. In one aspect, each R³¹ is F. In one aspect, each R³¹ is substituted from the same atom of R³. In one aspect, each of R⁴ and R⁵ is H. In one aspect, R⁶ is OH. In one aspect, G¹ is CH, G² is C, G³ is N, and G⁴ is N.

One embodiment of the present disclosure includes the use of a compound in a method of treating an apelin receptor related disorder the method comprising administering a therapeutically effective amount of a compound of the present disclosure, wherein the apelin receptor related disorder is selected from one or more of asthma, cardiomyopathy, diabetes, dyslipidemia, hypertension, inflammation, liver disease, metabolic disorder, neurodegenerative disease, obesity, preeclampsia, and renal dysfunction.

One embodiment of the present invention includes co-administration with one or more of an α-blocker, an angiotensin converting enzyme (ACE) inhibitor, an angiotensin-receptor blocker (ARB), a β-blocker, a calcium channel blocker, an immunosuppressant, a SGLT2 inhibitor, or a diuretic for the treatment of the apelin receptor (APJ) related disorder.

One embodiment of the present disclosure includes the use of a compound in a method for treating idiopathic pulmonary fibrosis in a patient in need thereof the method comprising administering a therapeutically effective amount of a compound of the present disclosure.

One embodiment of the present disclosure includes the use of a compound in a method for treating nephrotic syndrome in a patient in need thereof the method comprising administering a therapeutically effective amount of a compound of the present disclosure. In one aspect, the nephrotic syndrome is one or more of a glomerular disease or a chronic kidney disease.

One embodiment of the present disclosure includes the use of a compound in a method for promoting neovascularization through endothelial cell signaling or preservation of endothelial cell population in a patient in need thereof the method comprising administering a therapeutically effective amount of a compound of the present disclosure. In one aspect, capillary function is improved. In one aspect, receptor occupancy is prolonged. In one aspect, systemic circulation is prolonged.

In one aspect, the apelin receptor agonist is dosed as an aerosol, tablet, capsule, powder or liquid. In one aspect, the apelin receptor agonist is dosed systemically.

One embodiment of the present disclosure includes administration with one or more additional agent. In one aspect, the additional agent is one or more of pirfenidone, nintedanib, an immunosuppressant, an SGLT2 inhibitor, one or more corticosteroids, and one or more antibiotics.

In one aspect, the mean survival time of the patient is improved.

In one aspect, the compound is for use in a method to treat one or more of asthma, chronic obstructive pulmonary disease (COPD), bronchitis, emphysema, pulmonary edema, acute respiratory disease syndrome (ARDS), interstitial lung disease, sarcoidosis, co-morbid pulmonary disorder, an autoimmune condition, rheumatoid arthritis, and scleroderma.

One embodiment of the present disclosure includes the use of a compound in a method of preventing the progression of an apelin receptor related disorder the method comprising administering a therapeutically effective amount of a compound of the present disclosure, wherein the apelin receptor related disorder is selected from one or more of asthma, cardiomyopathy, diabetes, dyslipidemia, hypertension, inflammation, liver disease, metabolic disorder, neurodegenerative disease, obesity, preeclampsia, and renal dysfunction. In one aspect, the method includes co-administration with an α-blocker, an angiotensin converting enzyme (ACE) inhibitor, an angiotensin-receptor blocker (ARB), a β-blocker, a calcium channel blocker, an immunosuppressant, a SGLT2 inhibitor, or a diuretic for the treatment of the apelin receptor (APJ) related disorder.

One embodiment of the present disclosure includes the use of a compound in a method for preventing the progression of idiopathic pulmonary fibrosis in a patient in need thereof the method comprising administering a therapeutically effective amount of a compound of the present disclosure.

One embodiment of the present disclosure includes the use of a compound in a method for preventing the progression of nephrotic syndrome in a patient in need thereof the method comprising administering a therapeutically effective amount of a compound of the present disclosure.

In one aspect, the nephrotic syndrome is one or more of a glomerular disease or a chronic kidney disease. In one aspect, capillary function is improved. In one aspect, receptor occupancy is prolonged. In one aspect, systemic circulation is prolonged. In one aspect, the apelin receptor agonist is dosed as an aerosol, tablet, capsule, powder, or liquid. In one aspect, the apelin receptor agonist is dosed systemically.

One embodiment of the present disclosure includes co-administration with one or more additional agent. In one aspect, the additional agent is one or more of pirfenidone, nintedanib, an immunosuppressant, an SGLT2 inhibitor, one or more corticosteroids, and one or more antibiotics.

In one aspect, the mean survival time of the patient is improved.

In one aspect, the method is used to prevent the progression of one or more of asthma, chronic obstructive pulmonary disease (COPD), bronchitis, emphysema, pulmonary edema, acute respiratory disease syndrome (ARDS), interstitial lung disease, sarcoidosis, co-morbid pulmonary disorder, an autoimmune condition, rheumatoid arthritis, and scleroderma.

One embodiment of the present disclosure includes use of a compound of the present disclosure, in the preparation of a medicament for treating or preventing the progression of an apelin receptor related disorder, wherein the apelin receptor related disorder is selected from one or more of asthma, cardiomyopathy, diabetes, dyslipidemia, hypertension, inflammation, liver disease, metabolic disorder, neurodegenerative disease, obesity, preeclampsia, and renal dysfunction. In one aspect, the use further comprises co-administration with one or more of an α-blocker, an angiotensin converting enzyme (ACE) inhibitor, an angiotensin-receptor blocker (ARB), a β-blocker, a calcium channel blocker, an immunosuppressant, a SGLT2 inhibitor, or a diuretic for the treatment of the apelin receptor (APJ) related disorder.

One embodiment of the present disclosure includes use of a compound of the present disclosure, in the preparation of a medicament for treating or preventing the progression of idiopathic pulmonary fibrosis in a patient in need thereof.

One embodiment of the present disclosure includes use of a compound of the present disclosure, in the preparation of a medicament for treating or preventing the progression of nephrotic syndrome in a patient in need thereof. In one aspect, the nephrotic syndrome is one or more of a glomerular disease or a chronic kidney disease.

One embodiment of the present disclosure includes use of a compound of the present disclosure, in preparation of a medicament for promoting neovascularization through endothelial cell signaling or preservation of endothelial cell population in a patient in need thereof. In one aspect, capillary function is improved. In one aspect, receptor occupancy is prolonged. In one aspect, systemic circulation is prolonged.

In one aspect, the apelin receptor agonist is dosed as an aerosol, tablet, capsule, powder or liquid.

In one aspect, the apelin receptor agonist is dosed systemically.

One embodiment of the present disclosure includes co-administration with one or more additional agent. In one aspect, the additional agent is one or more of pirfenidone, nintedanib, an immunosuppressant, an SGLT2 inhibitor, one or more corticosteroids, and one or more antibiotics.

In one aspect, the mean survival time of the patient is improved.

In one aspect, the use of for one or more of asthma, chronic obstructive pulmonary disease (COPD), bronchitis, emphysema, pulmonary edema, acute respiratory disease syndrome (ARDS), interstitial lung disease, sarcoidosis, co-morbid pulmonary disorder, an autoimmune condition, rheumatoid arthritis, and scleroderma.

One embodiment of the present disclosure includes a compound of the present disclosure, for use in the preparation of a medicament for treating or preventing the progression of an apelin receptor related disorder, wherein the apelin receptor related disorder is selected from one or more of asthma, cardiomyopathy, diabetes, dyslipidemia, hypertension, inflammation, liver disease, metabolic disorder, neurodegenerative disease, obesity, preeclampsia, and renal dysfunction.

One embodiment of the present disclosure includes co-administration with one or more of an α-blocker, an angiotensin converting enzyme (ACE) inhibitor, an angiotensin-receptor blocker (ARB), a β-blocker, a calcium channel blocker, an immunosuppressant, a SGLT2 inhibitor, or a diuretic for the treatment of the apelin receptor (APJ) related disorder.

One embodiment of the present disclosure includes a compound of the present disclosure, for use in the preparation of a medicament for treating or preventing the progression of idiopathic pulmonary fibrosis in a patient in need thereof.

One embodiment of the present disclosure includes a compound of the present disclosure, for use in the preparation of a medicament for treating or preventing the progression of nephrotic syndrome in a patient in need thereof. In one aspect, the nephrotic syndrome is one or more of a glomerular disease or a chronic kidney disease.

One embodiment of the present disclosure includes a compound of the present disclosure, for use in preparation of a medicament for promoting neovascularization through endothelial cell signaling or preservation of endothelial cell population in a patient in need thereof. In one aspect, capillary function is improved. In one aspect, receptor occupancy is prolonged. In one aspect, systemic circulation is prolonged.

In one aspect, the apelin receptor agonist is dosed as an aerosol, tablet, capsule, powder or liquid.

In one aspect, the apelin receptor agonist is dosed systemically.

One embodiment of the present disclosure includes co-administration with one or more additional agent.

In one aspect, the additional agent is one or more of pirfenidone, nintedanib, an immunosuppressant, an SGLT2 inhibitor, one or more corticosteroids, and one or more antibiotics.

In one aspect, the mean survival time of the patient is improved.

In one aspect, the compound is used to treat one or more of asthma, chronic obstructive pulmonary disease (COPD), bronchitis, emphysema, pulmonary edema, acute respiratory disease syndrome (ARDS), interstitial lung disease, sarcoidosis, co-morbid pulmonary disorder, an autoimmune condition, rheumatoid arthritis, and scleroderma.

One embodiment of the present disclosure includes a compound of any one of the examples for use in the treatment or prevention of progression of an apelin receptor related disorder.

One embodiment of the present disclosure includes the use of a compound in a method of treating or reducing or preventing the progression of one or more disease or disorder associated with the apelinergic (apelin-APJ) system signaling path in endothelial cells the method comprising administering a compound of the present disclosure.

One embodiment of the present disclosure includes use of a compound of the present disclosure in a method for treating or preventing the progression of one or more disease or disorder associated with the apelinergic system signaling path in endothelial cells.

In one aspect, the compound promotes protection and regeneration of endothelial cells.

In one aspect, the disease or disorder is an infection of corona virus or corona viridae family.

### BRIEF DESCRIPTION OF THE DRAWINGS

FIG 1A-1B are a graphical illustration of day 5 treatment grouping body weight data, wherein BLEO-instilled mice placed to Vehicle or Test Compound (EXAMPLE 22) treatment groups had body weight and change body weight that were not different on Day 5.
FIG 2A-2C are a graphical illustration that BLEO instillation did not statistically affect serial BW relative to Vehicle-instilled controls. Neither Tx (A) nor Px (B) test compound (EXAMPLE 22) nor PIRF (C) affected serial BW relative to Vehicle-treated BLEO-instilled controls.
FIG 3 is a graphical illustration of Survival Data, wherein of the BLEO-instilled mice, the 21-day survival rates were 83% (Vehicle), 100% (60 mpk Tx EXAMPLE 22), 91% (15 and 30 mpk Px EXAMPLE 22), 92% (60 mpk Px EXAMPLE 22), and 94% (PIRF).

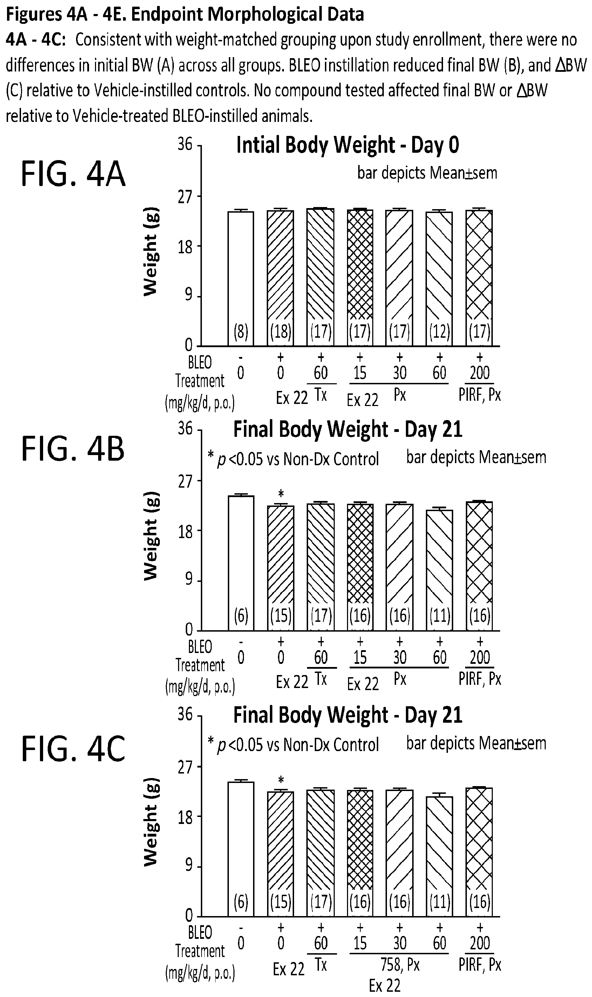

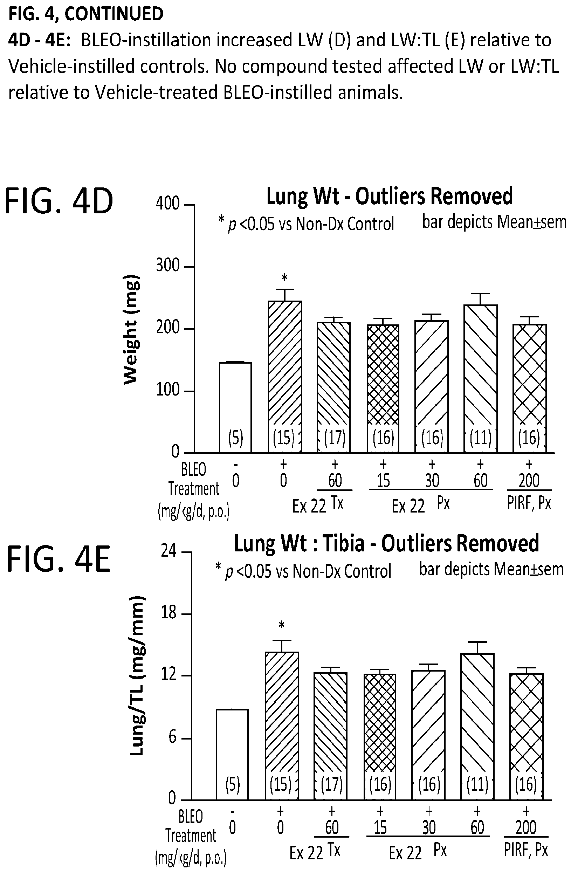

FIG 4 is a set of graphical illustrations. Figure 4A - 4E illustrate Endpoint Morphological Data, Consistent with weight-matched grouping upon study enrollment, there were no differences in initial BW (A) across all groups. BLEO instillation reduced final BW (B), and ΔBW (C) relative to Vehicle-instilled controls. No compound tested affected final BW or ΔBW relative to Vehicle-treated BLEO-instilled animals. Figure 4D - 4E illustrate BLEO-instillation increased LW (D) and LW:TL (E) relative to Vehicle-instilled controls. No compound tested affected LW or LW:TL relative to Vehicle-treated BLEO-instilled animals.
FIG 5 is a graphical illustration of Endpoint Lung OH-P data, BLEO instillation increased total lung OH-P content relative to Vehicle-instilled controls. No compound tested affected total lung OH-P relative to Vehicle-treated, BLEO-instilled controls.
FIG 6 is a set of representative images of MTB-Stained slides, where representative images obtained at 200x depicting MTB staining, demonstrating parenchymal collagen deposition (staining).
FIG 7 is a set of graphical illustrations for Endpoint Lung CVF and TCF data. Figures 7A - 7B illustrate BLEO-instilled animals had increased composite CVF (A) and TCF (B) relative to Vehicle-instilled controls. Tx PIRF reduced CVF and TCF and 15 mpk/day Px EXAMPLE 22 reduced TCF relative to Vehicle-treated BLEO instilled controls. 7C - 7E illustrate CVF data from anatomically distinct lung sections: Caudal (C), Medial (D), and Rostral (E). 7F - 7H illustrate TCF data from anatomically distinct lung sections: Caudal (F), Medial (G), and Rostral (H).
FIG 8 is a graphical illustration of selected parameters related to cardiac function at 4 weeks post-treatment.
FIG 9 is a graphical illustration demonstrating that test compound (EXAMPLE 22) improves ejection fraction post-MI.
FIG 10a is a graphical illustration of EXAMPLE 22 plasma concentration following oral administration at 5 mg/kg to make SD rats.
FIG 10b is a graphical illustration of the EXAMPLE 22 pharmacokinetic profile following a single iv administration at 2 mg/kg to make SD rats.

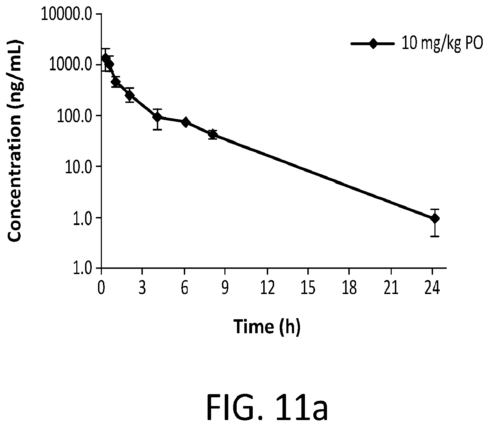

FIG 11a is a graphical illustration of the EXAMPLE 22 plasma concentration following oral administration at 10 mg/kg to male C57BL/6 Mice

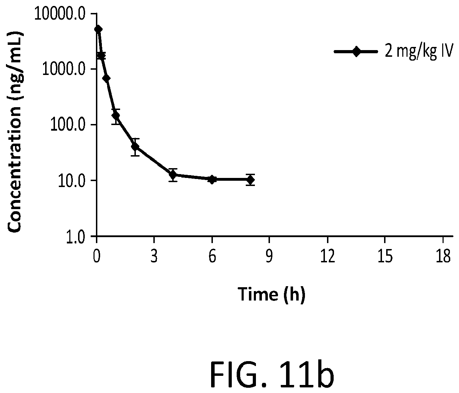

FIG 11b is a graphical illustration of EXAMPLE 22 plasma concentration following iv administration at 2 mg/kg to male C57BL/6 Mice.

### DETAILED DESCRIPTION OF THE DISCLOSURE

### Definitions

"Alkenyl" refers to an unsaturated branched, straight-chain or cyclic alkyl group having at least one carbon-carbon double bond derived by the removal of one hydrogen atom from a single carbon atom of a parent alkene. The group may be in either the Z- and E-forms (or cis or trans conformation) about the double bond(s). Typical alkenyl groups include, but are not limited to, ethenyl; propenyls such as prop-1-en-1-yl, prop-1-en-2-yl, prop-2-en-I-yl (allyl), prop-2-en-2-yl, cycloprop-1-en-1-yl; cycloprop-2-en-1-yl; butenyls such as but-1-en-1-yl, but-1-en-2-yl, 2-methyl-prop-1-en-1-yl, but-2-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl; and the like. The alkenyl group may be substituted or unsubstituted. In certain embodiments, an alkenyl group has from 2 to 20 carbon atoms and in other embodiments from 2 to 8 carbon atoms.

"Alkoxy" refers to a radical -OR where R represents an alkyl, , cycloalkyl, aryl, or heteroaryl group as defined herein. Representative examples include, but are not limited to, methoxy, ethoxy, propoxy, butoxy, cyclohexyloxy, and the like. The alkoxy group may be substituted or unsubstituted.

"Alkyl" refers to a saturated, branched or straight-chain monovalent hydrocarbon group derived by the removal of one hydrogen atom from a single carbon atom of a parent alkane. Typical alkyl groups include, but are not limited to, methyl, ethyl, propyls such as propan-1-yl, propan-2-yl, and cyclopropan-1-yl, butyls such as butan-1-yl, butan-2-yl, 2-methyl-propan-1-yl, 2-methyl-propan-2-yl, cyclobutan-1-yl, tert-butyl, and the like. The alkyl group may be substituted or unsubstituted; for example, with methyl or a halogen(s) such as difluoro or trifluoro. In certain embodiments, an alkyl group comprises from 1 to 20 carbon atoms. Alternatively, an alkyl group may comprise from 1 to 8 carbon atoms.

"Alkyl(aryl)" refers to an acyclic alkyl group in which one of the hydrogen atoms bonded to a carbon atom, typically a terminal or sp³ carbon atom, is replaced with an aryl group. Typical alkyl(aryl) groups include, but are not limited to, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like. In certain embodiments, an alkyl(aryl) group can be (C₆₋₂₀) alkyl(aryl) e.g., the alkyl group may be (C₁₋₁₀) and the aryl moiety may be (C₅₋₁₀). The alkyl(aryl) group may be substituted or unsubstituted.

"Alkynyl" refers to an unsaturated branched or straight-chain having at least one carbon-carbon triple bond derived by the removal of one hydrogen atom from a single carbon atom of a parent alkyne. Typical alkynyl groups include, but are not limited to, ethynyl, propynyl, butenyl, 2-pentynyl, 3-pentynyl, 2-hexynyl, 3-hexynyl and the like. The alkynyl group may be substituted or unsubstituted. In certain embodiments, an alkynyl group has from 3 to 20 carbon atoms and in other embodiments from 3 to 8 carbon atoms.

"Aryl" refers to a monovalent aromatic hydrocarbon group derived by the removal of one hydrogen atom from a single carbon atom of a parent aromatic ring system. Aryl encompasses 5- and 6-membered carbocyclic aromatic rings, for example, benzene; bicyclic ring systems wherein at least one ring is carbocyclic and aromatic, for example, naphthalene, indane; or two aromatic ring systems, for example benzyl phenyl, biphenyl, diphenylethane, diphenylmethane. The aryl group may be substituted or unsubstituted, for example with a halogen, such as fluorine.

"Cycloalkyl" refers to a saturated or unsaturated cyclic alkyl group. Where a specific level of saturation is intended, the nomenclature "cycloalkanyl" or "cycloalkenyl" is used. Typical cycloalkyl groups include, but are not limited to, groups derived from cyclopropane, cyclobutane, cyclopentane, cyclohexane, and the like. The cycloalkyl group may be substituted or unsubstituted. In certain embodiments, the cycloalkyl group can be C₃₋₁₀ cycloalkyl, such as, for example, C₆ cycloalkyl or cC₆H₁₂. The cycloalkyl group may also be a bridged bicyclic cycloalkyl group, a fused cycloalkyl group or a spiro cycloalkyl group. Non-limiting examples of bridged bicyclic cycloalkyl groups are bicyclo[2.2.1]heptane, bicyclo[2.2.1]hexane, bicycle[2.2.2]octane. An example of a fused cycloalkyl group is bicyclo[4.4.0]decane or decalin. Non-limiting examples of spiro cycloalkyl groups are spiro [3.3] heptane, spiro [4.3] octane, or spiro [5.4] decane.

"Disease" refers to any disease, disorder, condition, symptom, or indication.

"Halogen" refers to a fluoro, chloro, bromo, or iodo group.

"Heteroaryl" refers to a monovalent heteroaromatic group derived by the removal of one hydrogen atom from a single atom of a parent heteroaromatic ring system. Heteroaryl encompasses: 5- to 7-membered aromatic, monocyclic rings containing one or more, for example, from 1 to 4, or in certain embodiments, from 1 to 3, heteroatoms chosen from N, O, and S, with the remaining ring atoms being carbon; and polycyclic heterocycloalkyl rings containing one or more, for example, from 1 to 4, or in certain embodiments, from 1 to 3, heteroatoms chosen from N, O, and S, with the remaining ring atoms being carbon and wherein at least one heteroatom is present in an aromatic ring. The heteroaryl group may be substituted or unsubstituted.

For example, heteroaryl includes a 5- to 7-membered heteroaromatic ring fused to a 5- to 7-membered cycloalkyl ring and a 5- to 7-membered heteroaromatic ring fused to a 5- to 7-membered heterocycloalkyl ring. For such fused, bicyclic heteroaryl ring systems wherein only one of the rings contains one or more heteroatoms, the point of attachment may be at the heteroaromatic ring or the cycloalkyl ring. When the total number of S and O atoms in the heteroaryl group exceeds 1, those heteroatoms are not adjacent to one another. In certain embodiments, the total number of S and O atoms in the heteroaryl group is not more than 2. In certain embodiments, the total number of S and O atoms in the aromatic heterocycle is not more than 1. Typical heteroaryl groups include, but are not limited to, groups derived from acridine, arsindole, carbazole, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, piperidine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, and the like. In certain embodiments, the heteroaryl group can be between 5 to 20 membered heteroaryl, such as, for example, a 5 to 10 membered heteroaryl. In certain embodiments, heteroaryl groups can be those derived from thiophene, pyrrole, benzothiophene, benzofuran, indole, pyridine, quinoline, imidazole, oxazole, and pyrazine.

"Heterocycloalkyl" refers to a non-aromatic monocyclic ring or fused non-aromatic polycyclic rings with one or more heteroatom(s) independently selected from N, S and O, with the remaining ring atoms being carbon and wherein at least one heteroatom is present in each non-aromatic ring. The heterocycle group may be a three-member ring, a four member ring, a five member ring, a six member ring or a seven member ring. In certain embodiments, the heterocycloalkyl group is 1,4-dioxane, 1,3-dioxolane, 1,4-dithiane, imidazolidine, morpholine, piperidine, piperidone, piperazine, pyrolidone, pyrrolidine, or 1,3,5-trithiane. It may contain an imide. The heterocycloalkyl group may be bicyclic such as an heterospiro group, e.g., heterospiro [3.3] heptanyl, heterospiro [3.4] octanyl, or heterospiro [5.5] undecanyls. The heterocycloalkyl group may be substituted or unsubstituted. Thus, heterocycloalkyl group encompasses heterocycloalkyl groups substituted with one or more halogens, such as 3,3-difluoropiperidine, or 4,4-difluoropiperidine. In addition, the heterocycloalkyl group may be substituted with a C₁-C₄ alkyl or C₁-C₄ halo alkyl group such as a -CF₃ group.

"Pharmaceutically acceptable" refers to generally recognized for use in animals, and more particularly in humans.

"Pharmaceutically acceptable salt" refers to a salt of a compound that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, and the like; or (2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, N-methylglucamine, dicyclohexylamine, and the like.

"Pharmaceutically acceptable excipient," "pharmaceutically acceptable carrier," or "pharmaceutically acceptable adjuvant" refer, respectively, to an excipient, carrier or adjuvant with which at least one compound of the present disclosure is administered. "Pharmaceutically acceptable vehicle" refers to any of a diluent, adjuvant, excipient or carrier with which at least one compound of the present disclosure is administered.

"Prodrug" refers to a precursor or derivative form of a pharmaceutically active substance that is less bioactive compared to the parent drug and is capable of being enzymatically activated or converted into the more active parent form. Prodrug forms of the compounds described herein may designed to improve bioavailability or stability or reduce toxicity. For example, compounds of the invention having free amino, amido, carboxylic, hydroxyl, or thiol groups can be converted into prodrugs. See Rautio et al., 2008 Nat Rev Drug Dis 7 255-270. For instance, free carboxyl groups can be derivatized as amides, carbamates, esters, or N-Mannich bases. Free hydroxy groups may be derivatized using groups including but not limited to carbonates, dimethylaminoacetates, ethers, hemisuccinates, phosphate esters, and phosphoryloxymethyloxycarbonyls, as outlined in Fleisher et al., 1996 Advanced Drug Delivery Reviews 19, 115-130. Carbamate prodrugs of hydroxy and amino groups are also included, as are carbonate prodrugs, sulfonate esters and sulfate esters of hydroxy groups. Derivatization of hydroxy groups as (acyloxy)methyl and (acyloxy)ethyl ethers wherein the acyl group may be an alkyl ester, optionally substituted with groups including but not limited to ether, amine and carboxylic acid functionalities, or where the acyl group is an amino acid ester as described above, are also encompassed. Prodrugs of this type are described in Robinson et al., 1996 J Med Chem 39 10-18. Free amines can also be derivatized as amides, carbamates, imines, N-Mannich bases, oximes, phosphonamides, or sulfonamides. Carbonyls may be derivatized to imine or oxime prodrugs. Thiols may be derivatized as esters or ethers. Prodrugs may also include compounds wherein an amino acid residue, or a polypeptide chain of two or more (e.g., two, three or four) amino acid residues is covalently joined through an amide or ester bond to a free amino, hydroxy or carboxylic acid group of compounds of the invention. The amino acid residues include but are not limited to the 20 naturally occurring amino acids commonly designated by three letter symbols and also includes beta-alanine, citrulline, demosine, gamma-aminobutyric acid, homocysteine, homoserine, 4-hydroxyproline, hydroxylysine, isodemosine, 3-methylhistidine, norvalin, methionine sulfone, and ornithine.

"Stereoisomer" refers to an isomer that differs in the arrangement of the constituent atoms in space. Stereoisomers that are mirror images of each other and optically active are termed "enantiomers," and stereoisomers that are not mirror images of one another and are optically active are termed "diastereoisomers."

"Subject" includes mammals and humans. The terms "human" and "subject" are used interchangeably herein.

"Substituted" refers to a group in which one or more hydrogen atoms are each independently replaced with the same or different substituent(s). Typical substituents include, but are not limited to, CN, NO₂, OH, oxo, C₁-C₆ alkoxy, OC₁-C₆ haloalkyl, SC₁-C₆ alkyl, SC₁-C₆ haloalkyl, halogen, C₁-C₆ alkyl, C₁-C₆ haloalkyl, CO₂H, NH₂, NH(C₁-C₃ alkyl), N(C₁₋₃ alkyl)₂, SO₂H, NHSO₂C₁-C₃ alkyl, SO₂NH₂, SO₂C₁-C₃ alkyl, NHC(O)(C₁-C₃ alkyl), and C₃ - C₆ cycloalkyl.

"Therapeutically effective amount" refers to the amount of a compound that, when administered to a subject for treating a disease, or at least one of the clinical symptoms of a disease or disorder, is sufficient to affect such treatment for the disease, disorder, or symptom. The "therapeutically effective amount" can vary depending on the compound, the disease, disorder, and/or symptoms of the disease or disorder, severity of the disease, disorder, and/or symptoms of the disease or disorder, the age of the subject to be treated, and/or the weight of the subject to be treated. An appropriate amount in any given instance can be readily apparent to those skilled in the art or capable of determination by routine experimentation.

"Treating" or "treatment" of any disease or disorder refers to arresting or ameliorating a disease, disorder, or at least one of the clinical symptoms of a disease or disorder, reducing the risk of acquiring a disease, disorder, or at least one of the clinical symptoms of a disease or disorder, reducing the development of a disease, disorder or at least one of the clinical symptoms of the disease or disorder, or reducing the risk of developing a disease or disorder or at least one of the clinical symptoms of a disease or disorder. "Treating" or "treatment" also refers to inhibiting the disease or disorder, either physically, (e.g., stabilization of a discernible symptom), physiologically, (e.g., stabilization of a physical parameter), or both, or inhibiting at least one physical parameter which may not be discernible to the subject. Further, "treating" or "treatment" refers to delaying the onset of the disease or disorder or at least symptoms thereof in a subject which may be exposed to or predisposed to a disease or disorder even though that subject does not yet experience or display symptoms of the disease or disorder.

Pairs of the functional groups defined herein may be combined in a chemically rational way. For example, C₁-C₈ alkyl amino means the functional group C₁-C₈ alkyl, e.g., -nC₅H₁₁, is combined with the functional group, amino, e.g., -NH₂ to form in this example -nC₅H₁₀NH₂. Likewise, C₁-C₈ alkyl alcohol would mean a group, e.g., nC₃H₆OH. Similarly, C₁-C₈ alkoxy aryl means the functional group C₁-C₈ alkoxy, e.g., -CH₂CH₂OCH₂CH₃ or -OCH₂CH₃ combined with an aryl group, e.g., -C₆H₅F to form -CH₂CH₂OCH₂CH₂-C₆H₅F or -OCH₂CH₃-C₆H₅F, respectively.

As used herein, reference to an amino acid will include single α, β, γ, δ amino acids, or their corresponding side chains, such as the twenty naturally occurring amino acids, e.g., alanine (Ala/A); arginine (Arg/R); asparagine (Asn/N); aspartic acid (Asp/D); cysteine (Cys/C); glutamic acid (Glu/E); glutamine (Gln/Q); glycine (Gly/G); histidine (His/H); isoleucine (Ile/I); leucine (Leu/L); lysine (Lys/K); methionine (Met/M); phenylalanine (Phe/F); proline (Pro/P); Serine (Ser/S); threonine (Thr/T); tryptophan (Trp/W); tyrosine (Tyr/Y); and valine (Val/V). The individual amino acids may of either the R or the S chirality. Alternatively, two or three amino acids may linked by a peptide bond, or may be dipeptides or tripeptides (Hobbs et al., Proc Nat Acad Sci USA. 1993, 90, 6909-6913); US Pat. Nos. 6,075,121 (Bartlett et al.) peptoids; or vinylogous polypeptides (Hagihara et al., J Amer Chem Soc. 1992, 114, 6568). The groups may be part of the extended unnatural amino acids, e.g., Xie and Schultz, Nat Rev Mol Cell Biol. 2006, 7(10):775-82 or Wang et al., Chem Biol. 2009, 16(3):323-36.

### Deuterated and other isotopic variants

The invention also includes all suitable isotopic variations of a compound of the invention. An isotopic variation of a compound of the invention is defined as one in which at least one atom is replaced by an atom having the same atomic number but an atomic mass different from the atomic mass usually or predominantly found in nature. Examples of isotopes that can be incorporated into a compound of the invention include isotopes of hydrogen, carbon, nitrogen, oxygen, phosphorus, sulfur, fluorine, chlorine, bromine and iodine, such as ²H (deuterium), ³H (tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I and ¹³¹I, respectively. Certain isotopic variations of a compound of the invention, for example, those in which one or more radioactive isotopes such as ³H or ¹⁴C are incorporated, are useful in drug and/or substrate tissue distribution studies. Tritiated and carbon-14, i.e., ¹⁴C, isotopes are particularly preferred for their ease of preparation and detectability. Substitution with positron emitting isotopes, such as ¹¹C, ¹⁸F, ¹⁵O and ¹³N, can be useful in Positron Emission Topography (PET) studies.

Further, substitution with isotopes such as deuterium may afford certain therapeutic advantages resulting from greater metabolic stability, for example, increased in vivo half-life or reduced dosage requirements and hence may be preferred in some circumstances. Isotopic variations of a compound of the invention can generally be prepared by conventional procedures known by a person skilled in the art such as by the illustrative methods or by the preparations described in the examples hereafter using appropriate isotopic variations of suitable reagents. In another embodiment, the isotope-labeled compounds contain deuterium (²H), tritium (³H) or¹⁴C isotopes. Isotope-labeled compounds of this invention can be prepared by the general methods well known to persons having ordinary skill in the art.

Such isotope-labeled compounds can be conveniently prepared by carrying out the procedures disclosed in the Examples disclosed herein and Schemes by substituting a readily available isotope-labeled reagent for a non-labeled reagent. In some instances, compounds may be treated with isotope-labeled reagents to exchange a normal atom with its isotope, for example, hydrogen for deuterium can be exchanged by the action of a deuterated acid such as D₂SO₄/D₂O. Alternatively, deuterium may be also incorporated into a compound using methods such as through reduction such as using LiAlD₄ or NaBD₃, catalytic hydrogenation or acidic or basic isotopic exchange using appropriate deuterated reagents such as deuterides, D₂ and D₂O. In addition to the above, PCT publications, WO2014/169280; WO2015/058067; U.S. Pat. Nos. 8,354,557; 8,704,001 and US Patent Application Publication Nos.; 2010/0331540; 2014/0081019; 2014/0341994; 2015/0299166.

### PHARMACEUTICAL COMPOSITIONS

The disclosure also provides pharmaceutical compositions comprising an effective amount of a compound Formula I (e.g., any of the formulae and/or structures disclosed herein), or a pharmaceutically acceptable salt of said compound; and a pharmaceutically acceptable carrier.

Pharmaceutically acceptable carriers, adjuvants and vehicles that may be used in the pharmaceutical compositions of this disclosure include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. If required, the solubility and bioavailability of the compounds of the present disclosure in pharmaceutical compositions may be enhanced by methods well-known in the art. One method includes the use of lipid excipients in the formulation. See "Oral Lipid-Based Formulations: Enhancing the Bioavailability of Poorly Water-Soluble Drugs (Drugs and the Pharmaceutical Sciences)," David J. Hauss, ed. Informa Healthcare, 2007; and "Role of Lipid Excipients in Modifying Oral and Parenteral Drug Delivery: Basic Principles and Biological Examples," Kishor M. Wasan, ed. Wiley-Interscience, 2006.

Another known method of enhancing bioavailability is the use of an amorphous form of a compound of this disclosure optionally formulated with a poloxamer, such as LUTROL^{™} and PLURONIC^{™} (BASF Corporation), or block copolymers of ethylene oxide and propylene oxide. See US Pat. No. 7,014,866 (Infeld et al.); and US Pat. Pubs. 20060094744 (Maryanoff et al.) and 20060079502 (Lang).

The pharmaceutical compositions of the disclosure include those suitable for oral, rectal, nasal, topical (including buccal and sublingual), pulmonary, vaginal or parenteral (including subcutaneous, intramuscular, intravenous and intradermal) administration. In certain embodiments, the compound of the formulae herein is administered transdermally (e.g., using a transdermal patch or iontophoretic techniques). Other formulations may conveniently be presented in unit dosage form, e.g., tablets, sustained release capsules, and in liposomes, and may be prepared by any methods well known in the art of pharmacy. See, for example, Remington's Pharmaceutical Sciences, Mack Publishing Company, Philadelphia, PA (17th ed. 1985).

Such preparative methods include the step of bringing into association with the molecule to be administered ingredients such as the carrier that constitutes one or more accessory ingredients. In general, the compositions are prepared by uniformly and intimately bringing into association the active ingredients with liquid carriers, liposomes or finely divided solid carriers, or both, and then, if necessary, shaping the product. In certain embodiments, the compound is administered orally. Compositions of the present disclosure suitable for oral administration may be presented as discrete units such as capsules, sachets, or tablets each containing a predetermined amount of the active ingredient; a powder or granules; a solution or a suspension in an aqueous liquid or a non-aqueous liquid; an oil-in-water liquid emulsion; a water-in-oil liquid emulsion; packed in liposomes; or as a bolus, etc. Soft gelatin capsules can be useful for containing such suspensions, which may beneficially increase the rate of compound absorption.

In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are administered orally, the active ingredient is combined with emulsifying and suspending agents. If desired, certain sweetening and/or flavoring and/or coloring agents may be added.

Compositions suitable for oral administration include lozenges comprising the ingredients in a flavored basis, usually sucrose and acacia or tragacanth; and pastilles comprising the active ingredient in an inert basis such as gelatin and glycerin, or sucrose and acacia.

Compositions suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

Such injection solutions may be in the form, for example, of a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant.

The pharmaceutical compositions of this disclosure may be administered in the form of suppositories for rectal administration. These compositions can be prepared by mixing a compound of this disclosure with a suitable non-irritating excipient which is solid at room temperature but liquid at the rectal temperature and therefore will melt in the rectum to release the active components. Such materials include, but are not limited to, cocoa butter, beeswax and polyethylene glycols.

The pharmaceutical compositions of this disclosure may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. See, e.g., US Pat. No. 6,803,031 (Rabinowitz & Zaffaroni).

Topical administration of the pharmaceutical compositions of this disclosure is especially useful when the desired treatment involves areas or organs readily accessible by topical application. For topical application topically to the skin, the pharmaceutical composition should be formulated with a suitable ointment containing the active components suspended or dissolved in a carrier. Carriers for topical administration of the compounds of this disclosure include, but are not limited to, mineral oil, liquid petroleum, white petroleum, propylene glycol, polyoxyethylene or polyoxypropylene compounds, emulsifying wax, and water. Alternatively, the pharmaceutical composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol, and water. The pharmaceutical compositions of this disclosure may also be topically applied to the lower intestinal tract by rectal suppository formulation or in a suitable enema formulation. Topically-transdermal patches and iontophoretic administration are also included in this disclosure.

Application of the therapeutics may be local, so as to be administered at the site of interest. Various techniques can be used for providing the compositions at the site of interest, such as injection, use of catheters, trocars, projectiles, pluronic gels, stents, sustained drug release polymers or other devices which provide for internal access. Thus, according to yet another embodiment, the compounds of this disclosure may be incorporated into compositions for coating an implantable medical device, such as prostheses, artificial valves, vascular grafts, stents, or catheters. Suitable coatings and the general preparation of coated implantable devices are known in the art and are exemplified in US Pat. Nos. 6,099,562 (Ding & Helmus); 5,886,026 (Hunter et al.); and 5,304,121 (Sahatjian). The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccharides, polyethylene glycol, phospholipids or combinations thereof to impart controlled release characteristics in the composition. Coatings for invasive devices are to be included within the definition of pharmaceutically acceptable carrier, adjuvant or vehicle, as those terms are used herein.

According to another embodiment, the disclosure provides a method of coating an implantable medical device comprising the step of contacting said device with the coating composition described above. It will be obvious to those skilled in the art that the coating of the device will occur prior to implantation into a mammal.

According to another embodiment, the disclosure provides a method of impregnating an implantable drug release device comprising the step of contacting said drug release device with a compound or composition of this disclosure. Implantable drug release devices include, but are not limited to, biodegradable polymer capsules or bullets, non-degradable, diffusible polymer capsules and biodegradable polymer wafers.

According to another embodiment, the disclosure provides an implantable medical device coated with a compound or a composition comprising a compound of this disclosure, such that said compound is therapeutically active.

According to another embodiment, the disclosure provides an implantable drug release device impregnated with or containing a compound or a composition comprising a compound of this disclosure, such that said compound is released from said device and is therapeutically active. Where an organ or tissue is accessible because of removal from the subject, such organ or tissue may be bathed in a medium containing a composition of this disclosure, a composition of this disclosure may be painted onto the organ, or a composition of this disclosure may be applied in any other convenient way.

In one embodiment, this disclosure provides a composition comprising a compound of Formula I, or more specific compounds disclosed herein, to treat or prevent asthma, atherosclerosis, cancer, cardiomyopathy, diabetes, dyslipidemia, HIV neurodegeneration, hypertension, inflammation, liver disease, metabolic disorder, neurodegenerative disease, obesity, or preeclampsia. In another embodiment, the disclosure provides a composition comprising a compound of Formula I, or more specific compounds disclosed herein, to treat or prevent cancer, cell proliferation, diabetes, fluid homeostasis, heart diseases (e.g., hypertension and heart failure, such as congestive heart failure), HIV infection, immune function, obesity, stem cell trafficking, metastatic cancer or a vein-related disorder such as an angioma, a venous insufficiency, a stasis, or a thrombosis.

One embodiment of the present disclosure includes a composition comprising a compound of Formula I for treating idiopathic pulmonary fibrosis in a patient in need thereof comprising administering a therapeutically effective amount of an apelin receptor agonist.

One embodiment of the present disclosure includes a composition for promoting neovascularization or preservation of capillary architecture through endothelial cell signaling in a patient in need thereof comprising administering a therapeutically effective amount of an apelin receptor agonist, such as a compound of Formula I. Alternatively, the present disclosure includes a method for preservation of an endothelial cell population in a patient in need thereof comprising administering a therapeutically effective amount of an apelin receptor agonist.

One aspect of an embodiment of the present disclosure includes wherein capillary function is improved.

One aspect of an embodiment of the present disclosure includes wherein receptor occupancy is prolonged.

One aspect of an embodiment of the present disclosure includes wherein the apelin receptor agonist is dosed as an aerosol.

One aspect of an embodiment of the present disclosure includes wherein the apelin receptor agonist is dosed systemically.

One aspect of an embodiment of the present disclosure includes wherein the mean survival time of the patient is improved.

One aspect of any one of the embodiments and aspects of the present disclosure includes where the method of the present disclosure is used to treat one or more of asthma, chronic obstructive pulmonary disease (COPD), bronchitis, emphysema, pulmonary edema, acute respiratory disease syndrome (ARDS), interstitial lung disease, sarcoidosis, co-morbid pulmonary disorder, an autoimmune condition, rheumatoid arthritis, and scleroderma.

In another embodiment, a composition of this disclosure further comprises a second therapeutic agent. In one embodiment, the second therapeutic agent is one or more additional compounds of the disclosure. In another embodiment, the second therapeutic agent may be selected from any compound or therapeutic agent known to have or that demonstrates advantageous properties when administered with a compound having the same mechanism of action as the APJ receptor compound of Formula I.

In a particular embodiment, the second therapeutic is an agent useful in the treatment or prevention of a disease or condition selected from acute decompensated heart failure (ADHF), amyotrophic lateral sclerosis, arrhythmia, asthma, atherosclerosis, atherosclerosis, atrial fibrillation, Brugada syndrome, burn injuries (including sunburn), cancer, cardiac fibrosis, cardiomyopathy, cerebrovascular accidents, chronic heart failure, diabetes (including gestational diabetes), dyslipidemia, HIV neurodegeneration, hypertension, inflammation, ischemic cardiovascular diseases, liver disease, metabolic disorder, neurodegenerative disease, obesity, peripheral arterial disease, preeclampsia, pulmonary hypertension, restenosis, transient ischemic attacks, traumatic brain injuries, ventricular tachycardia, or water retention. In another embodiment, the second therapeutic is an agent useful in the treatment or prevention of a disease or condition selected from cancer, cell proliferation, diabetes, fluid homeostasis, heart diseases (e.g., hypertension and heart failure, such as congestive heart failure), HIV infection, immune function, obesity, stem cell trafficking, or metastatic cancer. One aspect of an embodiment of the present disclosure includes wherein the additional agent is one or more of pirfenidone, nintedanib, one or more corticosteroids, and one or more antibiotics.

For example, when the disease or condition is idiopathic pulmonary fibrosis, asthma, chronic obstructive pulmonary disease (COPD), bronchitis, emphysema, pulmonary edema, acute respiratory disease syndrome (ARDS), interstitial lung disease, sarcoidosis, co-morbid pulmonary disorder, an autoimmune condition, rheumatoid arthritis, or scleroderma, the second therapeutic agent may be selected from one or more of pirfenidone, nintedanib, one or more corticosteroids, and one or more antibiotics.

For example, when the disease or condition is congestive heart failure, the second therapeutic agent can be selected from: ACE inhibitors, beta blockers, vasodilators, calcium channel blockers, loop diuretics, aldosterone antagonists, and angiotensin receptor blockers.

When the disease or condition being treated is hypertension, the second therapeutic agent can be selected from: α-blockers, β-blockers, calcium channel blockers, diuretics, natriuretics, saluretics, centrally acting antihypertensives, angiotensin converting enzyme (ACE) inhibitors, dual ACE and neutral endopeptidase (NEP) inhibitors, angiotensin-receptor blockers (ARBs), aldosterone synthase inhibitors, aldosterone-receptor antagonists, or endothelin receptor antagonists.

Non-limiting examples of α-Blockers include doxazosin, prazosin, tamsulosin, and terazosin.

Non-limiting examples of β-Blockers for combination therapy are selected from acebutolol, acetutolol, atenolol, bisoprol, bupranolol, carteolol, carvedilol, celiprolol, esmolol, mepindolol, metoprolol, nadolol, oxprenolol, penbutolol, pindolol, propanolol, taliprolol, and their pharmaceutically acceptable salts.

Non-limiting examples of calcium channel blockers include dihydropyridines (DHPs) and non-DHPs. The preferred DHPs are selected from the group consisting of amlodipine, felodipine, isradipine, lacidipine, nicardipine, nifedipine, nigulpidine, niludipine, nimodiphine, nisoldipine, nitrendipine, nivaldipine, ryosidine, and their pharmaceutically acceptable salts. Non-DHPs are selected from anipamil, diltiazem, fendiline, flunarizine, gallopamil, mibefradil, prenylamine, tiapamil, and verampimil and their pharmaceutically acceptable salts.

Non-limiting examples of thiazide derivative include amiloride, chlorothalidon, chlorothiazide, hydrochlorothiazide, and methylchlorothiazide.

Non-limiting examples of centrally acting antiphypertensives include clonidine, guanabenz, guanfacine and methyldopa.

Non-limiting examples of ACE inhibitors include alacepril, benazepril, benazaprilat, captopril, ceronapril, cilazapril, delapril, enalapril, enalaprilat, fosinopril, lisinopril, moexipiril, moveltopril, perindopril, quinapril, quinaprilat, ramipril, ramiprilat, spirapril, temocapril, trandolapril, and zofenopril. Preferred ACE inhibitors are benazepril, enalpril, lisinopril, and ramipril.

Non-limiting examples of dual ACE/NEP inhibitors are, for example, omapatrilat, fasidotril, and fasidotrilat.

Non-limiting examples of preferred ARBs include candesartan, eprosartan, irbesartan, losartan, olmesartan, tasosartan, telmisartan, and valsartan.

Non-limiting examples of preferred aldosterone synthase inhibitors are anastrozole, fadrozole, and exemestane.

Non-limiting examples of preferred aldosterone-receptor antagonists are spironolactone and eplerenone.

Non-limiting examples of preferred endothelin antagonist include, for example, bosentan, enrasentan, atrasentan, darusentan, sitaxentan, and tezosentan and their pharmaceutically acceptable salts.

In one embodiment, the disclosure provides separate dosage forms of a compound of this disclosure and one or more of any of the above-described second therapeutic agents, wherein the compound and second therapeutic agent are associated with one another. The term "associated with one another" as used herein means that the separate dosage forms are packaged together or otherwise attached to one another such that it is readily apparent that the separate dosage forms are intended to be sold and administered together (within less than 24 hours of one another, consecutively or simultaneously).

In the pharmaceutical compositions of the disclosure, the compound of the present disclosure is present in an effective amount. As used herein, the term "effective amount" refers to an amount which, when administered in a proper dosing regimen, is sufficient to treat (therapeutically or prophylactically) the target disorder. For example, and effective amount is sufficient to reduce or ameliorate the severity, duration or progression of the disorder being treated, prevent the advancement of the disorder being treated, cause the regression of the disorder being treated, or enhance or improve the prophylactic or therapeutic effect(s) of another therapy. Preferably, the compound is present in the composition in an amount of from 0.1 to 50 wt.%, more preferably from 1 to 30 wt.%, most preferably from 5 to 20 wt.%.

The interrelationship of dosages for animals and humans (based on milligrams per meter squared of body surface) is described in Freireich et al., (1966) Cancer Chemother. Rep 50: 219. Body surface area may be approximately determined from height and weight of the subject. See, e.g., Scientific Tables, Geigy Pharmaceuticals, Ardsley, N.Y., 1970,537.

For pharmaceutical compositions that comprise a second therapeutic agent, an effective amount of the second therapeutic agent is between about 20% and 100% of the dosage normally utilized in a monotherapy regime using just that agent. Preferably, an effective amount is between about 70% and 100% of the normal monotherapeutic dose. The normal monotherapeutic dosages of these second therapeutic agents are well known in the art. See, e.g., Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000).

The compounds for use in the method of the disclosure can be formulated in unit dosage form. The term "unit dosage form" refers to physically discrete units suitable as unitary dosage for subjects undergoing treatment, with each unit containing a predetermined quantity of active material calculated to produce the desired therapeutic effect, optionally in association with a suitable pharmaceutical carrier. The unit dosage form can be for a single daily treatment dose or one of multiple daily treatment doses (e.g., about 1 to 4 or more times per day). When multiple daily treatment doses are used, the unit dosage form can be the same or different for each dose.

### METHODS OF TREATMENT

References in the present description to methods of medical treatment or medical uses are to be understood as references to the claimed compounds for use in methods of medical treatment. Methods of medical treatment *per se* are not encompassed by the claimed invention.

The disclosure also includes methods of treating diseases, disorders or pathological conditions which benefit from modulation of the APJ receptor comprising administering an effective amount of an APJ receptor compound of the disclosure to a subject in need thereof. Diseases and conditions which can benefit from modulation (inhibition or activation) of the APJ receptor include, but are not limited to, acute decompensated heart failure (ADHF), amyotrophic lateral sclerosis, arrhythmia, asthma, atherosclerosis, atherosclerosis, atrial fibrillation, Brugada syndrome, bum injuries (including sunburn), cancer, cardiac fibrosis, cardiomyopathy, cerebrovascular accidents, chronic heart failure, diabetes (including gestational diabetes), dyslipidemia, HIV neurodegeneration, hypertension, inflammation, ischemic cardiovascular diseases, liver disease, metabolic disorder, neurodegenerative disease, obesity, peripheral arterial disease, preeclampsia, pulmonary hypertension, restenosis, transient ischemic attacks, traumatic brain injuries, ventricular tachycardia, or water retention. More specifically, the hypertension may be pulmonary arterial hypertension. The liver disease may be alcoholic liver disease, toxicant-induced liver disease or viral-induced liver disease and the renal dysfunction may be polycystic kidney disease. The apelin receptor system is involved in vein-related disorders. See, e.g., Lathen et al., "ERG-APLNR Axis Controls Pulmonary Venule Endothelial Proliferation in Pulmonary Veno-Occlusive Disease" 2014 Circulation 130: 1179-1191. Apelin receptor system has also been implicated in heart failure. See, e.g., Sheikh et al., "In vivo genetic profiling and cellular localization of apelin reveals a hypoxia-sensitive, endothelial-centered pathway activated in ischemic heart failure" 2007 Am J Physiol Heart Circ Physiol 294:H88-H98. The contents of both Lathen *et al.* and Sheikh *et al.*

In one non-limiting embodiment, the disclosure provides a method of treating an apelin receptor (APJ) related disorder in a subject which comprises administering to the subject the compound of Formula I. The apelin receptor (APJ) related disorder may be asthma, atherosclerosis, cancer, cardiomyopathy, diabetes, dyslipidemia, hypertension, inflammation, liver disease, metabolic disorder, neurodegenerative disease, obesity, or preeclampsia. The disclosure provides methods further comprising treating the subject with an α-blocker, an angiotensin converting enzyme (ACE) inhibitor, an angiotensin-receptor blocker (ARB), a β-blocker, a calcium channel blocker, or a diuretic. Alternatively, the disclosure provides a method to treat or prevent a vein-related disorder such as an angioma, a venous insufficiency, a stasis or a thrombosis.

In addition, the disclosure provides a method of preventing HIV neurodegeneration in a subject which comprises administering to the subject the compound of embodiment 1.

Apelin receptors are widely expressed in endothelial cell lining lung capillaries and vessels. IPF, which is believed to be cause by prolonged airway insult, leads to basement membrane degradation as endothelial cells are lost. As a result, fibroblasts proliferate, scar tissue is formed, and lung function is compromised. APJ agonists, which may block endothelial cell injury present a novel treatment strategy for IPF. APJ agonists are believed to either block endothelial cell injury or promote regeneration or both. Apelinergic systems are believed to facilitate post-injury vascular development through endothelial cell signaling. Reference is made to Hou, Exp Mol Pathol (2017), 103, 203; Azizi, Eur J Pharmacol (2015), 761, 101; and Lathen, Circulation (2014), 130, 1179. Apelinergic system activation may preserve lung architecture and promote vascular regeneration in IPF.

One aspect of an embodiment of the present disclosure includes wherein capillary function is improved. One aspect of an embodiment of the present disclosure includes wherein receptor occupancy is prolonged. One aspect of an embodiment of the present disclosure includes wherein the mean survival time of the patient is improved. One aspect of any one of the embodiments and aspects of the present disclosure includes where the method of the present disclosure is used to treat one or more of idiopathic pulmonary fibrosis, asthma, chronic obstructive pulmonary disease (COPD), bronchitis, emphysema, pulmonary edema, acute respiratory disease syndrome (ARDS), interstitial lung disease, sarcoidosis, co-morbid pulmonary disorder, an autoimmune condition, rheumatoid arthritis, and scleroderma.

In one non-limiting embodiment, the present disclosure includes activation of the Apelin/APJ receptor in the endothelium /endothelial cells. Apelin/APJ receptor activation protects and repairs endothelial cells, and preserves the integrity of the basement membrane, thereby preserving organ function. The endothelium, which forms the inner cell lining of all blood vessels and lymphatics in the body, is a spatially distributed organ and present in the human lungs, heart, liver, kidneys and brain. Drugs targeting the Apelin/APJ in the apelinergic system pathway have been proposed for the treatment of Idiopathic Pulmonary Fibrosis, Pulmonary Artery Hypertension, Acute Lung Injury, Post Infection Pulmonary Fibrosis, Pulmonary Veno-Occlusive Disorder, Heart Failure, Hypertension, Metabolic Syndrome and Non-Alcoholic Steatosis Hepatitis.

Clinical and pre-clinical findings support the hypothesis that COVID-19 impairs endothelial function. Researchers have indicated that the virus is targeting the endothelium and supports the hypothesis that the endothelium organ is a key target in COVID-19. COVID-19 infections have shown a multi-organ damage and the status of the endothelial dysfunction in each patient play a role on outcome. The hypothesis is based on COVID-19 patients' diseases where pulmonary vascular endothelialitis, thrombosis, angiogenesis, pulmonary fibrosis, high blood pressure, arterial and venous thromboembolism, kidney disease, neurologic disorders, and diabetes mellitus have been observed as a result on endothelium injury via autopsies findings and/or post COVID-19 patients disease manifestation. See, for example, Journal Clinical Medicine (2020), 9;1417 / The New England Journal of Medicine (2020), 383;2 / Medical Hyppthesis (2020) 144;110015*.*

Embodiments of the present disclosure have been developed to target the apelinergic system signaling path in the endothelial cells to promote protection and regeneration on the endothelial cells to achieve improved health outcomes. Further profiling has led to the selection of preferential compounds, such as Example 22, which were synthetically designed to be biased to apelin (also known as GPCR selectivity) and which results in longer activation of the receptor therefore requiring lower doses to see efficacy. Therefore, COVID-19 patients treated with biased agonist to the apelin/APJ receptor system may provide protection and regeneration of the endothelial cells resulting in prevention of permanent injury various organs in the body. The compounds of the present disclosure offer potential for an opportunity as a multi organ prevention / protection of the endothelial cells against the post- or after-effects of COVID-19 injuries, including preventing fatalities and/or post COVID-19 diseases that could eventually become fatal.

In one embodiment, an effective amount of a compound of this disclosure can range from about .005 mg to about 5000 mg per treatment. In more specific embodiments, the range is from about .05 mg to about 1000 mg, or from about 0.5 mg to about 500 mg, or from about 5 mg to about 50 mg. Treatment can be administered one or more times per day (for example, once per day, twice per day, three times per day, four times per day, five times per day, etc.). When multiple treatments are used, the amount can be the same or different. It is understood that a treatment can be administered every day, every other day, every 2 days, every 3 days, every 4 days, every 5 days, etc. For example, with every other day administration, a treatment dose can be initiated on Monday with a first subsequent treatment administered on Wednesday, a second subsequent treatment administered on Friday, etc. Treatment is typically administered from one to two times daily. Effective doses will also vary, as recognized by those skilled in the art, depending on the diseases treated, the severity of the disease, the route of administration, the sex, age and general health condition of the subject, excipient usage, the possibility of co-usage with other therapeutic treatments such as use of other agents and the judgment of the treating physician.

Alternatively, the effective amount of a compound of the disclosure is from about 0.01 mg/kg/day to about 1000 mg/kg/day, from about 0.1 mg/kg/day to about 100 mg/kg/day, from about 0.5 mg/kg/day to about 50 mg/kg/day, or from about 1 mg/kg/day to 10 mg/kg/day.

In another embodiment, any of the above methods of treatment comprises the further step of co-administering to said subject one or more second therapeutic agents. The choice of second therapeutic agent may be made from any second therapeutic agent known to be useful for co-administration with a compound that modulates the APJ receptor. The choice of second therapeutic agent is also dependent upon the particular disease or condition to be treated. Examples of second therapeutic agents that may be employed in the methods of this disclosure are those set forth above for use in combination compositions comprising a compound of this disclosure and a second therapeutic agent.

The term "co-administered" as used herein means that the second therapeutic agent may be administered together with a compound of this disclosure as part of a single dosage form (such as a composition of this disclosure comprising a compound of the disclosure and a second therapeutic agent as described above) or as separate, multiple dosage forms. Alternatively, the additional agent may be administered prior to, consecutively with, or following the administration of a compound of this disclosure. In such combination therapy treatment, both the compounds of this disclosure and the second therapeutic agent(s) are administered by conventional methods. The administration of a composition of this disclosure, comprising both a compound of the disclosure and a second therapeutic agent, to a subject does not preclude the separate administration of that same therapeutic agent, any other second therapeutic agent or any compound of this disclosure to said subject at another time during a course of treatment.

In one embodiment of the disclosure, where a second therapeutic agent is administered to a subject, the effective amount of the compound of this disclosure is less than its effective amount would be where the second therapeutic agent is not administered. In another embodiment, the effective amount of the second therapeutic agent is less than its effective amount would be where the compound of this disclosure is not administered. In this way, undesired side effects associated with high doses of either agent may be minimized. Other potential advantages (including without limitation improved dosing regimens and/or reduced drug cost) will be apparent to those of skill in the art.

### KITS

The present disclosure also provides kits for use to treat the target disease, disorder or condition. These kits comprise (a) a pharmaceutical composition comprising a compound of Formula I, or a salt thereof, wherein said pharmaceutical composition is in a container; and (b) instructions describing a method of using the pharmaceutical composition to treat the target disease, disorder or condition.

The container may be any vessel or other sealed or sealable apparatus that can hold said pharmaceutical composition. Examples include bottles, ampules, divided or multi-chambered holders bottles, wherein each division or chamber comprises a single dose of said composition, a divided foil packet wherein each division comprises a single dose of said composition, or a dispenser that dispenses single doses of said composition. The container can be in any conventional shape or form as known in the art which is made of a pharmaceutically acceptable material, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. The container employed can depend on the exact dosage form involved, for example a conventional cardboard box would not generally be used to hold a liquid suspension. It is feasible that more than one container can be used together in a single package to market a single dosage form. For example, tablets may be contained in a bottle, which is in turn contained within a box. In one embodiment, the container is a blister pack.

The kits of this disclosure may also comprise a device to administer or to measure out a unit dose of the pharmaceutical composition. Such a device may include an inhaler if said composition is an inhalable composition; a syringe and needle if said composition is an injectable composition; a syringe, spoon, pump, or a vessel with or without volume markings if said composition is an oral liquid composition; or any other measuring or delivery device appropriate to the dosage formulation of the composition present in the kit.

In certain embodiments, the kits of this disclosure may comprise in a separate vessel of container a pharmaceutical composition comprising a second therapeutic agent, such as one of those listed above for use for co-administration with a compound of this disclosure.

Unless defined otherwise, all technical and scientific terms used herein have the same meaning as commonly understood by one of ordinary skill in the art to which this disclosure belongs. The article "a" and "an" are used herein to refer to one or more than one (i.e., to at least one) of the grammatical object(s) of the article. By way of example, "an element" means one or more elements.

Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps. The present disclosure may suitably "comprise", "consist of", or "consist essentially of", the steps, elements, and/or reagents described in the claims.

It is further noted that the claims may be drafted to exclude any optional element. As such, this statement is intended to serve as antecedent basis for use of such exclusive terminology as "solely", "only" and the like in connection with the recitation of claim elements, or the use of a "negative" limitation.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the disclosure. The upper and lower limits of these smaller ranges may independently be included or excluded in the range, and each range where either, neither or both limits are included in the smaller ranges is also encompassed within the disclosure, subject to any specifically excluded limit in the stated range. Where the stated range includes one or both of the limits, ranges excluding either or both of those included limits are also included in the disclosure.

The following Examples further illustrate the disclosure. It is also to be understood that the terminology used herein is for the purpose of describing particular embodiments only, the scope of the present disclosure will be limited only by the appended claims.

### EXAMPLES

### REPRESENTATIVE COMPOUNDS

**TABLE 1**

| Ex # | Structure |
|---|---|
| 1* | 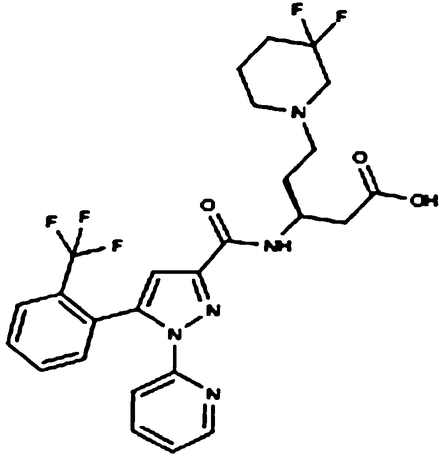 (3S)-5-(3,3-difluoropiperidin-1-yl)-3-{[1-(pyridin-2-yl)-5-[2-(trifluoromethyl)phenyl]-1H-pyrazol-3-yl]formamido}pentanoic acid |
| 2* | 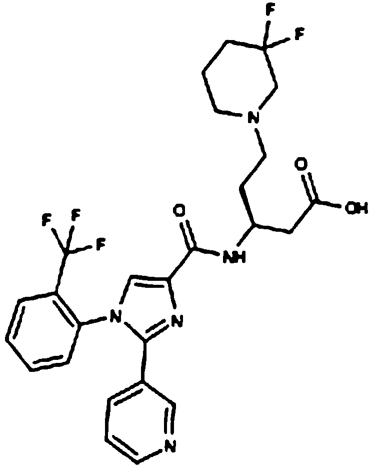 (3S)-5-(3,3-difluoropiperidin-1-yl)-3-{[2-(pyridin-3-yl)-1-[2-(trifluoromethyl)phenyl]-1H-imidazol-4-yl]formamido}pentanoic acid |
| 3* | 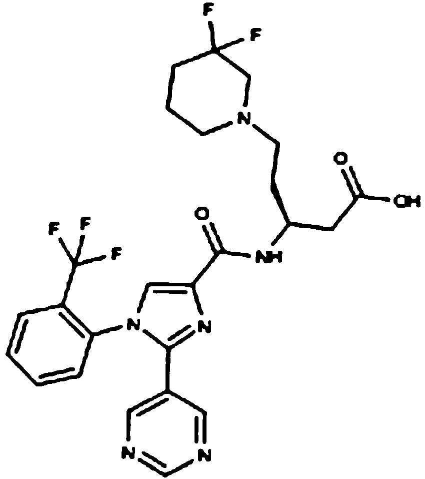 (3S)-5-(3,3-difluoropiperidin-1-yl)-3-{[2-(pyrimidin-5-yl)-1-[2-(trifluoromethyl)phenyl]-1H-imidazol-4-yl]formamido}pentanoic acid |
| 4* | 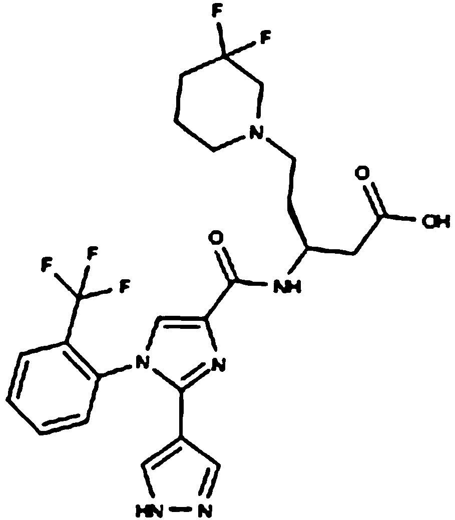 (3S)-5-(3,3-difluoropiperidin-1-yl)-3-{[2-(1H-pyrazol-4-yl)-1-[2-(trifluoromethyl)phenyl]-1H-imidazol-4-yl]formamido}pentanoic acid |
| 5* | 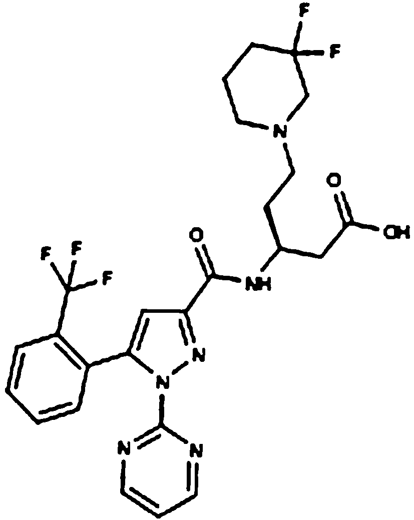 (3S)-5-(3,3-difluoropiperidin-1-yl)-3-{[1-(pyrimidin-2-yl)-5-[2-(trifluoromethyl)phenyl]-1H-pyrazol-3-yl]formamido}pentanoic acid |
| 6* | 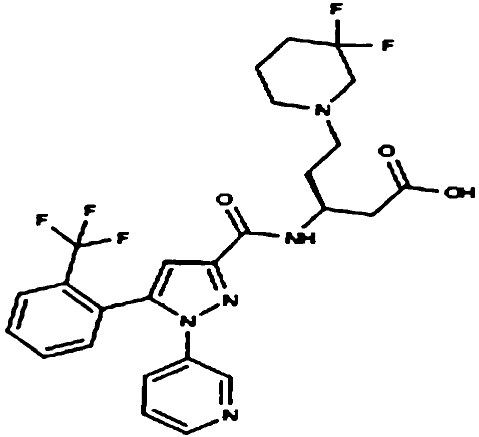 (3S)-5-(3,3-difluoropiperidin-1-yl)-3-{[1-(pyridin-3-yl)-5-[2-(trifluoromethyl)phenyl]-1H-pyrazol-3-yl]formamido}pentanoic acid |
| 7* | 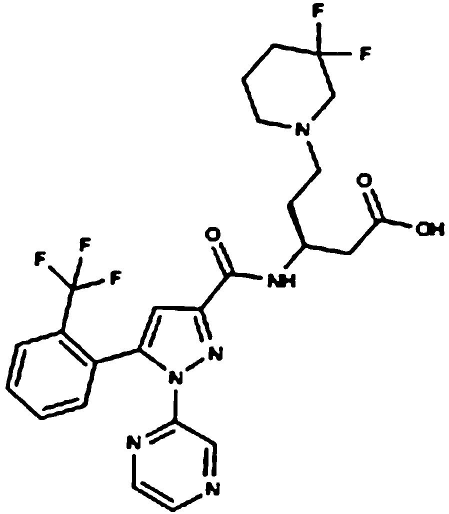 (3S)-5-(3,3-difluoropiperidin-1-yl)-3-{[1-(pyrazin-2-yl)-5-[2-(trifluoromethyl)phenyl]-1H-pyrazol-3-yl]formamido}pentanoic acid |
| 8* | 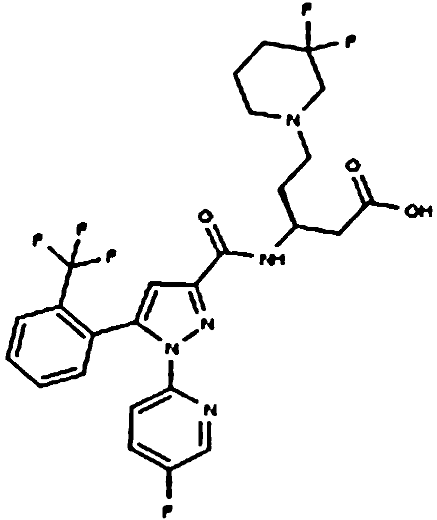 (3S)-5-(3,3-difluoropiperidin-1-yl)-3-{[1-(5-fluoropyridin-2-yl)-5-[2-(trifluoromethyl)phenyl]-1H-pyrazol-3-yl]formamido}pentanoic acid |
| 9* | 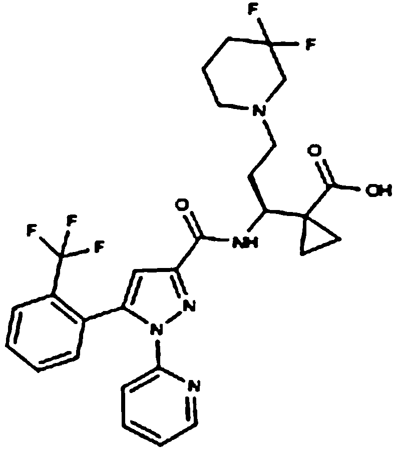 1-[(1S)-3-(3,3-difluoropiperidin-1-yl)-1-{[1-(pyridin-2-yl)-5-[2-(trifluoromethyl)phenyl]-1H-pyrazol-3-yl]formamido}propyl]cyclopropane-1-carboxylic acid |
| 10* | 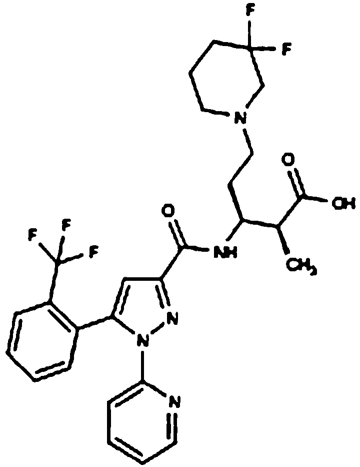 (2S,3S)-5-(3,3-difluoropiperidin-1-yl)-2-methyl-3-{[1-(pyridin-2-yl)-5-[2-(trifluoromethyl)phenyl]-1H-pyrazol-3-yl]formamido}pentanoic acid |
| 11* | 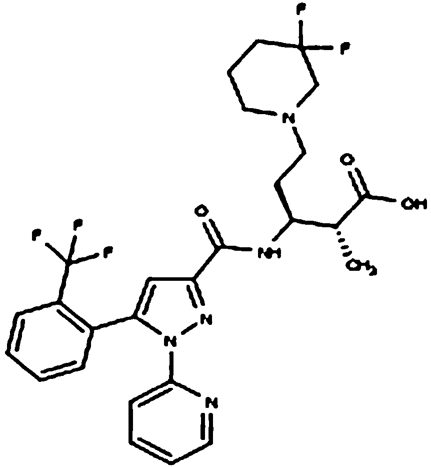 2R,3S)-5-(3,3-difluoropiperidin-1-yl)-2-methyl-3-{[1-(pyridin-2-yl)-5-[2-(trifluoromethyl)phenyl]-1H-pyrazol-3-yl]formamido}pentanoic acid |
| 12* | 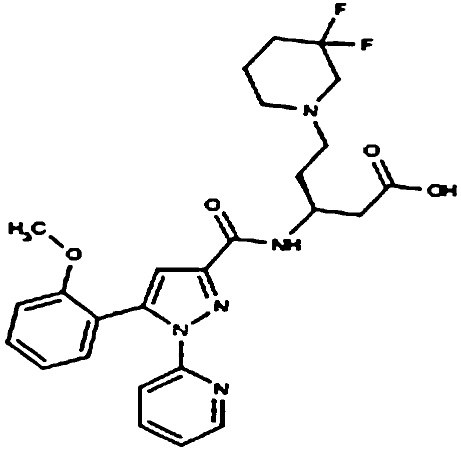 (3S)-5-(3,3-difluoropiperidin-1-yl)-3-{[5-(2-methoxyphenyl)-1-(pyridin-2-yl)-1H-pyrazol-3-yl]formamido}pentanoic acid |
| 13* | 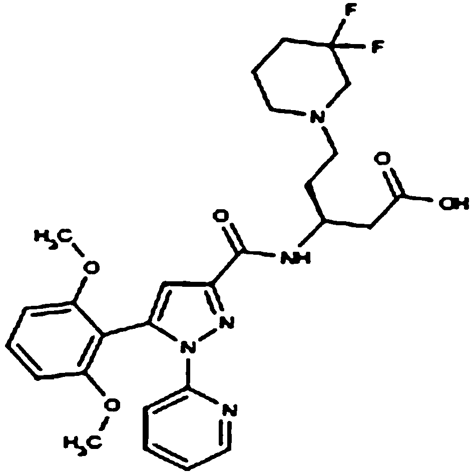 (3S)-5-(3,3-difluoropiperidin-1-yl)-3-{[5-(2,6-dimethoxyphenyl)-1-(pyridin-2-yl)-1H-pyrazol-3-yl]formamido}pentanoic acid |
| 14* | 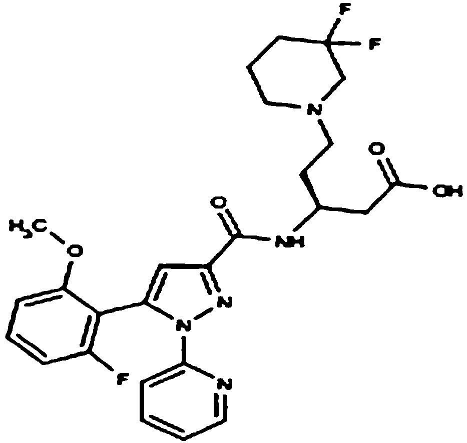 (3S)-5-(3,3-difluoropiperidin-1-yl)-3-{[5-(2-fluoro-6-methoxyphenyl)-1-(pyridin-2-yl)-1H-pyrazol-3-yl]formamido}pentanoic acid |
| 15* | 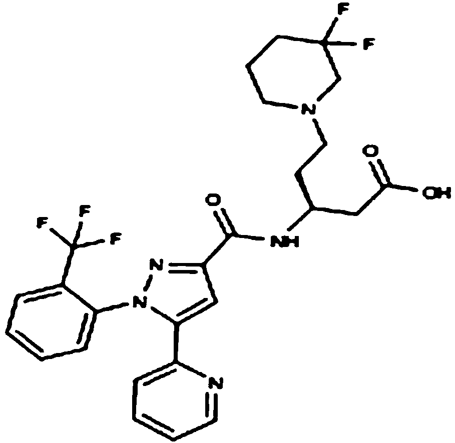 (3S)-5-(3,3-difluoropiperidin-1-yl)-3-{[5-(pyridin-2-yl)-1-[2-(trifluoromethyl)phenyl]-1H-pyrazol-3-yl]formamido}pentanoic acid |
| 16* | 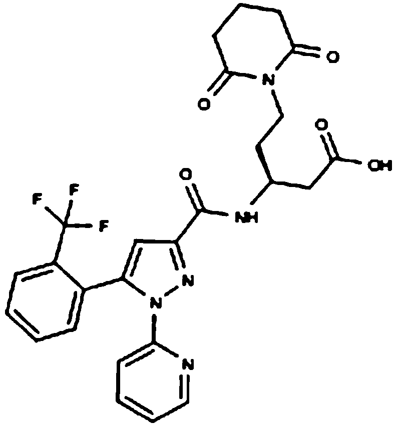 (3S)-5-(2,6-dioxopiperidin-1-yl)-3-{[1-(pyridin-2-yl)-5-[2-(trifluoromethyl)phenyl]-1H-pyrazol-3-yl]formamido}pentanoic acid |
| 17* | 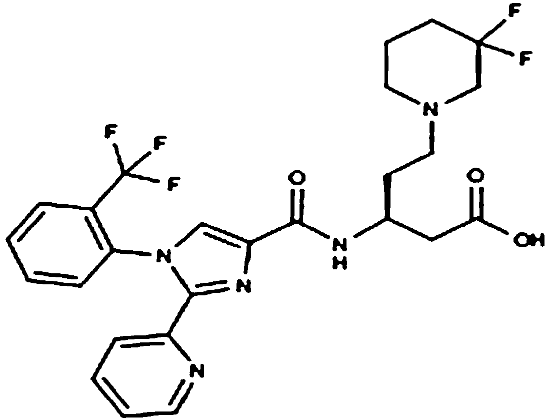 (3S)-5-(3,3-difluoropiperidin-1-yl)-3-{[2-(pyridin-2-yl)-1-[2-(trifluoromethyl)phenyl]-1H-imidazol-4-yl]formamido}pentanoic acid |
| 18* | 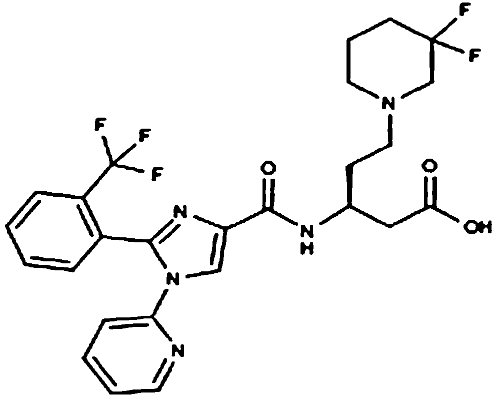 (3S)-5-(3,3-difluoropiperidin-1-yl)-3-{[1-(pyridin-2-yl)-2-[2-(trifluoromethyl)phenyl]-1H-imidazol-4-yl]formamido}pentanoic acid |
| 19* | 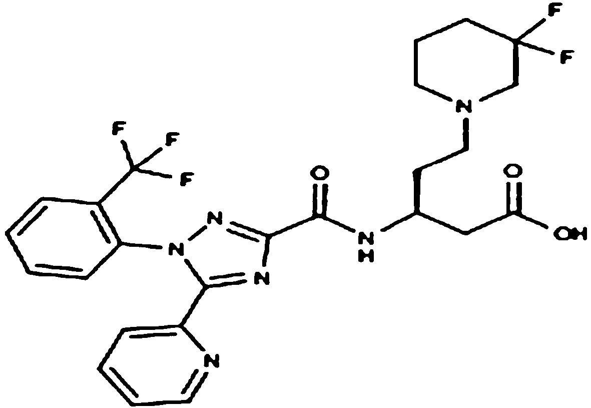 (3S)-5-(3,3-difluoropiperidin-1-yl)-3-{[5-(pyridin-2-yl)-1-[2-(trifluoromethyl)phenyl]-1H-1,2,4-triazol-3-yl]formamido}pentanoic acid |
| 20* | 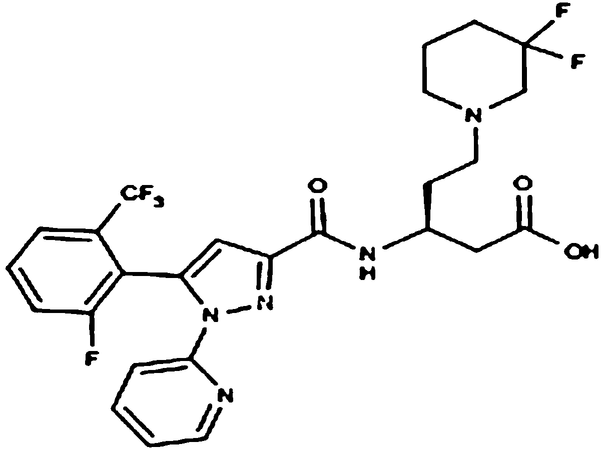 (3S)-5-(3,3-difluoropiperidin-1-yl)-3-({5-[2-fluoro-6-(trifluoromethyl)phenyl]-1-(pyridin-2-yl)-1H-pyrazol-3-yl}formamido)pentanoic acid |
| 21* | 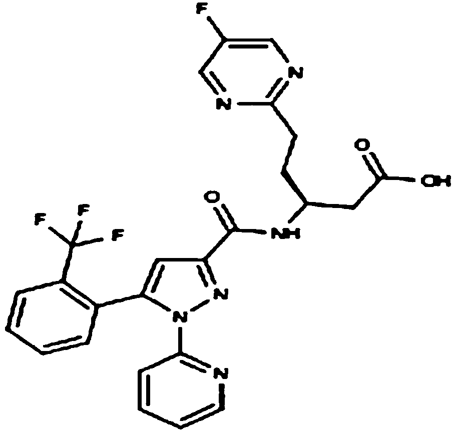 (3S)-5-(5-fluoropyrimidin-2-yl)-3-{[1-(pyridin-2-yl)-5-[2-(trifluoromethyl)phenyl]-1H-pyrazol-3-yl]formamido}pentanoic acid |
| 22 | 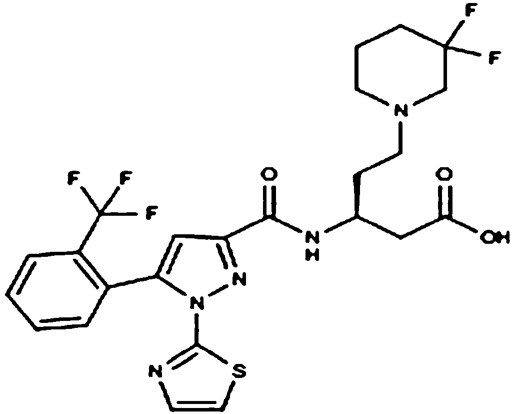 (3S)-5-(3,3-difluoropiperidin-1-yl)-3-{[1-(1,3-thiazol-2-yl)-5-[2-(trifluoromethyl)phenyl]-1H-pyrazol-3-yl]formamido}pentanoic acid |
| 23* | 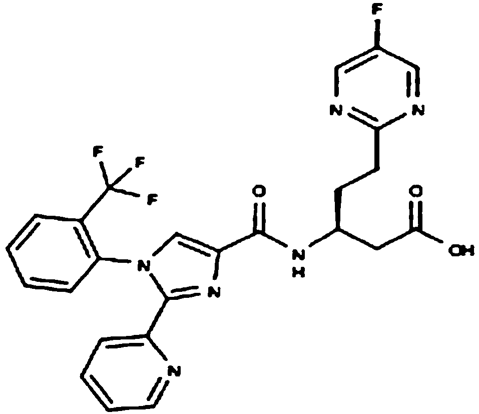 (3S)-5-(5-fluoropyrimidin-2-yl)-3-{[2-(pyridin-2-yl)-1-[2-(trifluoromethyl)phenyl]-1H-imidazol-4-yl]formamido}pentanoic acid |
| 24* | 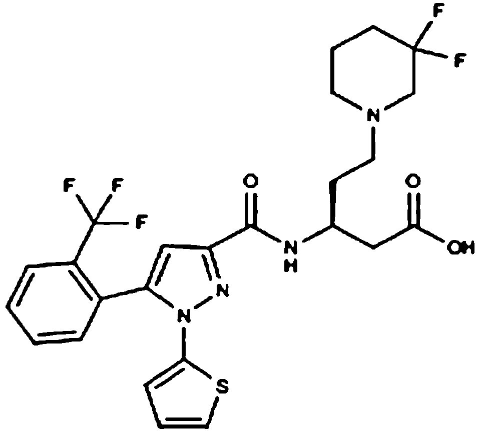 (3S)-5-(3,3-difluoropiperidin-1-yl)-3-{[1-(thiophen-2-yl)-5-[2-(trifluoromethyl)phenyl]-1H-pyrazol-3-yl]formamido}pentanoic acid |
| 25 | 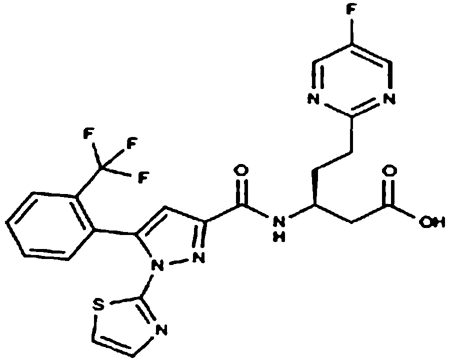 (3S)-5-(5-fluoropyrimidin-2-yl)-3-{[1-(1,3-thiazol-2-yl)-5-[2-(trifluoromethyl)phenyl]-1H-pyrazol-3-yl]formamido}pentanoic acid |
| 26 | 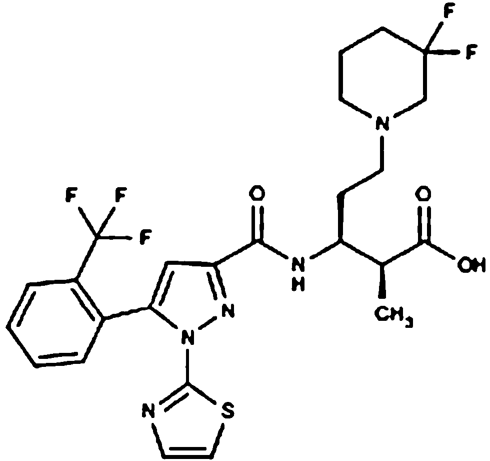 (2S,3S)-5-(3,3-difluoropiperidin-1-yl)-2-methyl-3-{[1-(1 ,3-thiazol-2-yl)-5-[2-(trifluoromethyl)phenyl]-1H-pyrazol-3-yl]formamido}pentanoic acid |
| 27* | 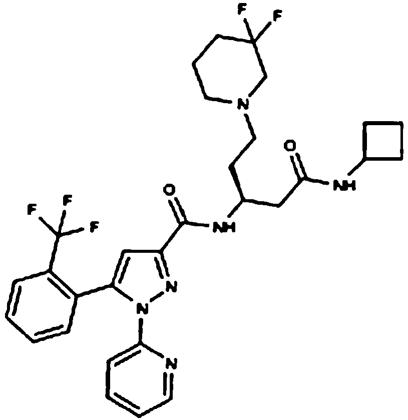 (3S)-N-cyclobutyl-5-(3,3-difluoropiperidin-1-yl)-3-{[1-(pyridin-2-yl)-5-[2-(trifluoromethyl)phenyl]-1H-pyrazol-3-yl]formamido}pentanamide |
| 28* | 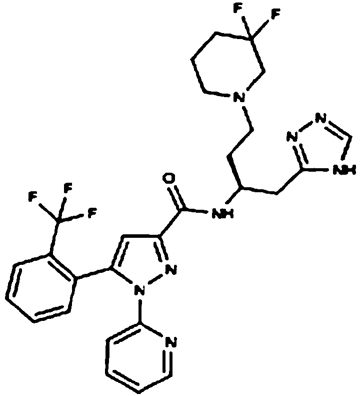 N-[(2S)-4-(3,3-difluoropiperidin-1-yl)-1-(4H-1 ,2,4-triazol-3-yl)butan-2-yl]-1-(pyridin-2-yl)-5-[2-(trifluoromethyl)phenyl]-1H-pyrazole-3-carboxamide |
| 29 | 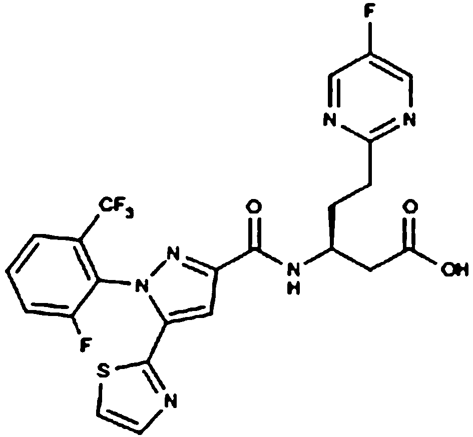 (3S)-3-({1-[2-fluoro-6-(trifluoromethyl)phenyl]-5-(1,3-thiazol-2-yl)-1H-pyrazol-3-yl}formamido)-5-(5-fluoropyrimidin-2-yl)pentanoic acid |
| 30 | 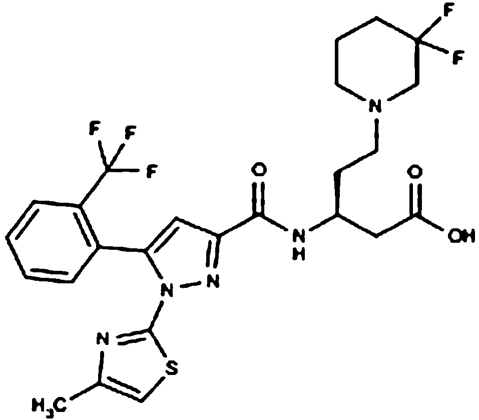 (3S)-5-(3,3-difluoropiperidin-1-yl)-3-{[1-(4-methyl-1,3-thiazol-2-yl)-5-[2-(trifluoromethyl)phenyl]-1H-pyrazol-3-yl]formamido}pentanoic acid |
| 31 | 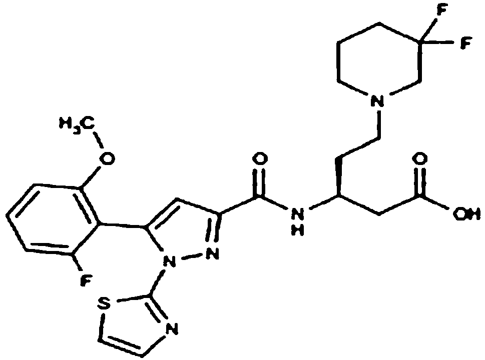 (3S)-5-(3,3-difluoropiperidin-1-yl)-3-{[5-(2-fluoro-6-methoxyphenyl)-1-(1,3-thiazol-2-yl)-1H-pyrazol-3-yl]formamido}pentanoic acid |
| 32 | 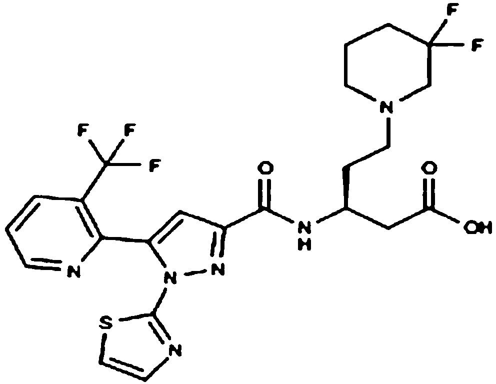 (3S)-5-(3,3-difluoropiperidin-1-yl)-3-{[1-(1,3-thiazol-2-yl)-5-[3-(trifluoromethyl)pyridin-2-yl]-1H-pyrazol-3-yl]formamido}pentanoic acid |
| 33* | 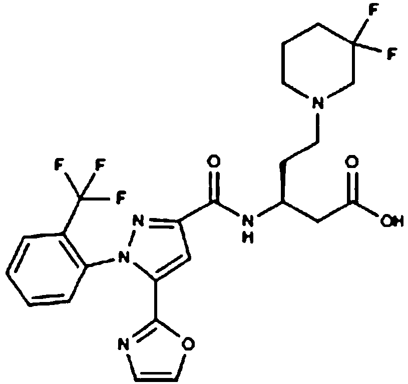 (3S)-5-(3,3-difluoropiperidin-1-yl)-3-{[5-(1,3-oxazol-2-yl)-1-[2-(trifluoromethyl)phenyl]-1H-pyrazol-3-yl]formamido}pentanoic acid |
| 34 | 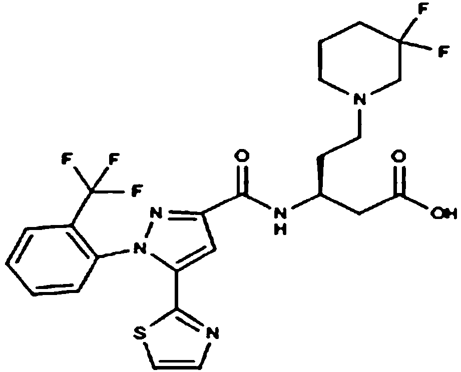 (3S)-5-(3,3-difluoropiperidin-1-yl)-3-{[5-(1,3-thiazol-2-yl)-1-[2-(trifluoromethyl)phenyl]-1H-pyrazol-3-yl]formamido}pentanoic acid |
| 35 | 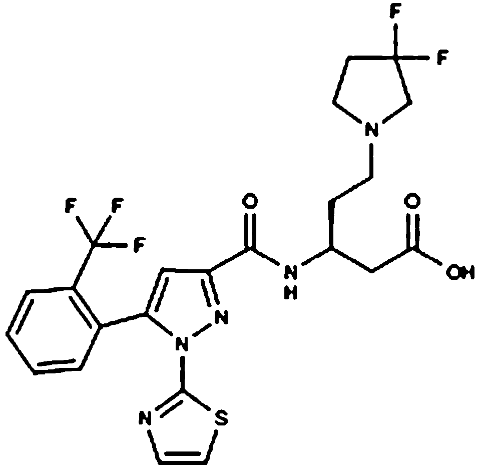 (3S)-5-(3,3-difluoropyrrolidin-1-yl)-3-{[1-(1,3-thiazol-2-yl)-5-[2-(trifluoromethyl)phenyl]-1H-pyrazol-3-yl]formamido}pentanoic acid |
| 36 | 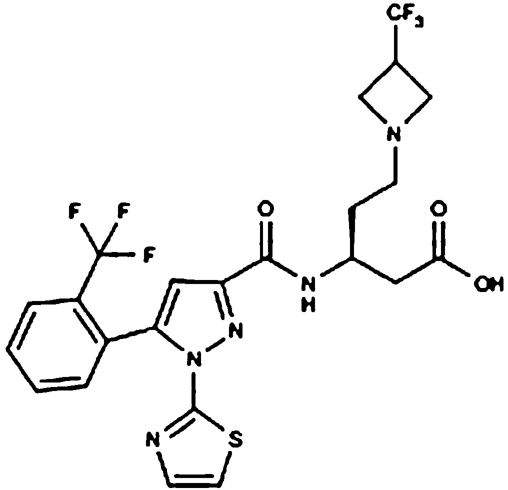 (3S)-3-{[1-(1,3-thiazol-2-yl)-5-[2-(trifluoromethyl)phenyl]-1H-pyrazol-3-yl]formamido}-5-[3-(trifluoromethyl)azetidin-1-yl]pentanoic acid |
| 37 | 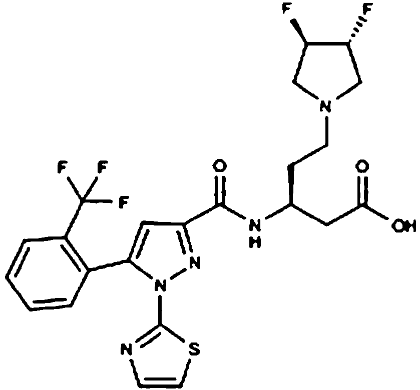 (3S)-5-[(3R,4R)-3,4-difluoropyrrolidin-1-yl]-3-{[1-(1,3-thiazol-2-yl)-5-[2-(trifluoromethyl)phenyl]-1H-pyrazol-3-yl]formamido}pentanoic acid |
| 38 | 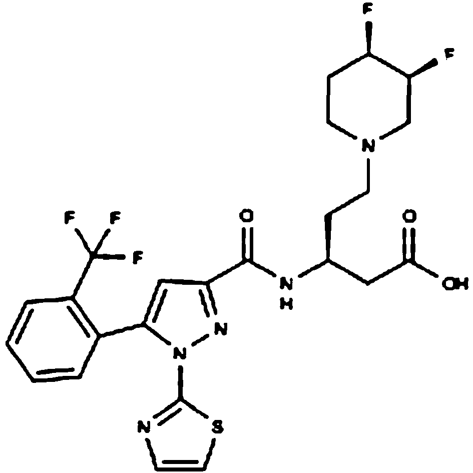 (3S)-5-[(3S,4R)-3,4-difluoropiperidin-1-yl]-3-{[1-(1,3-thiazol-2-yl)-5-[2-(trifluoromethyl)phenyl]-1 H-pyrazol-3-yl]formamido}pentanoic acid |

| | |
|---|---|
| * denotes a refence example that does not fall within the scope of the claims. | |

### METHOD AND PREPARATION OF REPRESENTATIVE COMPOUNDS

### Synthetic Examples

### Example 22

### Intermediate 1. Synthesis of 1-(thiazol-2-yl)-5-(2-(trifluoromethyl)phenyl)-1H-pyrazole-3-carboxylic acid

### Step 1: Synthesis of methyl 2,4-dioxo-4-(2-(trifluoromethyl)phenyl)butanoate (2)

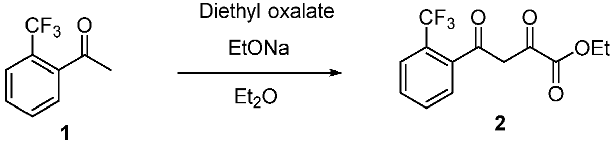

Sodium ethoxide (21% in ethanol) (12 mL) was added dropwise to a solution of 2-(trifluoromethyl)acetophenone 1 (5.00 g, 26.70 mmol) and diethyl oxalate (3.6 mL, 26.70 mmol) in Et₂O (30 mL). The reaction mixture was stirred at rt for 16 h. After stirring for 16 h, the mixture was concentrated under reduced pressure. To the residue, water (100 mL) was added and the mixture was stirred at rt for 1 h then acidified with glacial acetic acid to pH 2-3. The mixture was extracted with Et₂O (2x100 mL). The combined organic layers were washed with saturated aqueous NaHCO3 (100 mL) and brine (100 mL). The resultant solution was dried over Na₂SO4, filtered, concentrated under reduced pressure and dried under vacuum to provide 1.90 g (25%) of the title compound 2 as light brown solid. ¹H NMR (300 MHz, CDCl₃): δ 1.31 (t, J=7.06 Hz, 3 H), 4.15 - 4.35 (m, 2 H) 6.40-6.56 (m, 1 H) 7.42 - 7.81 (m, 5 H). LCMS (ESI): m/z calculated for C₁₃H₁₁F₃O₄ [M]⁺: 288.06, found: 289.20 [M+H]⁺.

### Step 2: Synthesis of 2-hydrazineylthiazole hydrochloride

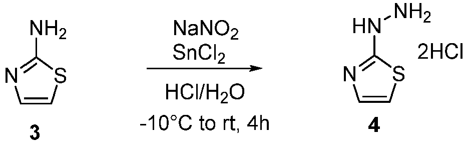

A chilled solution of sodium nitrite (20.7 g, 299.58 mmol) in water (150 mL) was gradually added to a suspension of 2-aminothiazole 3 (30 g, 299.58 mmol) in concentrated HCl (240 mL) at -10 °C (ice/acetone bath). The reaction mixture was stirred at -10 °C for 0.5 h. A solution of tin (II) chloride (113.6 g, 599.16 mmol) in concentrated HCl (70 mL) was added at -10 °C. The reaction mixture was stirred 1 h, then warmed to room temperature. The solid was collected by filtration, washed with ether and vacuum dried overnight to give 70.6 g yellow solid. This yellow solid was boiled in ether (400 mL), filtered and vacuum dried overnight to give 64.6 g of the title compound 4. ¹H NMR (300 MHz, DMSO-*d*₆): δ 2.96 (br.s., 2 H), 7.01 (d, J=3.96 Hz, 1 H), 7.29 (d, J=4.14 Hz, 1 H), 10.45 ((br.s., 1 H).

### Step 3: Synthesis of 1-(thiazol-2-yl)-5-(2-(trifluoromethyl)phenyl)-1H-pyrazole-3-carboxylic acid

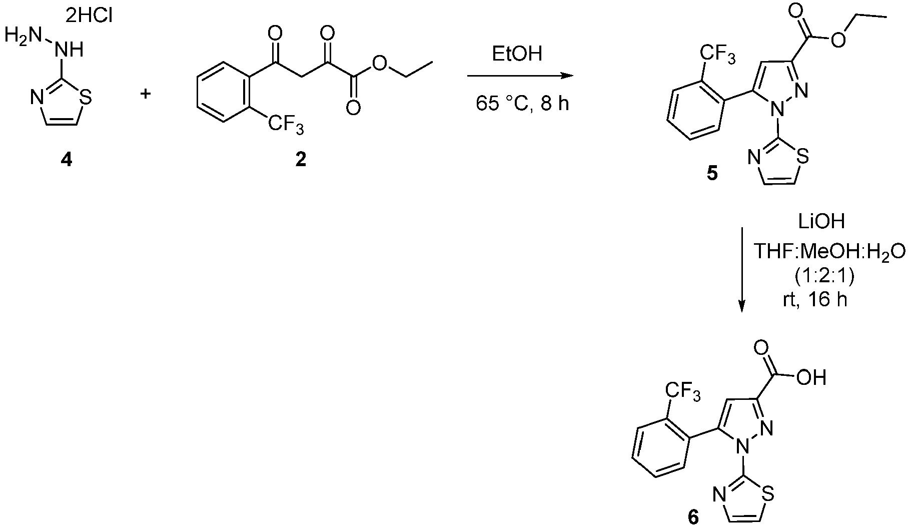

In a 2L round bottom flask, a solution of 2 (30 g, 104 mmol) and 4 (34.3 g, 182 mmol, 1.75 eq) in ethanol (675 mL) was stirred for 8 h at 65 °C while monitoring by TLC (25% EtOAc/hexane). After 1 h a clear, amber solution was observed. After 8 h the heat was turned off and the reaction continued stirring overnight at room temperature. The reaction mixture was concentrated, taken up and filtered from DCM, and purified by column chromatography (EtOAc/hexanes, 330 g silica gel column, 0-10% over 15 CV to 50% EtOAc at 20 CV). Impure fractions were re-purified by column to give 26.8 g (70%) as a yellow-tint solid of **5.**

¹H NMR (300 MHz, CDCl₃): δ 1.44 (t, *J*=7.06 Hz, 3 H), 4.47 (q, *J*=7.16 Hz, 2 H), 6.97 (s, 1 H) 7.05 - 7.12 (m, 1 H), 7.43 (dd, *J*=4.99, 3.86 Hz, 1 H) 7.55 - 7.65 (m, 3 H) 7.77 (dd, *J*=5.46, 3.96 Hz, 1 H). LCMS (ESI): m/z calculated for C₁₆H₁₂F₃N₃O₂S [M]⁺: 367.06, found: 368.20 [M+H]⁺.

To a solution of 5 (26.4 g, 72 mmol) in THF (60 mL), MeOH (120) and H₂O (60 mL) was gradually added LiOH (5 g, 215 mmol). The solution was stirred overnight at room temperature and monitored by TLC. The reaction mixture was concentrated to remove organic solvent, acidified with 1N HCl (~250 mL) and extracted with EtOAc. The organic layer was dried (Na₂SO₄), concentrated to a purple solid, then swirled in a small amount of DCM. After chilling, the mixture was filtered and solid rinsed with cold DCM to give a white powder, vacuum dried overnight to give 23.19 g (95%) of acid 6.

¹H NMR (300 MHz, CDCl₃): δ 7.05 (s, 1 H), 7.13 (dd, J=3.49, 1.22 Hz, 1 H), 7.30 (d, J=3.39 Hz, 1 H), 7.42 - 7.48 (m, 1 H), 7.58 - 7.67 (m, 2 H), 7.74 - 7.82 (m, 1 H). LCMS (ESI): *m*/*z* calculated for C₁₄H₈F₃N₃O₂S [M]⁺: 339.03, found: 340.20 [M+H]⁺.

### Intermediate 2. Synthesis of tert-butyl (S)-3-amino-5-(3,3-difluoropiperidin-1-yl)pentanoate (11)

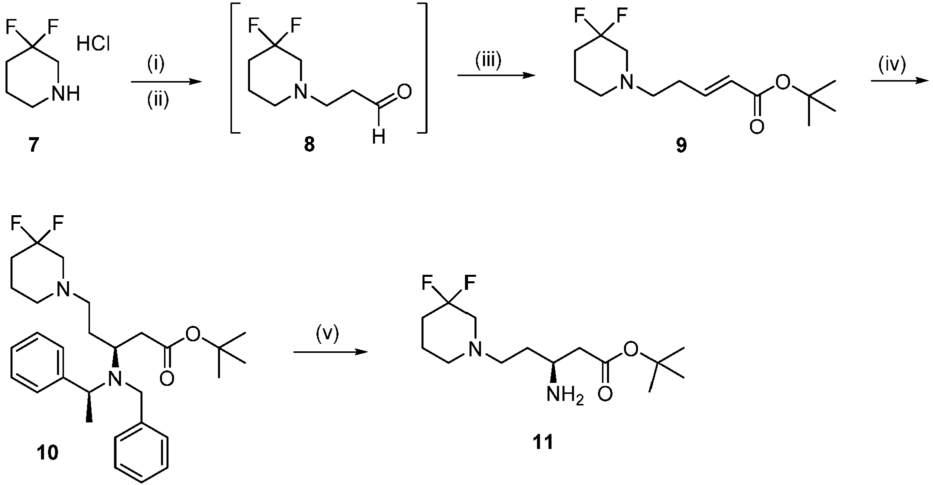

***Reagents and conditions:*** (i) KOtBu, EtOH, rt; (ii) acrolein, DBU, THF, 0 °C; (iii) tert-butyl diethylphosphonoacetate, KOtBu, THF, 0 °C, 30 min, rt, 1 h; (iv) (*S*)-*N*-benzyl-*N*-α-methyl-benzylamine, BuLi, THF, -78 °C; (v) 10% Pd/C, H₂, 45 psi, MeOH, DCM, rt.

### tert-Butyl (E)-5-(3,3-difluoropiperidin-1-yl)pent-2-enoate (9)

To a suspension of 3,3-difluoropiperidine HCl (7) (30 g, 190 mmol) in EtOH (165 mL) at -50° C was gradually added KOtBu (23.5 g, 209 mmol) while monitoring the internal temperature. After stirring 60 min the mixture was filtered in a large Buchner funnel to spread out the fine solid precipitate. The filtrate was cooled to -40° C and the precipitate was rinsed with THF (150 mL). To the filtrate was added more THF (300 mL) and DBU (1.42 mL, 9.5 mmol) at -40° C, followed by dropwise addition of acrolein (15.5 mL, 230 mmol). The reaction mixture was stirred at -40° C for 60 min to provide the crude aldehyde **8,** developing a greenish to light yellow color. Meanwhile, to a solution of t-butyl diethylphosphonoacetate (58 g, 230 mmol) in THF (500 mL) was gradually added KOtBu (25.8 g, 230 mmol) and the solution was stirred at - 40° C for 30 min. To this solution was added the above piperidine aldehyde solution via cannula over 30 min, giving a brown solution. The reaction mixture was gradually allowed to warm to rt and stirred 2-3 h. The solution was diluted with EtOAc (500 mL) and washed with water (300 mL). The organic portion was washed with brine (3 x 200 mL), dried (Na₂SO₄) and concentrated. The crude product was purified by silica gel flash chromatography (0-2% and 2-4% of EtOAc in hexanes) to give the product in three fractions (30 g, 57%) as colorless liquid. TLC *R_{f}* = 0.50 (hexanes/EtOAc, 10:1, UV light and/or KMnO₄ stain). ¹H NMR (200 MHz, DMSO-*d*₆): δ 1.45 (s, 9H), 1.70-2.00 (m, 4H), 2.30-2.70 (m, 8H), 5.70-5.80 (m, 1H), 6.70-6.90 (m, 1H). LCMS (ESI): *m*/*z* calculated for C₁₄H₂₃F₂NO₂ 275.17 [M]⁺; found: 276.0 [M+H]⁺.

***tert-Butyl* (S)-3-(benzyl((S)-1-phenylethyl)amino)-5-(3,3-difluoropiperidin-1-yl)pentanoate (10).** To a stirred solution of (*S*)-*N*-benzyl-N-α-methylbenzylamine (46.2 g, 0.218 mol) in THF (430 mL) at -78 °C was added n-BuLi (2.5 M in hexanes) (87.5 mL, 0.218 mol) using cannula over 20 min. Then solution of **9** (42.1 g, 0.156 mol) in THF (100 mL) also at -78 °C was transferred via cannula. The resulting solution was stirred at -78 °C for 3 h before quenching at 0 °C with 20% citric acid solution (300 mL). The aqueous layer was extracted with hexanes (2 x 200 mL). The combined organic layers were washed with 20% citric acid solution (2 x 100 mL) to remove the excess amine. The organic layer was washed with brine (200 mL), dried with Na₂SO₄ and the solvent was removed in vacuo to give the crude product. Crude product was purified by Combiflash *R_{f}* (0-5% of EtOAc in hexanes) and the fractions containing the product (TLC) were pooled and evaporated to obtain 51 g (67%) of **10** as white crystalline solid. TLC *R_{f}* = 0.75 (hexanes/EtOAc, 3:1). ¹H NMR (200 MHz, DMSO-*d*₆): δ 1.35 (d, *J =* 7.0 Hz, 3 H), 1.40 (s, 9H), 1.45-1.60 (m, 4H), 1.70-2.00 (m, 6H), 2.40-2.70 (m, 6H), 3.40-3.60 (m, 2H), 7.20-7.40 (m, 10H). LCMS (ESI): m/z calculated for C₂₉H₄₀F₂N₂O₂ 486.31 [M]⁺; found: 487.2 [M+H]⁺.

***tert-Butyl* (S)-3-amino-5-(3,3-difluoropiperidin-1-yl)pentanoate (11).** Compound **10** (30 g, 61.7 mmol) was dissolved in MeOH (150 mL) and DCM (50 mL) and to it was added 10 % Pd/C (6.6 g). The mixture was hydrogenated in Parr hydrogenator at 45 psi pressure for 24 h. The catalyst was removed by filtration through celite and the solvent was evaporated to obtain 18 g (quant.) of **11** as an off-white solid oil. ¹H NMR (200 MHz, DMSO-*d*₆): δ 1.40 (s, 9H), 1.70-2.00 (m, 4H), 2.20-3.00 (m, 10H), 3.60-3.80 (m, 1H), 8.80 (br, 2H). ¹H NMR purity: >95%. LCMS (ESI): *m*/*z* calculated for C₁₄H₂₆F₂N₂O₂ 292.20 [M]⁺; found: 293.10 [M+H]⁺.

### Synthesis of target compound 13 (Example 22)

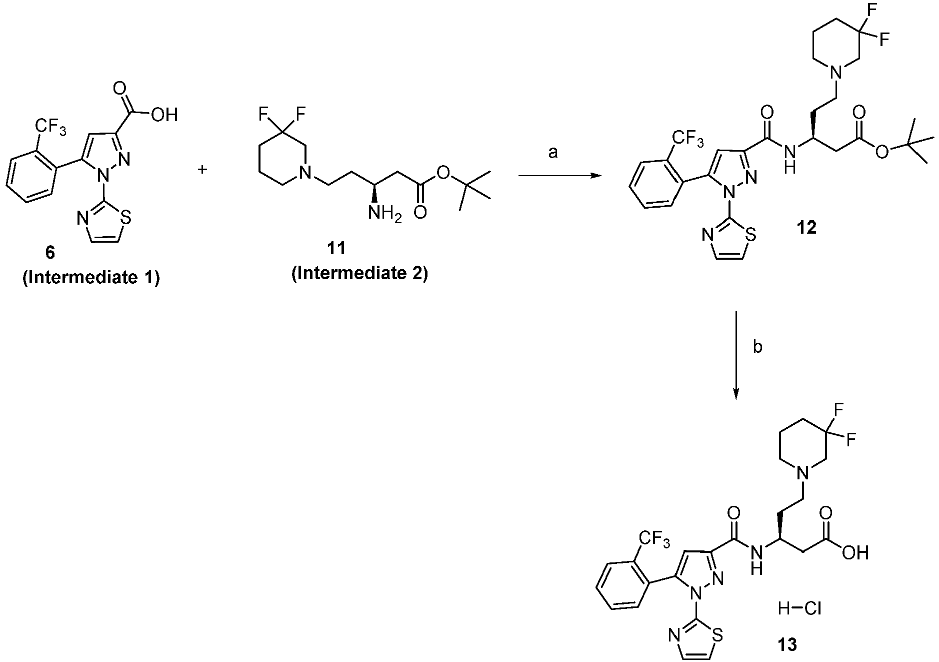

### Reagents and conditions: (a) HBTU, CH₃CN, rt, 16 h; (b) 4M HCl in dioxane, DCM, rt, 16 h

### tert-Butyl(S)-5-(3,3-difluoropiperidin-1-yl)-3-(1-(thiazol-2-yl)-5-(2-(trifluoromethyl)phenyl)-1H-pyrazole-3-carboxamido)pentanoate (12)

1-(thiazol-2-yl)-5-(2-(trifluoromethyl)phenyl)-1*H*-pyrazole-3-carboxylic acid (6) (50 mg, 0.147 mmol) was dissolved in acetonitrile (7 mL). To the solution was added HBTU (84 mg, 0.220 mmol) and tert-butyl (S)-3-amino-5-(3,3-difluoropiperidin-1-yl)pentanoate (11) (48 mg, 0.162 mmol). To the mixture at rt was added dropwise triethylamine (0.062 mL, 0.441 mmol). The mixture was stirred at rt for 16 h. Acetonitrile was evaporated in vacuo. The residue was diluted with DCM and washed with water (30 mL), brine (30 mL). The combined organic layers were dried with Na₂SO₄ and concentrated in vacuo. The crude product was purified by silica flash chromatography (0-40% EtOAc:hexanes) to give pure **12** as white solid (65 mg, 90%). ¹H NMR (300 MHz, CDCl₃): δ 1.48 (s, 9 H), 1.73 - 1.83 (m, 3 H), 1.83 - 1.97 (m, 4 H), 2.44-2.42 (m, 2 H), 2.53 - 2.75 (m, 6 H), 4.44 - 4.63 (m, 1 H), 6.97 (s, 1 H), 7.07 (d, J=3.58 Hz, 1 H), 7.40-7.45 (m, 1 H), 7.57 - 7.62 (m, 2 H), 7.65 (d, *J*=9.61 Hz, 1 H), 7.73 - 7.80 (m, 1 H). LCMS (ESI): *m*/*z* calculated for C₂₈H₃₂F₅N₅O₃S [M]⁺: 613.21, found: 614.60 [M+H]⁺.

### (S)-5-(3,3-Difluoropiperidin-1-yl)-3-(1-(thiazol-2-yl)-5-(2-(trifluoromethyl)phenyl)-1H-pyrazole-3-carboxamido)pentanoic acid (13)

4M HCl in dioxane (0.300 mL) was added dropwise at rt to a solution of tert-butyl(S)-5-(3,3-difluoropiperidin-1-yl)-3-(1-(thiazol-2-yl)-5-(2-(trifluoromethyl)phenyl)-1 H-pyrazole-3-carboxamido)pentanoate (12) (65 mg, 0.105 mmol) in CH₂Cl₂ (4 mL). The reaction mixture was stirred at rt for 16 h. The solvent was evaporated in vacuo and dried under high vacuum. Residue was triturated with ether, filtered to give the title compound **13** (60 mg, 63%) as white solid. ¹H NMR (300 MHz, DMSO-*d*₆) δ 1.72-2.30 (m, 5 H), 2.61-2.81 (m, 3 H), 2.92-3.30 (m, 3 H), 3.46-3.57 (m, 2 H), 3.83-4.06 (m, 1 H), 4.31-4.45 (br. s., 1 H), 7.01 (s, 1 H), 7.41 (s, 1 H), 7.52 - 7.66 (m, 2 H), 7.75 (d, J=3.39 Hz, 2 H), 7.88 (d, *J*=6.97 Hz, 1 H), 8.53 (br. s., 1 H), 10.40 (s, 1 H). LCMS (ESI): *m*/*z* calculated for freebase C₂₄H₂₄F₅N₅O₃S [M]⁺: 557.15, Found: 558.60 [M+H]⁺.

Additional compounds of the present disclosure were made using the procedures as herein disclosed in conjunction with the following general schemes.

### Schemes

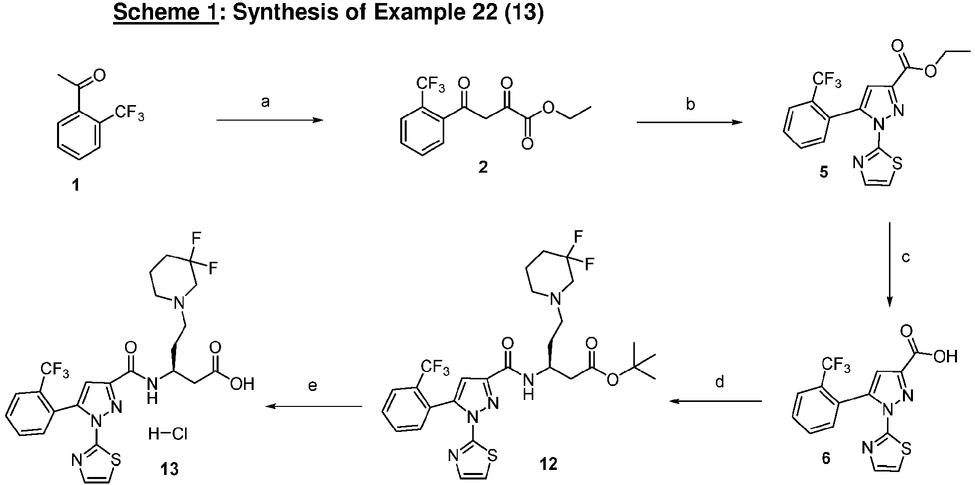

**Scheme** 1: *Reagents and conditions:* a) Diethyl oxalate, Et₂O, rt, 16 h; b) 2-hydrazineylthiazole dihydrochloride (4), EtOH, 65 °C, 8 h; c) LiOH, MeOH/THF/H₂O (2:1:1), rt, 16 h; d) tert-butyl *(S)-*3-amino-5-(3,3-difluoropiperidin-1-yl)pentanoate (11), HBTU, CH₃CN, rt, 16 h; e) 4M HCl in dioxane, DCM, rt, 16.

### Intermediate 1. Synthesis of 2-hydrazineylthiazole hydrochloride

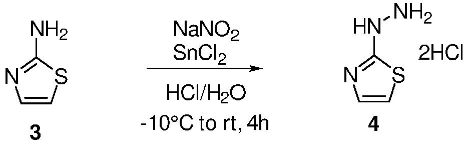

### Intermediate 2. Synthesis of tert-butyl (S)-3-amino-5-(3,3-difluoropiperidin-1-yl)pentanoate (11)

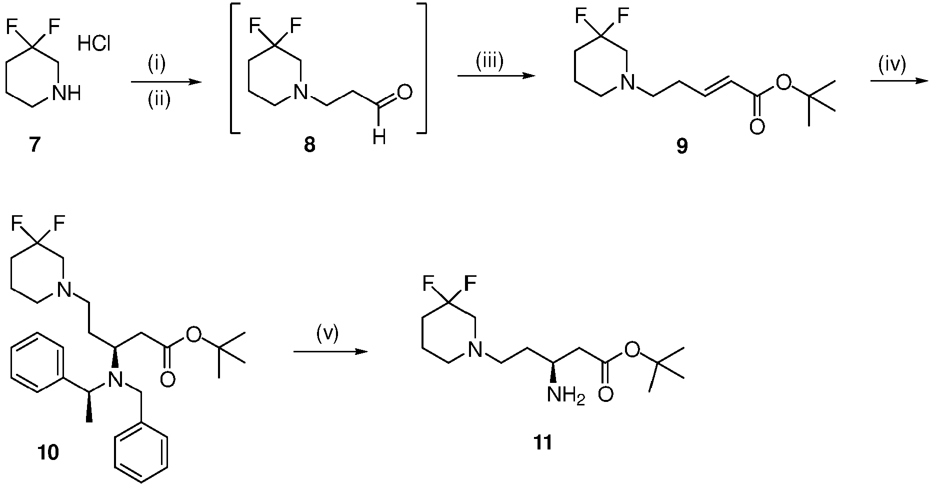

***Reagents** and conditions:* (i) KOtBu, EtOH, rt; (ii) acrolein, DBU, THF, 0 °C; (iii) tert-butyl diethylphosphonoacetate, KOtBu, THF, 0 °C, 30 min, rt, 1 h; (iv) (*S*)-*N*-benzyl-*N*-α-methylbenzylamine, BuLi, THF, -78 °C; (v) 10% Pd/C, H₂, 45 psi, MeOH, DCM, rt.

Based upon the general teachings of the present disclosure, the following compounds of the present invention were made:

**Exemplification Table**

| **#** | **Compound** | **Exact Mass** | **Observed Mass (M + H)+** | **lupac Name** |
|---|---|---|---|---|
| 1 | 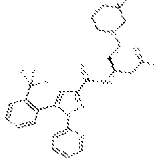 | 551.1955807 | 552.2 | (3S)-5-(3,3-difluoropiperidin-1-yl)-3-{[1-(pyridin-2-yl)-5-[2-(trifluoromethyl)phenyl]-1H-pyrazol-3-yl]formamido}pentanoic acid |
| 2 | 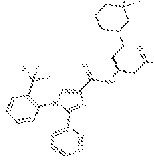 | 551.1955807 | 552.4 | (3S)-5-(3,3-difluoropiperidin-1-yl)-3-{[2-(pyridin-3-yl)-1-[2-(trifluoromethyl)phenyl]-1H-imidazol-4-yl]formamido}pentanoic acid |
| 3 | 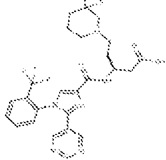 | 552.1908297 | 553.3 | (3S)-5-(3,3-difluoropiperidin-1-yl)-3-{[2-(pyrimidin-5-yl)-1-[2-(trifluoromethyl)phenyl]-1H-imidazol-4-yl]formamido}pentanoic acid |
| 4 | 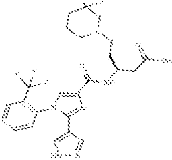 | 540.1908297 | 541.3 | (3S)-5-(3,3-difluoropiperidin-1-yl)-3-{[2-(1H-pyrazol-4-yl)-1-[2-(trifluoromethyl)phenyl]-1H-imidazol-4-yl]formamido}pentanoic acid |
| 5 | 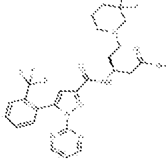 | 552.1908297 | 553.2 | (3S)-5-(3,3-difluoropiperidin-1-yl)-3-{[1-(pyrimidin-2-yl)-5-[2-(trifluoromethyl)phenyl]-1H-pyrazol-3-yl]formamido}pentanoic acid |
| 6 | 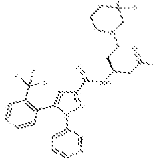 | 551.1955807 | 552.2 | (3S)-5-(3,3-difluoropiperidin-1-yl)-3-{[1-(pyridin-3-yl)-5-[2-(trifluoromethyl)phenyl]-1H-pyrazol-3-yl]formamido}pentanoic acid |
| 7 | 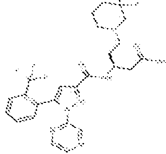 | 552.1908297 | 553.1 | (3S)-5-(3,3-difluoropiperidin-1-yl)-3-{[1-(pyrazin-2-yl)-5-[2-(trifluoromethyl)phenyl]-1H-pyrazol-3-yl]formamido}pentanoic acid |
| 8 | 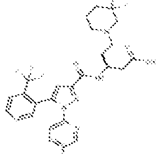 | 569.1861589 | 570.6 | (3S)-5-(3,3-difluoropiperidin-1-yl)-3-{[1-(5-fluoropyridin-2-yl)-5-[2-(trifluoromethyl)phenyl]-1H-pyrazol-3-yl]formamido}pentanoic acid |
| 9 | 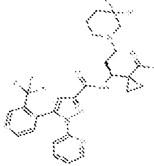 | 577.2112308 | 578.1 | 1-[(1 S)-3-(3,3-difluoropiperidin-1-yl)-1-{[1-(pyridin-2-yl)-5-[2-(trifluoromethyl)phenyl]-1H-pyrazol-3-yl]formamido}propyl]cyclopropane-1-carboxylic acid |
| 10 | 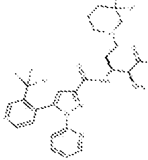 | 565.2112308 | 566.1 | (2S,3S)-5-(3,3-difluoropiperidin-1-yl)-2-methyl-3-{[1-(pyridin-2-yl)-5-[2-(trifluoromethyl)phenyl]-1H-pyrazol-3-yl]formamido}pentanoic acid |
| 11 | 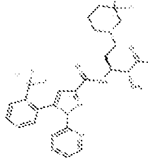 | 565.2112308 | 566.1 | (2R,3S)-5-(3,3-difluoropiperidin-1-yl)-2-methyl-3-{[1-(pyridin-2-yl)-5-[2-(trifluoromethyl)phenyl]-1H-pyrazol-3-yl]formamido}pentanoic acid |
| 12 | 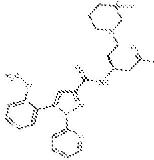 | 513.2187609 | 514 | (3S)-5-(3,3-difluoropiperidin-1-yl)-3-{[5-(2-methoxyphenyl)-1-(pyridin-2-yl)-1H-pyrazol-3-yl]formamido}pentanoic acid |
| 13 | 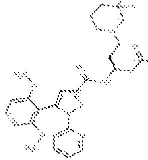 | 543.2293255 | 544.1 | (3S)-5-(3,3-difluoropiperidin-1-yl)-3-{[5-(2,6-dimethoxyphenyl)-1-(pyridin-2-yl)-1H-pyrazol-3-yl]formamido}pentanoic acid |
| 14 | 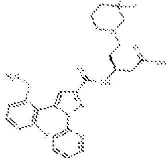 | 531.209339 | 532.0 | (3S)-5-(3,3-difluoropiperidin-1-yl)-3-{[5-(2-fluoro-6-methoxyphenyl)-1-(pyridin-2-yl)-1H-pyrazol-3-yl]formamido}pentanoic acid |
| 15 | 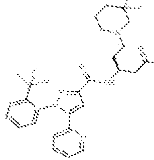 | 551.1955807 | 552.0 | (3S)-5-(3,3-difluoropiperidin-1-yl)-3-{[5-(pyridin-2-yl)-1-[2-(trifluoromethyl)phenyl]-1H-pyrazol-3-yl]formamido}pentanoic acid |
| 16 | 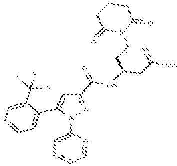 | 543.1729535 | 544.0 | (3S)-5-(2,6-dioxopiperidin-1-yl)-3-{[1-(pyridin-2-yl)-5-[2-(trifluoromethyl)phenyl]-1H-pyrazol-3-yl]formamido}pentanoic acid |
| 17 | 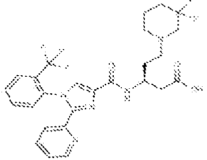 | 551.1955807 | 552.1 | (3S)-5-(3,3-difluoropiperidin-1-yl)-3-{[2-(pyridin-2-yl)-1-[2-(trifluoromethyl)phenyl]-1H-imidazol-4-yl]formamido}pentanoic acid |
| 18 | 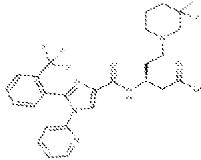 | 551.1955807 | 552.0 | (3S)-5-(3,3-difluoropiperidin-1-yl)-3-{[1-(pyridin-2-yl)-2-[2-(trifluoromethyl)phenyl]-1H-imidazol-4-yl]formamido}pentanoic acid |
| 19 | 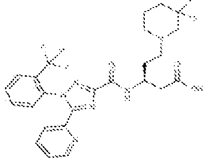 | 552.1908297 | 553.1 | (3S)-5-(3,3-difluoropiperidin-1-yl)-3-{[5-(pyridin-2-yl)-1-[2-(trifluoromethyl)phenyl]-1H-1,2,4-triazol-3-yl]formamido}pentanoic acid |
| 20 | 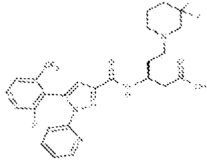 | 569.1861589 | 570.1 | (3S)-5-(3,3-difluoropiperidin-1-yl)-3-({5-[2-fluoro-6-(trifluoromethyl)phenyl]-1-(pyridin-2-yl)-1H-pyrazol-3-yl}formamido)pentanoic acid |
| 21 | 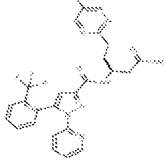 | 528.1533014 | 529.1 | (3S)-5-(5-fluoropyrimidin-2-yl)-3-{[1-(pyridin-2-yl)-5-[2-(trifluoromethyl)phenyl]-1H-pyrazol-3-yl]formamido}pentanoic acid |
| 22 | 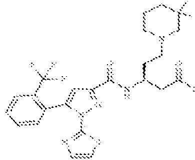 | 557.1520014 | 558.6 | (3S)-5-(3,3-difluoropiperidin-1-yl)-3-{[1-(1,3-thiazol-2-yl)-5-[2-(trifluoromethyl)phenyl]-1H-pyrazol-3-yl]formamido}pentanoic acid |
| 23 | 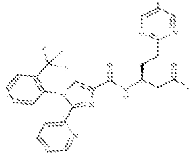 | 528.1533014 | 529.2 | (3S)-5-(5-fluoropyrimidin-2-yl)-3-{[2-(pyridin-2-yl)-1-[2-(trifluoromethyl)phenyl]-1H-imidazol-4-yl]formamido}pentanoic acid |
| 24 | 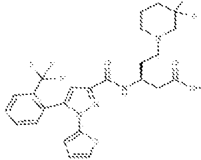 | 556.1567524 | 557.6 | (3S)-5-(3,3-difluoropiperidin-1-yl)-3-{[1-(thiophen-2-yl)-5-[2-(trifluoromethyl)phenyl]-1H-pyrazol-3-yl]formamido}pentanoic acid |
| 25 | 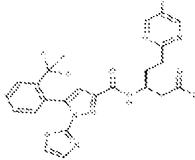 | 534.109722 | 535.1 | (3S)-5-(5-fluoropyrimidin-2-yl)-3-{[1-(1,3-thiazol-2-yl)-5-[2-(trifluoromethyl)phenyl]-1H-pyrazol-3-yl]formamido}pentanoic acid |
| 26 | 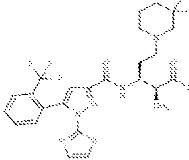 | 571.1676514 | 572 | (2S,3S)-5-(3,3-difluoropiperidin-1-yl)-2-methyl-3-{[1-(1,3-thiazol-2-yl)-5-[2-(trifluoromethyl)phenyl]-1H-pyrazol-3-yl]formamido}pentanoic acid |
| 27 | 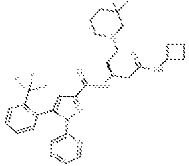 | 604.2585154 | 605 | (3S)-N-cyclobutyl-5-(3,3-difluoropiperidin-1-yl)-3-{[1-(pyridin-2-yl)-5-[2-(trifluoromethyl)phenyl]-1H-pyrazol-3-yl]formamido}pentanamide |
| 28 | 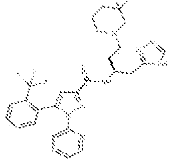 | 574.2227986 | 575 | N-[(2S)-4-(3,3-difluoropiperidin-1-yl)-1-(4H-1,2,4-triazol-3-yl)butan-2-yl]-1-(pyridin-2-yl)-5-[2-(trifluoromethyl)phenyl]-1H-pyrazole-3-carboxamide |
| 29 | 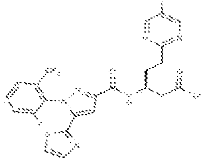 | 552.1003002 | 553.0 | (3S)-3-({1-[2-fluoro-6-(trifluoromethyl)phenyl]-5-(1,3-thiazol-2-yl)-1H-pyrazol-3-yl}formamido)-5-(5-fluoropyrimidin-2-yl)pentanoic acid |
| 30 | 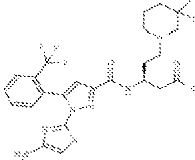 | 571.1676514 | 572.1 | (3S)-5-(3,3-difluoropiperidin-1-yl)-3-{[1-(4-methyl-1,3-thiazol-2-yl)-5-[2-(trifluoromethyl)phenyl]-1H-pyrazol-3-yl]formamido}pentanoic acid |
| 31 | 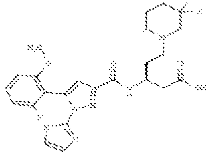 | 537.1657597 | 538.3 | (3S)-5-(3,3-difluoropiperidin-1-yl)-3-{[5-(2-fluoro-6-methoxyphenyl)-1-(1,3-thiazol-2-yl)-1H-pyrazol-3-yl]formamido}pentanoic acid |
| 32 | 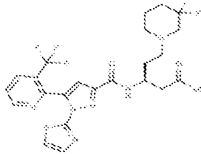 | 558.1472503 | 559.3 | (3S)-5-(3,3-difluoropiperidin-1-yl)-3-{[1-(1,3-thiazol-2-yl)-5-[3-(trifluoromethyl)pyridin-2-yl]-1H-pyrazol-3-yl]formamido}pentanoic acid |
| 33 | 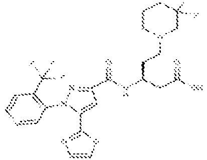 | 541.1748453 | 542 | (3S)-5-(3,3-difluoropiperidin-1-yl)-3-{[5-(1,3-oxazol-2-yl)-1-[2-(trifluoromethyl)phenyl]-1H-pyrazol-3-yl]formamido}pentanoic acid |
| 34 | 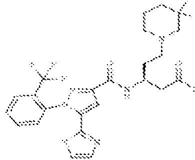 | 557.1520014 | 558 | (3S)-5-(3,3-difluoropiperidin-1-yl)-3-{[5-(1,3-thiazol-2-yl)-1-[2-(trifluoromethyl)phenyl]-1H-pyrazol-3-yl]formamido}pentanoic acid |
| 35 | 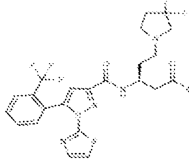 | 543.1363513 | 544 | (3S)-5-(3,3-difluoropyrrolidin-1-yl)-3-{[1-(1,3-thiazol-2-yl)-5-[2-(trifluoromethyl)phenyl]-1H-pyrazol-3-yl]formamido}pentanoic acid |
| 36 | 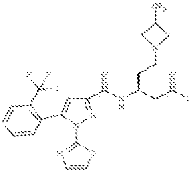 | 561.1269295 | 562 | (3S)-3-{[1-(1,3-thiazol-2-yl)-5-[2-(trifluoromethyl)phenyl]-1H-pyrazol-3-yl]formamido}-5-[3-(trifluoromethyl)azetidin-1-yl]pentanoic acid |
| 37 | 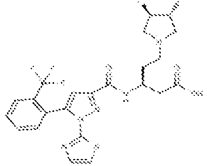 | 543.1363513 | 544 | (3S)-5-[(3R,4R)-3,4-difluoropyrrolidin-1-yl]-3-{[1-(1,3-thiazol-2-yl)-5-[2-(trifluoromethyl)phenyl]-1H-pyrazol-3-yl]formamido}pentanoic acid |
| 38 | 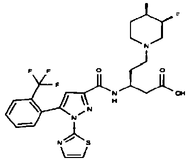 | 557.1520014 | 558 | (3S)-5-[(3S,4R)-3,4-difluoropiperidin-1-yl]-3-{[1-(1,3-thiazol-2-yl)-5-[2-(trifluoromethyl)phenyl]-1H-pyrazol-3-yl]formamido}pentanoic acid |

### Characterization of the Apelin Agonist Activity of the Compounds

The compounds above were studied for their *in vitro* activity as apelin agonists using the methods described by Giddings *et al.* Giddings et al., 2010 Int J High Thro Screen. 1:39-47.

### Cellular Uptake Assay

Caco-2 cells (clone C2BBe1) were obtained from American Type Culture Collection (Manassas, VA). Cell monolayers were grown to confluence on collagen-coated, microporous, polycarbonate membranes in 12-well Costar Transwell plates. Details of the plates and their certification are shown below. The permeability assay buffer was Hanks' balanced salt solution (HBSS) containing 10 mM HEPES and 15 mM glucose at a pH of 7.4. The buffer in the receiver chamber also contained 1% bovine serum albumin. The dosing solution concentration was 5 µM for each test article in the assay buffer. Cell monolayers were dosed on the apical side (A-to-B) or basolateral side (B-to-A) and incubated at 37 °C with 5% CO₂ in a humidified incubator. Samples were taken from the donor and receiver chambers at 120 minutes. Each determination was performed in duplicate. After the experiment, all assay buffers were removed from the inserts. Cell monolayers were dosed with blank 500 µM lucifer yellow on the A-to-B side and blank HBSS on the B-to-A side and incubated at 37 °C. Samples were taken from the B-to-A side at 60 minutes. The flux of lucifer yellow was measured for each monolayer to ensure no damage was inflicted to the cell monolayers during the flux period. All samples were analyzed by LC-MS/MS using electrospray ionization. The apparent permeability (Pₐₚₚ) and percent recovery were calculated as follows: ${P}_{app} = \left(d{C}_{r}/dt\right) \times {V}_{r} / \left(A\times {C}_{A}\right)$ $Percent Recovery = 100 \times \left(\left({V}_{r}\times {C}_{r}^{final}\right)+\left({V}_{d}\times {C}_{d}^{final}\right)\right) / \left({V}_{d}\times {C}_{N}\right)$
Where, dCᵣ /dt is the slope of the cumulative concentration in the receiver compartment versus time in µM s⁻¹;
Vᵣ is the volume of the receiver compartment in cm³;
V_{d} is the volume of the donor compartment in cm³;
A is the area of the insert (1.13 cm² for 12-well Transwell);
C_{A} is the average of the nominal dosing concentration and the measured 120-minute donor concentration in µM;
C_{N} is the nominal concentration of the dosing solution in µM;
Cᵣ^{final} is the cumulative receiver concentration in µM at the end of the incubation period;
C_{d}^{final} is the concentration of the donor in µM at the end of the incubation period. $Efflux ratio \left(ER\right) is defined as {P}_{app} \left(B-to-A\right) / {P}_{app} \left(A-to-B\right) .$

### Absorption Potential Classification:

| | |
|---|---|
| Pₐₚₚ (A-to-B) < 1.0 × 10⁻⁶ cm/s: | Low |
| Pₐₚₚ (A-to-B) ≥ 1.0 × 10⁻⁶ cm/s: | High |
| Significant Efflux is defined as: | ER ≥ 2.0 and Pₐₚₚ (B-to-A) ≥ 1.0 × 10⁻⁶ cm/ |

### Dose Dependent Efficacy in a Chronic (21-Day) Bleomycin-induced Lung Fibrosis Model in Male C57B/L6 Mice

Bleomycin is widely used to induce pulmonary fibrosis in rodents in order to study potential novel therapies for fibrosis. This study was designed to evaluate the dose-dependent efficacy of test compounds, in a 21-day model of bleomycin-induced pulmonary fibrosis in male C57BL/6 mice. One compound is EXAMPLE 22.

The study included both prophylactic and therapeutic arms to evaluate test compound (EXAMPLE 22) to inhibit and to treat BLEO-induced lung fibrosis. Pirfenidone (PIRF) was evaluated as a reference agent in the study.

Mice incepted oral (p.o.) Px dosing of one of three dose levels of EXAMPLE 22 (twice daily, BID; 15, 30, or 60 mg/kg/day) or PIRF (p.o., BID; 200 mg/kg/day) one day prior to BLEO instillation; oral Tx dosing of EXAMPLE 22 (p.o. BID; 60 mg/kg/day) incepted 5 days post-BLEO instillation. Mice continued p.o. BID dosing for the remainder of the study.

Neither EXAMPLE 22 nor PIRF affected serial BW, final BW, or ΔBW relative to Vehicle-treated BLEO-instilled controls.

Neither EXAMPLE 22 nor PIRF affected endpoint lung weight (LW) or LW indexed to tibia length (LW:TL) relative to Vehicle-treated BLEO-instilled controls.

Neither EXAMPLE 22 nor PIRF affected endpoint lung OH-P content relative to Vehicle-treated BLEO-instilled controls.

Reductions in lung collagen content were observed with the administration of 15 mpk Px EXAMPLE 22 (TCF) and Px PIRF (CVF and TCF) relative to Vehicle-treated controls.

Overall, EXAMPLE 22 showed positive impact on one or more end-point readouts for fibrosis that were evaluated in the study. Prophylactic treatment with EXAMPLE 22 twice daily improved the overt symptoms associated with bleomycin administration in this 21-day non-GLP study. EXAMPLE 22 at 30 mg/kg appeared somewhat more effective. The administration of EXAMPLE 22 reduced absolute and normalized lung weights and decreased total number of leukocytes from bronchoalveolar lavage (BAL) fluid.

The objective of this non-GLP study was to evaluate the prophylactic efficacy of EXAMPLE 22 in a 21-day model of bleomycin-induced pulmonary fibrosis in mice.

### Materials and Methods

Mice were administered bleomycin (Catalog number C-61703-323-22, lot number D011495AA, Hospira) via oropharyngeal route to induce lung fibrosis.

Bleomycin-induced animals were treated twice daily with either of the three different doses of EXAMPLE 22 or pirfenidone starting from a day prior to disease induction and continued till study termination. Study animals were harvested on day 21 post-bleomycin administration. As an end point analysis, fibrosis symptoms such as body weight, absolute lung weight, lung weight normalized to body weight, and total leukocytes in BAL fluid were evaluated and compared with vehicle-treated mice.

### Study Animals

One week prior to study initiation, 88 C57BL/6 mice, six to eight weeks of age were obtained from Simonsen Laboratories (Gilroy, CA). Animals were weighed prior to study initiation and 60 animals were randomized into groups such that mean body weights were similar for the different groups. Remaining animals with lower or higher body weight were not included into the study. Food and water was provided *ad libitum,* with a light/dark cycle of 12 hours.

A summary is shown in Table 1 PBI-19-065 Study Design:

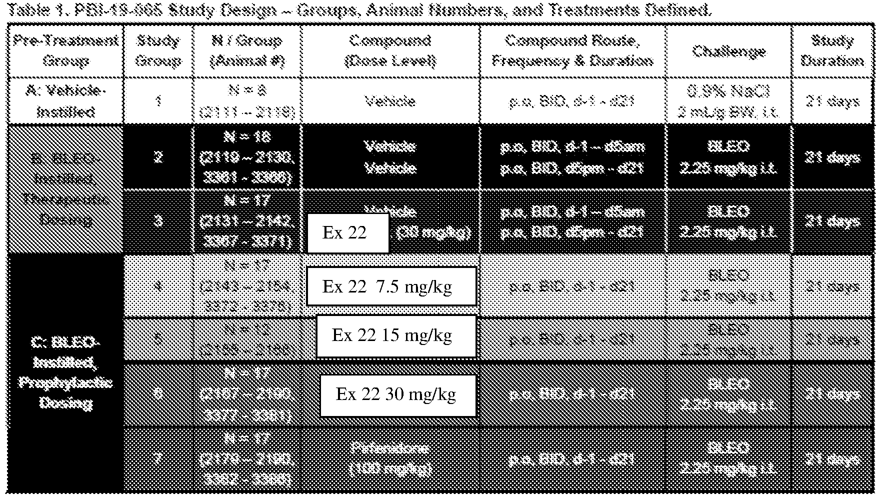

### Experimental Procedures

### In Life Phase of Study

To control for variances in animal age and weight, prior to the inception of study, mice were weighed, sorted heaviest-to-lightest, and placed into balanced enrollment groups with the heaviest animals enrolled first. The current study employed both prophylactic (Px) and therapeutic (Tx) study arms to both evaluate the ability of EXAMPLE 22 to inhibit and treat, respectively, BLEO-induced lung fibrosis. Thus, mice in the Px arm of the study (Groups 4 - 7) had compound dosing commence one day prior (D-1) to BLEO instillation while mice in the Tx arm of the study (Group 3) had compound dosing commence 5 days (D5) post-BLEO instillation. Since PBI's best practices are to use data whenever possible to place animals to treatment groups, the study was arranged into "Pre-Groups", as detailed in Table 1, such that some mice were orally dosed twice daily (p.o., BID: 10 mL/kg) with Vehicle (Pre-Groups A and B) on D-1, while others (Pre-Group C) received an incremental doses of EXAMPLE 22 (7.5, 15, or 30 mg/kg (mpk) /dose) or a single dose-level (100 mpk/dose) of PIRF on D-1. Then, on D0, Pre-Group A received i.t. instillation of either 0.9% NaCl while mice in Pre-Groups B and C received i.t. instillation of BLEO, as described in Section 3.2 above. On D5 post-BLEO instillation, animals in Pre-Group B were weighed and grouped based on that day's body weight (BW) and body weight change from date of BLEO instillation (ΔBW), then enrolled into balanced vehicle or EXAMPLE 22 treatment groups based on those parameters. On D20, mice received their morning dose of vehicle or compound on a timetable to enable timed blood collection for determination of compound exposure as described in Section 3.3.2 below. On D21, mice were dosed on a timetable to enable terminal blood and tissue collection ~2 hours post-final dosing. Then, mice were anesthetized with isoflurane and had an endotracheal tube placed and interfaced with a positive pressure ventilator. Each mouse's chest was then opened, the diaphragm was sectioned, and the abdominal contents were displaced to reveal the abdominal great vessels. The inferior vena cava and abdominal aorta were sectioned and a needle interfaced with an infusion pump was introduced to the right ventricle. While still ventilating, the infusion pump was engaged to perfuse the pulmonary vasculature with oxygenated 0.9% NaCl and remove all blood from the lung. The entire heart-lung pluck was then harvested. After removal of the heart and extraneous tissue, wet lung weights were recorded, the lungs were immediately immersed in ice-cold (4°C) 0.9% NaCl, and then processed for the collection of bronchoalveolar lavage fluid (BALF). The lungs were then gross-dissected, the left lung was inflation-fixed for subsequent histological analyses, and the right lung was flash-frozen on liquid nitrogen for biochemical analyses as detailed in Section 3.3.3. Tibias were dissected and lengths recorded in order to express indexed lung ratio.

### Pharmacokinetic Samples

To enable determination of dose-dependent pharmacokinetics in the setting of BLEO-induced lung injury, on D20, 150 µL of whole blood were collected on K3EDTA from two mice per BLEO group at each of six prescribed times post-compound administration (0.5, 1, 2, 3, 4, and 6 hours) via conscious tail venipuncture and processed appropriately to produce plasma. At endpoint (D21), in concert with exsanguination and sacrifice, 200 µL of whole blood were collected from all BLEO groups on K3EDTA ~2 hours following final compound administration, to reflect peak compound concentration, and processed appropriately to produce plasma. Time of blood collection relative to final compound dosing was recorded_and provided to the Study Sponsor.

For each collection, one 75 - 100 µL plasma sample was aliquoted to pre-labelled microcentrifuge tubes, immediately flash-frozen on liquid N2, and stored at -80 °C until shipped to the Study Sponsor on dry ice.

### Endpoint Procedures

### BALF Sample Preparation

At study endpoint (D21), following exsanguination and flushing of the pulmonary circulation to clear blood, the entire lung was immediately removed and placed in ice-cold (4 °C) 0.9% NaCl. The trachea was cannulated with PE-50 tubing connected to a syringe containing cold lavage solution (1X Hank's Balanced Salt Solution, HBSS). The trachea was secured to the cannula with a ligature and the entire lung lavaged at least three times with 1.5 mL lavage solution. Bronchoalveolar lavage fluid (BALF) was collected and placed on wet ice until processed as described in below. BALF was centrifuged for 10 min at 500 x g at 4 °C. The supernatant was transferred to new tubes (Cell-Free BALF fraction), being careful not to disturb the cell pellet. Cell-free BALF was prepared to 2 x 200 µL aliquots and flash frozen on liquid N2. The BALF cells were pelleted with supernatant removed and frozen on liquid N2. BALF Cell and Cell-Free fractions were stored at -80 °C until shipped to the Study Sponsor on dry ice.

### Biochemical Sample Preparation

The right and left main bronchi were separated with the right bronchus ligated. The right bronchus was sectioned and the right lung lobes immediately frozen on liquid N2 and stored at -80 °C until undergoing processing for biochemical analysis. The left lung was processed for histological analyses as described hereinbelow. The right lung was cryopowdered using a mortar and pestle on liquid N2 to ensure homogeneity for all subsequent biochemical analyses. Samples from each group were aliquoted into two tubes: i) 20 mg for the determination of hydroxyproline (OH-P) content, as described in Section 3.3.4; ii) 40 - 45 mg banked (-80°C) for future potential biochemical analysis.

### Histological Sample Preparation

After obtaining whole lung wet weight, a short length of PE-50 tubing connected to a needle hub (23G) and a 3 mL syringe filled with fixative (10% neutral buffered formalin, NBF) was inserted into the left bronchus, secured with tied suture, and the left lung was gently inflated until it was fully, uniformly, and consistently expanded (but fixative was not permeating through lung surface). The needle was then removed, the bronchus ligated, and the inflated left lung was immersed in 10% NBF for 48 hours. After 48 hours of fixation, the suture was removed, NBF was gently drained from the lung, and the entire lung was transferred to 70% alcohol. Following conclusion of the in-life phase of study, all histological tissues were processed and paraffin embedded until sectioned, mounted, and stained.

### Biochemical Phase of Study

Hydroxyproline (OH-P) Analysis - Right lungs were cryopowdered using a mortar and pestle over liquid nitrogen and aliquots (~20 mg) were weighed and lysed in dH2O using the bead-based TissueLyzer II homogenizer (Qiagen, Valencia CA). Tissue lysates were maintained on wet ice for the duration of assay performance. Tissue lysate protein concentration was determined. Lysates were then vortexed and 100 µL were added to a 2 mL polypropylene screw top tube followed by the addition of 100 µL of 12N HCl. Samples were hydrolyzed overnight by incubation in a 110 ºC oven. Hydrolysates were brought to room temperature (RT) and centrifuged for 5 min at 13,000 x g. Stock OH-P solution (cis-4-Hydroxy-L-proline) was diluted to 0.1 mg/mL in 6N HCl. Then, 2 - 0.056 µg of OH-P was added to duplicate wells in a clear 96-well microtiter plate in a total volume of 10 µL 6N HCl to serve as standards. The hydrolysates (10 µL) were then added to duplicate wells and the plate was placed under vacuum for approximately 5 hours at RT until the complete desiccation of samples and standards. 100 µL of Chloramine T solution (Chloramine T in an n-propanol, citrate-acetate buffer) were added to all wells and the plate was incubated at RT for 20 min on a small orbital shaker. Then, 100 µL of Ehrlich's Reagent were added to all wells and the plate sealed and incubated at 65 ºC for 20 min. The plate was brought to RT and then optical densities (O.D.s) were measured at 560 nm on a SpectraMax 190 plate reader (Molecular Devices, Sunnyvale CA). O.D.s were background corrected against blank samples and an 8-point standard curve for conversion of O.D.s to mass was determined using a 4-parameter curve-fit method using SoftMax Pro5 software (Molecular Devices, Sunnyvale CA).

All remaining cryopowdered lung tissue was maintained at -80 °C until shipped to the Study Sponsor on dry ice.

### Histological Phase of Study

Following fixation and transfer to EtOH, samples were paraffin embedded, sectioned, mounted, and stained as described below.

Four serial histological sections, referred to as sections A - D, were obtained at each of three anatomically-distinct regions (apical, mid-, and basal lung regions) per animal with an ordered section from each region mounted to an individual slide.

Slides were then stained as follows: i) a slide containing Section B from each anatomical region was subjected to staining with Masson's Trichrome Blue (MTB) for assessment of collagen content; *ii)* a slide containing Section C from each anatomical region was subjected to staining with Hematoxylin & Eosin (H&E); and *iii)* the remaining two serial sections (sections C and D) from each anatomically distinct region was mounted on slides and submitted to the Study Sponsor for future potential immunohistochemical staining.

The following analyses were then performed on the MTB and H&E- stained slides:

Collagen Volume Fraction (CVF; collagen positively-stained tissue per histological field) and Tissue Collagen Fraction (TCF; collagen positive-stained tissue per amount of histological tissue) was determined by quantitative histological analysis of MTB-stained sections using standard image acquisition and analysis techniques. Data for each histological depth as well as data representing a Composite (average of all 3 histological depths) value for each mouse in study were generated.

Ashcroft Score was determined by evaluation of H&E stained slides. Findings were provided in a separate formal Pathology Report inclusive of 2 photomicrographs (2x and 10x) per group.

Remaining paraffin embedded tissue blocks were shipped to Study Sponsor following conclusion histological analysis.

### Statistical Analysis

Data are expressed as mean ± SEM. Statistical analyses were performed using GraphPad Prism 8.2.1 for Windows (GraphPad Software, Inc., San Diego, CA) and are depicted in Table 2. Outliers were identified in the biochemical and histological data as individual values above or below a threshold of three times the standard deviation from the group mean and are flagged in the tabular data and excluded from the graphical data. Graphical data depicting the inclusion of all animals, including outliers, are depicted in the present disclosure.

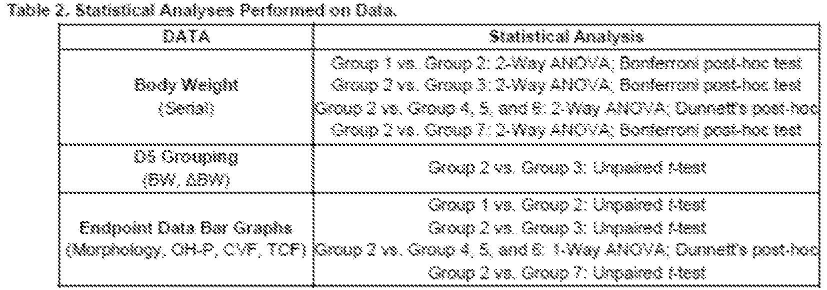

### RESULTS

### Day 5 Treatment Grouping Body Weight Data

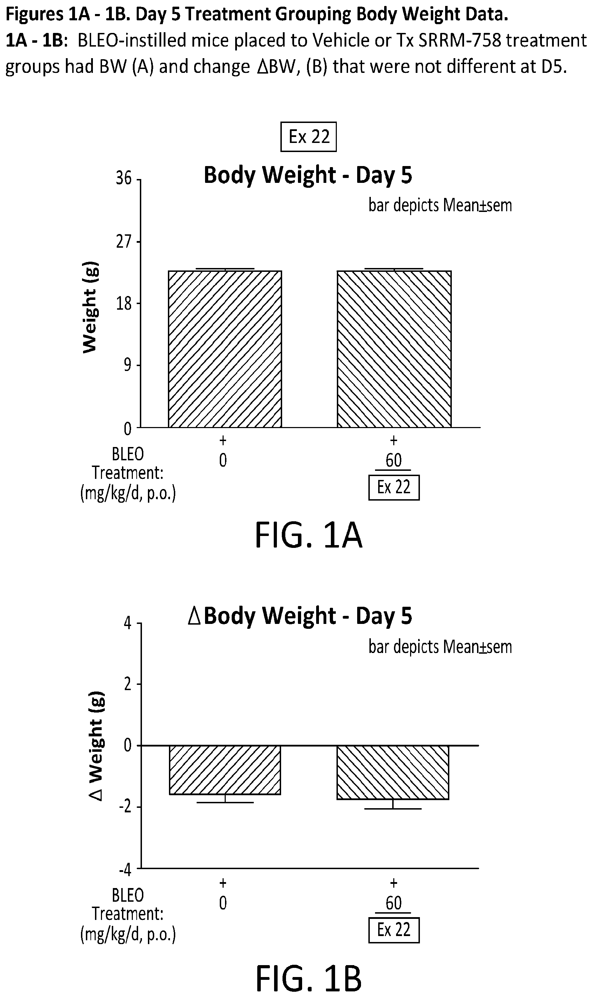

Summary graphical and tabular Day (D)5 body weight (BW) grouping data are depicted in Figures 1A - 1B. There were no differences in body weight (BW, Figure 1A) or change in D0 - D5 body weights (ΔBW, Figure 1B) between BLEO-instilled mice placed to Vehicle or Tx EXAMPLE 22 treatment groups at D5.

### Serial Body Weight Data

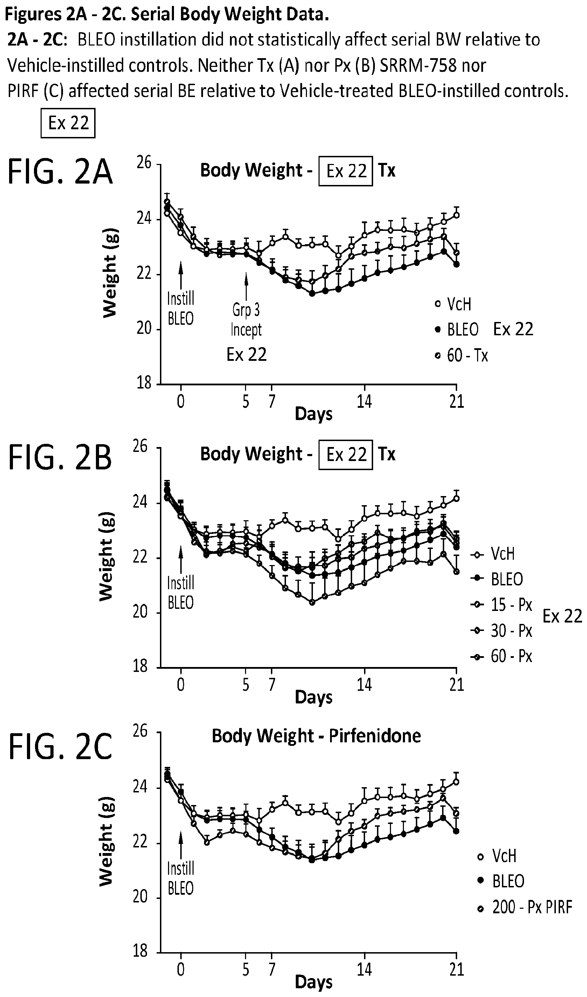

Summary graphical and tabular body weight data are depicted in Figures 2A - 2C. BLEO instillation did not affect serial BW over the course of the study relative to Vehicle-instilled controls. Neither Tx (Figure 2A) nor Px (Figure 2B) EXAMPLE 22 nor PIRF (Figure 2C) affected BW over the course of the study relative to Vehicle-treated BLEO-instilled controls.

### Survival Data

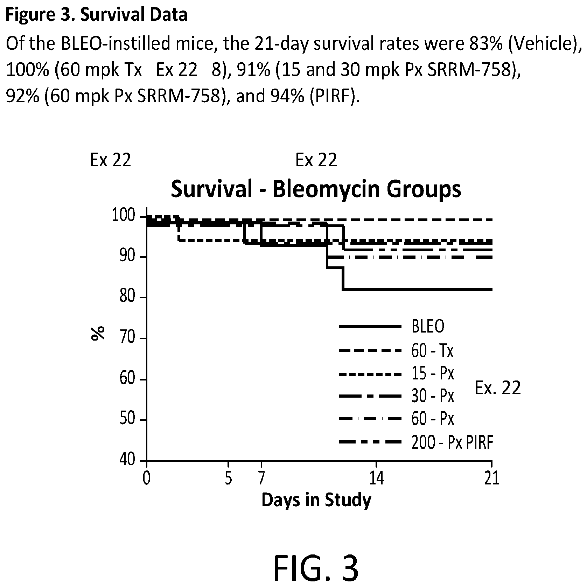

Summary graphical survival data are depicted in Figure 3. Of the BLEO-instilled mice, the 21-day survival rates were 83% (Vehicle), 100% (Tx EXAMPLE 22), 91% (15 and 30 mpk Px EXAMPLE 22), 92% (60 mpk EXAMPLE 22), and 94% (PIRF).

### Endpoint Morphological Data

Summary graphical and tabular endpoint morphology data are depicted in Figures 4A - 4E. Consistent with weight-matched grouping upon study enrollment, there was no difference in initial body weight (BW, Figure 4A) prior to vehicle or BLEO instillation across all groups.

BLEO instillation reduced final BW (Figure 4B), and D0 - D21 ΔBW (Figure 4C) relative to Vehicle-instilled controls (BW: 22.37 ± 0.45 vs. 24.15 ± 0.32 g; and ΔBW: -2.17 ± 0.38 vs. 0.03 ± 0.30 g). No compound tested affected BW or ΔBW relative to Vehicle-treated BLEO-instilled animals.

BLEO-instillation increased lung weight (LW, Figure 4D) and LW indexed to tibia length (LW:TL, Figure 4E) relative to Vehicle-instilled controls (LW: 243.16 ± 19.74 vs. 146.24 ± 5.26 mg; and LW:TL: 14.13 ± 1.18 vs. 8.50 ± 0.30 mg/mm). No compound tested affected LW or LW:TL relative to Vehicle-treated BLEO-instilled animals.

### Endpoint Lung Hydroxyproline (OH-P) Content Data

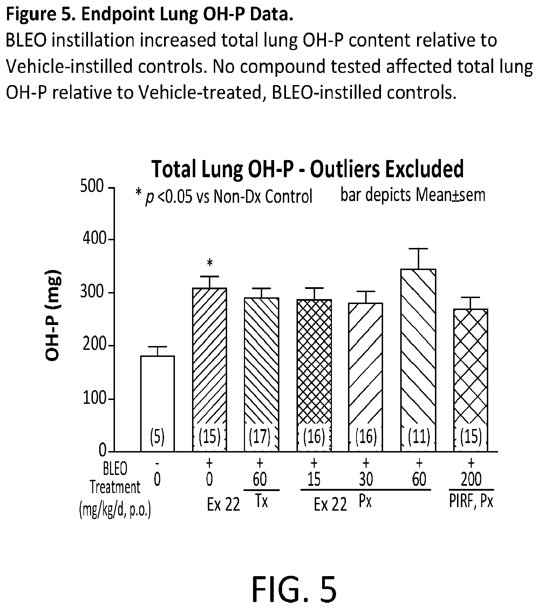

Summary graphical and tabular lung hydroxyproline (OH-P) data are depicted in Figure 5. BLEO instillation increased total lung OH-P content relative to Vehicle-instilled controls (308.89 ± 23.83 vs. 183.77 ± 17.04 µg/lung). No compound tested affected total lung OH-P relative to Vehicle-treated, BLEO-instilled controls.

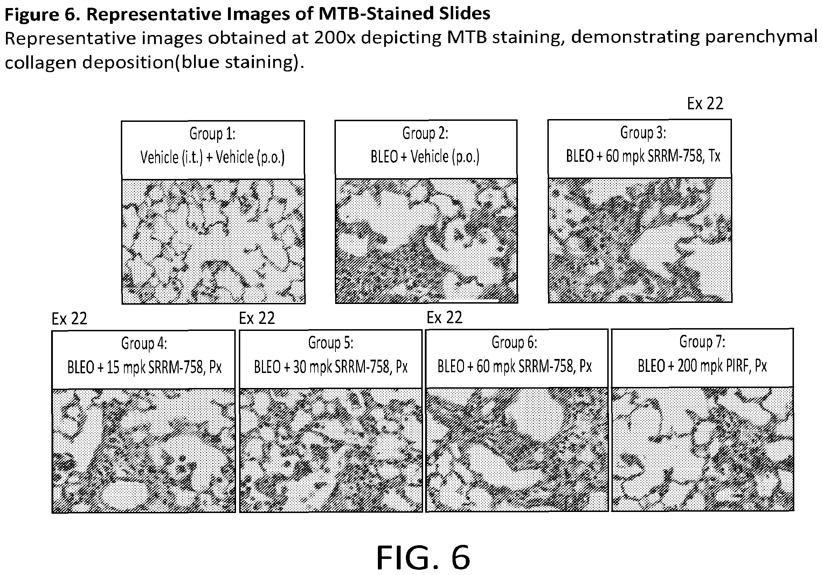

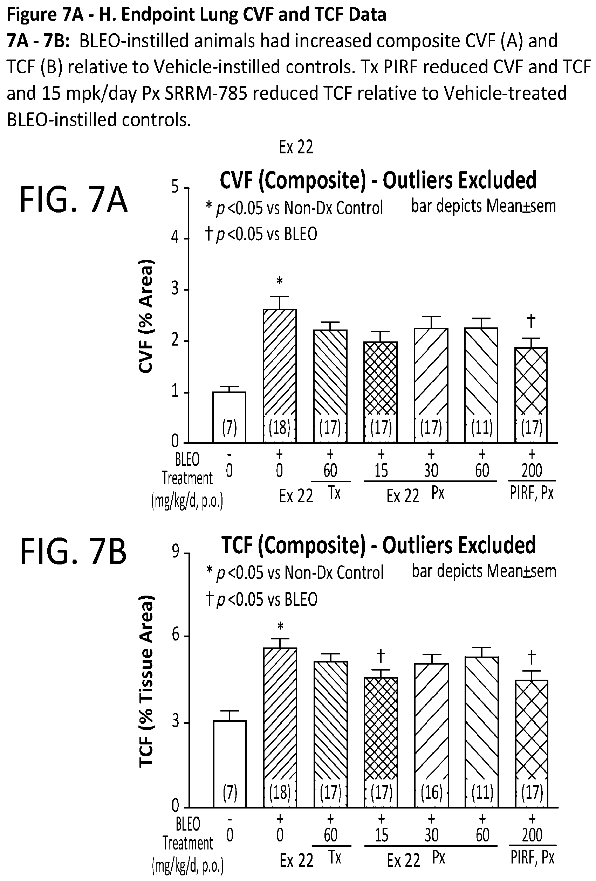

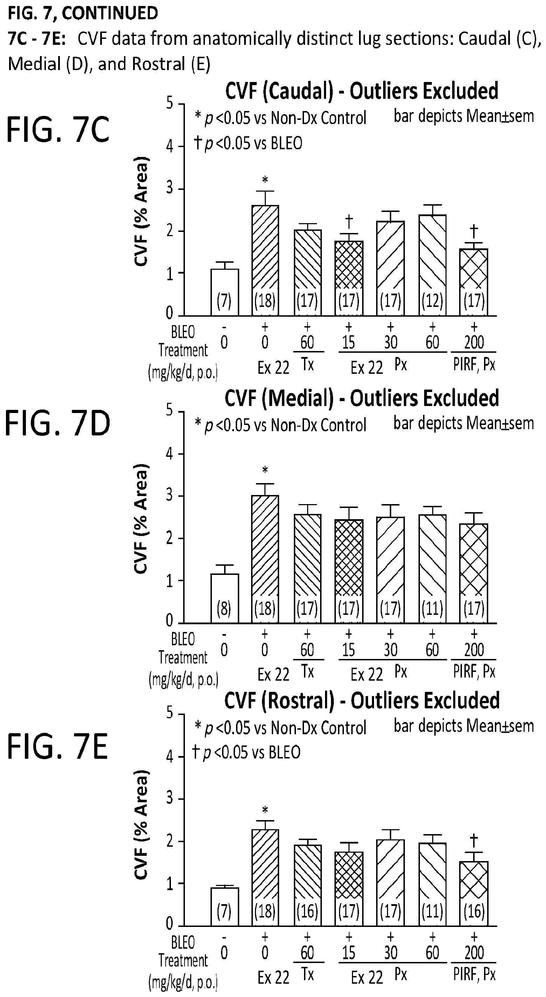

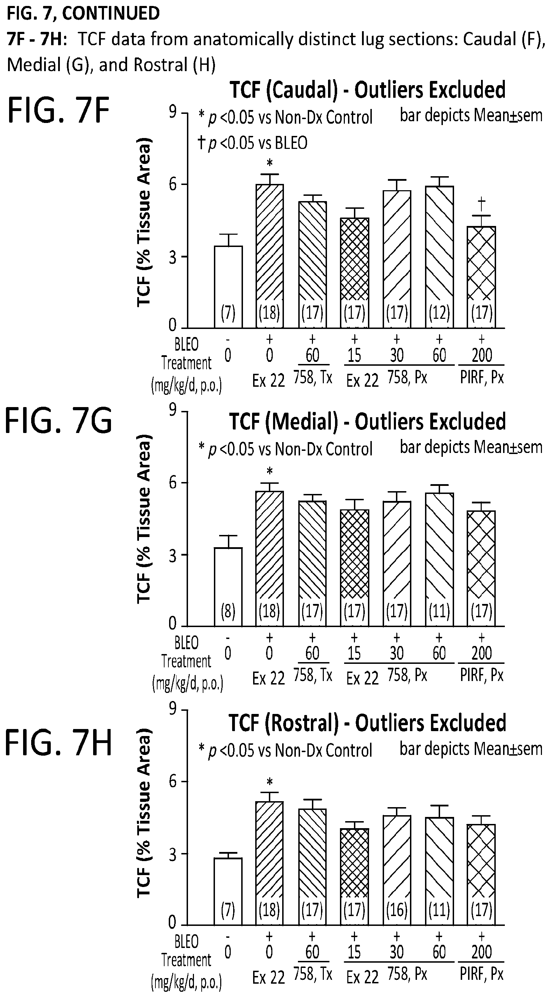

Endpoint Lung Collagen Volume Fraction (CVF) and Tissue Collagen Fraction (TCF) Data Representative images of Masson's Trichrome Blue (MTB)-stained lung tissue are depicted in Figure 6. Summary graphical endpoint CVF and TCF data are depicted in Figures 7A - 7H. Composite CVF data (Caudal + Medial + Rostral CVF) are depicted in Figure 7A. CVF data from serial sections are depicted in Figures 7C - 7E. Composite TCF data (Caudal + Medial + Rostral CVF) are depicted in Figure 7B. TCF data from serial sections are depicted in Figures 7F - 7H.

BLEO-instilled mice had increased composite CVF and TCF relative to Vehicle-instilled controls (CVF: 2.63 ± 0.24 vs. 1.21 ± 0.21%; and TCF: 5.58 ± 0.34 vs. 3.45 ± 0.51%). Of the dosing paradigms tested in BLEO-instilled mice, Px PIRF reduced CVF; Px PIRF and 15 mpk/day Px EXAMPLE 22 reduced TCF relative to Vehicle-treated controls (CVF - Px PIRF: 1.87 ± 0.19 vs. Vehicle: 2.63 ± 0.24%; and CVF - PIRF:4.43 ± 0.34 and 15mpk EXAMPLE 22: 4.51 ± 0.31 vs. Vehicle: 5.58 ± 0.34%).

### DISCUSSION AND CONCLUSION

The objective of the current study was to evaluate the dose-dependent effects of EXAMPLE 22 on BLEO-induced lung fibrosis in male C57BL/6 mice.

Idiopathic pulmonary fibrosis (IPF) is a progressive and lethal lung disease whose median survival (2.5 - 3 years) is unaffected by current medical treatments. Although there are several rodent models of IPF, none perfectly recapitulates the human disease. Bleomycin sulfate (BLEO) has emerged as the pulmonary fibrosing agent of choice because of its ability to mimic several aspects of human disease. BLEO is an antineoplastic antibiotic that induces basement membrane damage, patchy parenchymal inflammation of variable intensity, epithelial cell injury, and interstitial as well as intra-alveolar fibrosis.

Chronic (21-day) BLEO challenge elicited expected outcomes characteristic of pulmonary fibrosis, including reduced endpoint body weight (BW) and a negative change in initial-to-endpoint BW (ΔBW), associated with the increased effort of breathing. Moreover, 21-day BLEO challenge led to increased lung weight (LW) and lung weight indexed to tibia length (LW:TL), consistent with increased lung inflammation. Biochemical analysis of whole lung lysates demonstrated an increase in hydroxyproline (OH-P) content in BLEO-instilled animals relative to Vehicle- instilled controls, reflective of increased collagen deposition during fibrotic remodeling. Furthermore, histological analysis of lung tissue demonstrated an increase in collagen content (Collagen Volume Fraction, CVF and Tissue Collagen Fraction, TCF) as assessed by Masson's Trichrome Blue (MTB) staining.

The current study employed both prophylactic (Px) and therapeutic (Tx) arms to both evaluate the ability of EXAMPLE 22 to inhibit and the treat BLEO-induced lung fibrosis. Px Pirfenidone (PIRF) was evaluated as a reference agent in the study. Mice incepted oral (p.o.) Px dosing of one of three dose levels of EXAMPLE 22 (twice daily, BID; 15, 30, or 60 mg/kg/day) or PIRF (p.o., BID; 200 mg/kg/day) one day prior to BLEO instillation; oral Tx dosing of EXAMPLE 22 (p.o. BID; 60 mg/kg/day) incepted 5 days post-BLEO instillation. Mice continued p.o. BID dosing for the remainder of the study.

Neither EXAMPLE 22 nor PIRF affected serial BW, final BW, or ΔBW relative to Vehicle-treated BLEO-instilled controls.

Neither EXAMPLE 22 nor PIRF affected endpoint lung weight (LW) or LW indexed to tibia length (LW:TL) relative to Vehicle-treated BLEO-instilled controls.

Neither EXAMPLE 22 nor PIRF affected endpoint lung OH-P content relative to Vehicle-treated BLEO-instilled controls.

Reductions in lung collagen content were observed with the administration of 15 mpk Px EXAMPLE 22 (TCF) and Px PIRF (CVF and TCF) relative to Vehicle-treated controls.

### Effect of EXAMPLE 22 on Cardiac Function after Coronary Artery Ligation in Mice

### Pre-Surgical Conditioning and Surgical Procedures:

Adult male C57BI/6 mice, 3 months old, were purchased from Jackson Laboratories and acclimated for 3 or more days in standard housing. All mice underwent twice daily intra-gastric administration of physiologic saline for 7 days pre-operatively (acclimation period). They also underwent standard B- and M-mode echocardiographic (ECHO) imaging (VisualSonics Vevo 2100) of the left heart under conscious restraint, 1-5 days prior to surgery.

Under ketamine/xylazine anesthesia, mice were intubated and ventilated, then incised through the left thoracic wall between the 3^{rd} and 4^{th} ribs to expose the heart. For ligation of the left anterior descending coronary artery (LAD), an 8-0 nylon suture was inserted under microscopic view (Wild/Leica operating microscope), placing and tying the ligature at a point 1 mm distal to the point where the artery emerges from the edge of the left atrium. Confirmation was provided by clear blanching of the downstream ventricle wall. Control sham-treated mice underwent needle and 8-0 suture passage under the coronary artery but with suture removal without ligation. The wound was closed with 6-0 suture in two layers (muscle and skin, respectively) and mice were recovered on a warming blanket. They received buprenorphine intraoperatively and twice daily postoperatively for 48 hours, by subcutaneous injection (50 ug/kg body weight).

### Treatment Groups:

A total of 48 mice entered the study. Eight mice underwent sham surgery, and these mice received vehicle treatment after surgery. 40 mice underwent LAD ligation, with 20 in each treatment group. Treatment was by oral gavage (intra-gastric administration) at 10 mL/kg body weight, twice daily (7-9 am and 4-6 pm), starting 1-2 hours after surgery and continuing until study termination. Treatment was either with EXAMPLE 22 at a concentration of 3 mg/mL (30 mg/kg) or vehicle for the test agent.

### Evaluation:

Echocardiography was done under brief, conscious restraint using a VisualSonics Vevo 2100 ultrasound machine, pre-operatively and at 2 and 4 weeks postoperatively. Standard B-mode and M-mode ultrasound imaging was captured at each imaging session. Echocardiographic images were analyzed for left ventricular function, measuring the interventricular septal thickness, internal ventricular cavity dimension, and posterior wall thickness at both diastole and systole from M-mode images at the level of the papillary muscles. The left ventricle ejection fraction and fractional shortening were calculated from these values: 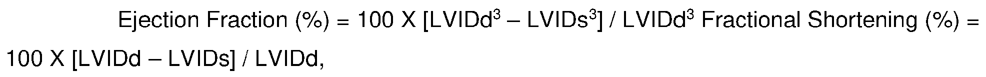
Ejection Fraction (%) = 100 X [LVIDd3 - LVIDs3] / LVIDd3 Fractional Shortening (%) = 100 X [LVIDd - LVIDs] / LVIDd,
where LVID = left ventricle inner diameter (internal ventricular cavity dimension), d = diastole, s = systole

After the 4-week ECHO imaging, mice were anesthetized and a terminal blood draw was taken from the abdominal aorta into 3.8% citrate (9:1), and the plasma was obtained by centrifugation and frozen. Mice were then euthanized (anesthesia overdose followed by cervical dislocation) and the hearts were weighed and the area of infarction was visually measured with t ruler for cranial-caudal and for lateral axes lengths of the approximately oval infarction zone (ligated hearts only). The hearts, and both kidneys, were then immersion-fixed in neutral-buffered formalin for subsequent histopathological evaluation. Plasma, kidney, and heart specimens were returned to Study Sponsor for further evaluation. The time between the last drug dosing and the blood draw was recorded, for future dose-response evaluation by Study Sponsor.

### Statistical analyses:

Summary statistics by "treatment" were provided for the change from baseline to week 2 and change from baseline to week 4 where baseline was defined as the pre-echos time-point. For each parameter, the mean "treatment" difference between "Example 22" and "Vehicle" was analyzed using a 2-sample t-test. The analysis was conducted using SAS Version 9.4 via PROC TTEST under unequal variances for the "treatments" using the Satterthwaite method. The 2-sided 95% confidence interval (a=0.05) and corresponding p-value are presented.

If there are any concerns about the t-test's normality assumption, a sensitivity analysis can be conducted using the non-parametric Wilcoxon rank-sum test (via PROC NPAR1WAY). A quick review suggested that were no obvious deviations from normality with the exception of the FS parameter.

### Results and Discussion:

There were no deaths in the sham-surgery group, 2 deaths in the EXAMPLE 22-treated ligated group (2/20), and 7 deaths in the vehicle-treated ligated group (7/20); all deaths were between 3 and 11 days postoperatively. All surviving mice had echocardiography done at 2 and 4 week postoperatively. One of the EXAMPLE 22-treated mice with LAD ligation had normal echocardiographic data at 2 and 4 weeks; upon harvest, there was no indication of an infarcted zone, suggesting that the ligation did not capture the LAD. Another two of the EXAMPLE 22-treated mice had unusual appearances of the heart upon harvest: one had a necrosing left atrium due to the ligature suture having caught an edge of the atrium, and the other had scarring of the heart to the chest wall. Data for these three mice were excluded in the analyses. Thus, a total of 15 EXAMPLE 22-treated mice and 13 vehicle-treated mice that had undergone LAD ligation completed the 4-week course of study; all 8 sham-surgery mice also completed the 4-week course.

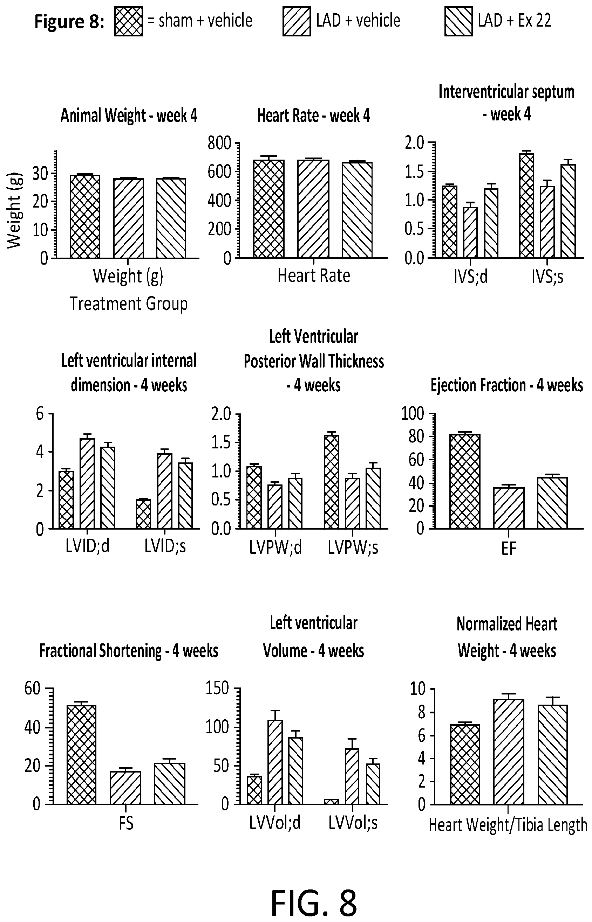

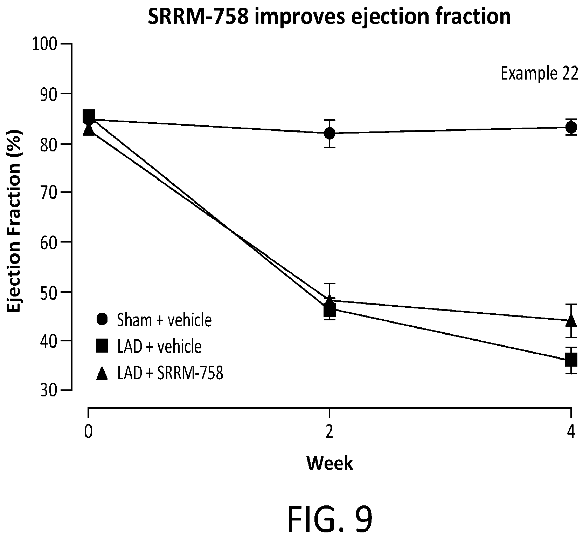

The LAD-ligated mice showed loss of left ventricular function at 2 and 4 weeks of approximately 45-50%. Treatment with EXAMPLE 22 led to improved cardiac fraction after MI compared to vehicle treated animals. Statistically significant changes were noted with several parameters **(Table 3).** Representative parameters of heart dysfunction are shown in **Figure 8****.** Statistically significant improvement in ejection fraction is noted at 4 weeks post-treatment **(****Figure 9****).**

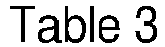

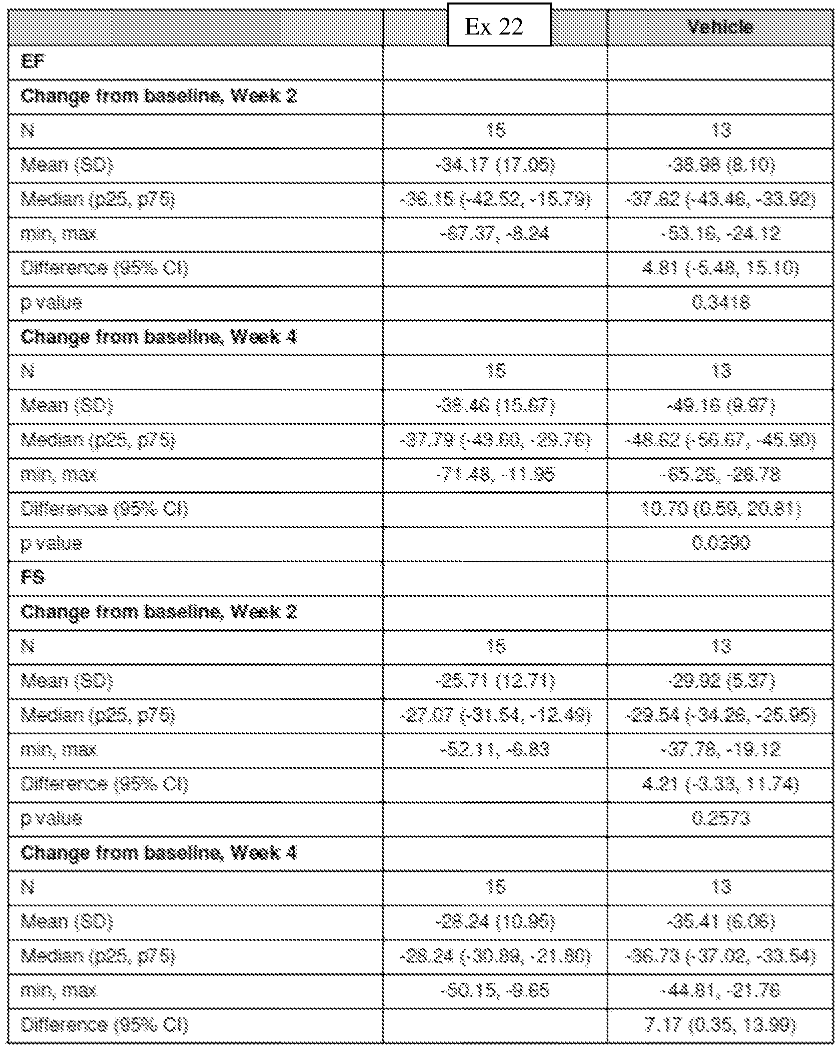

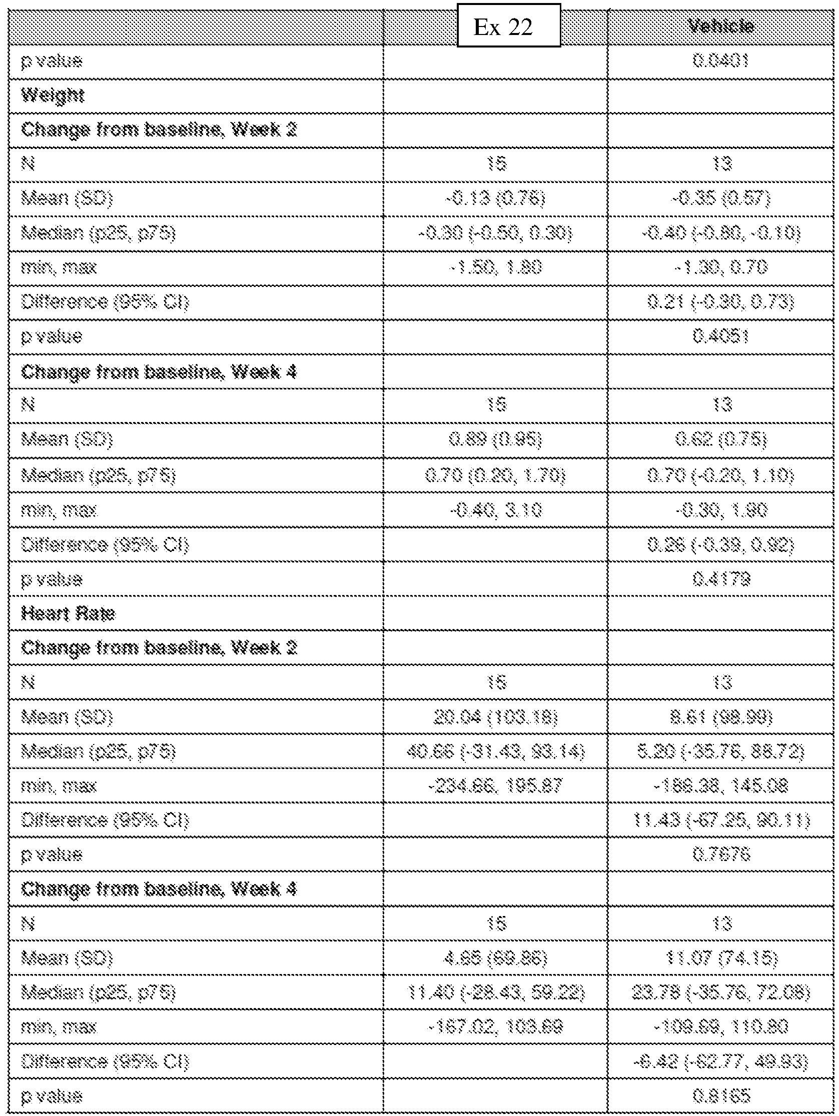

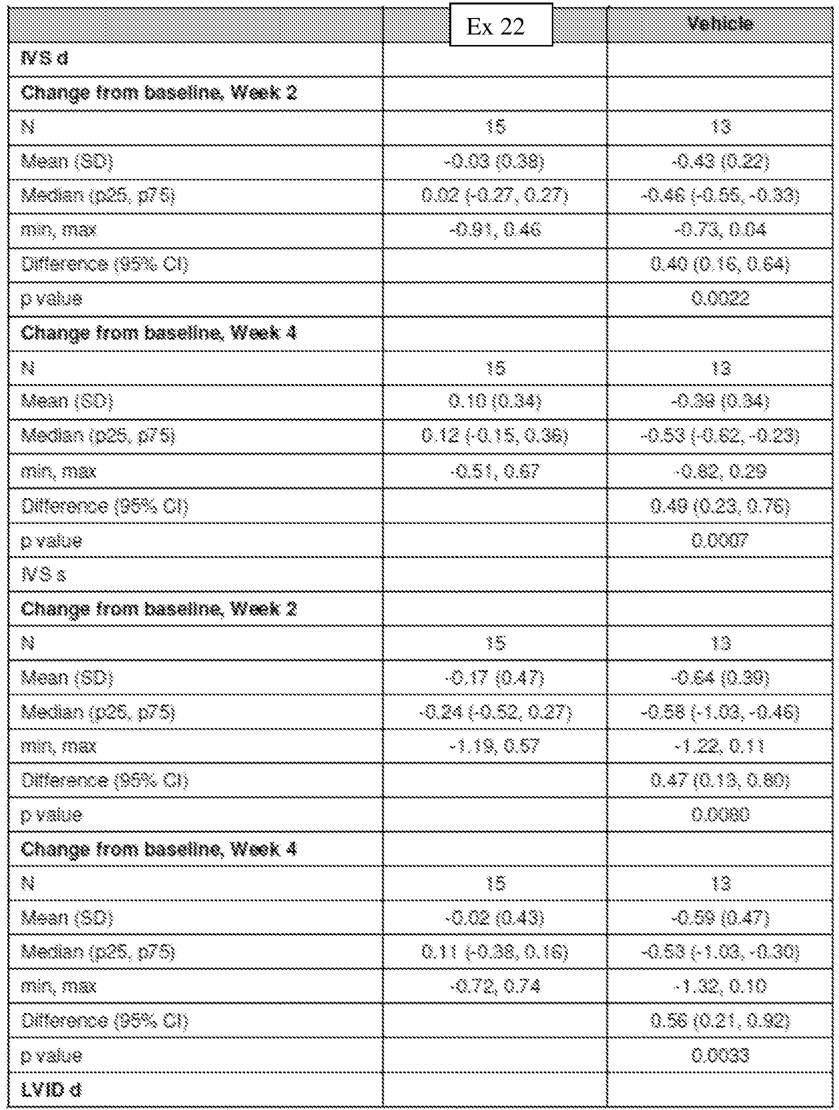

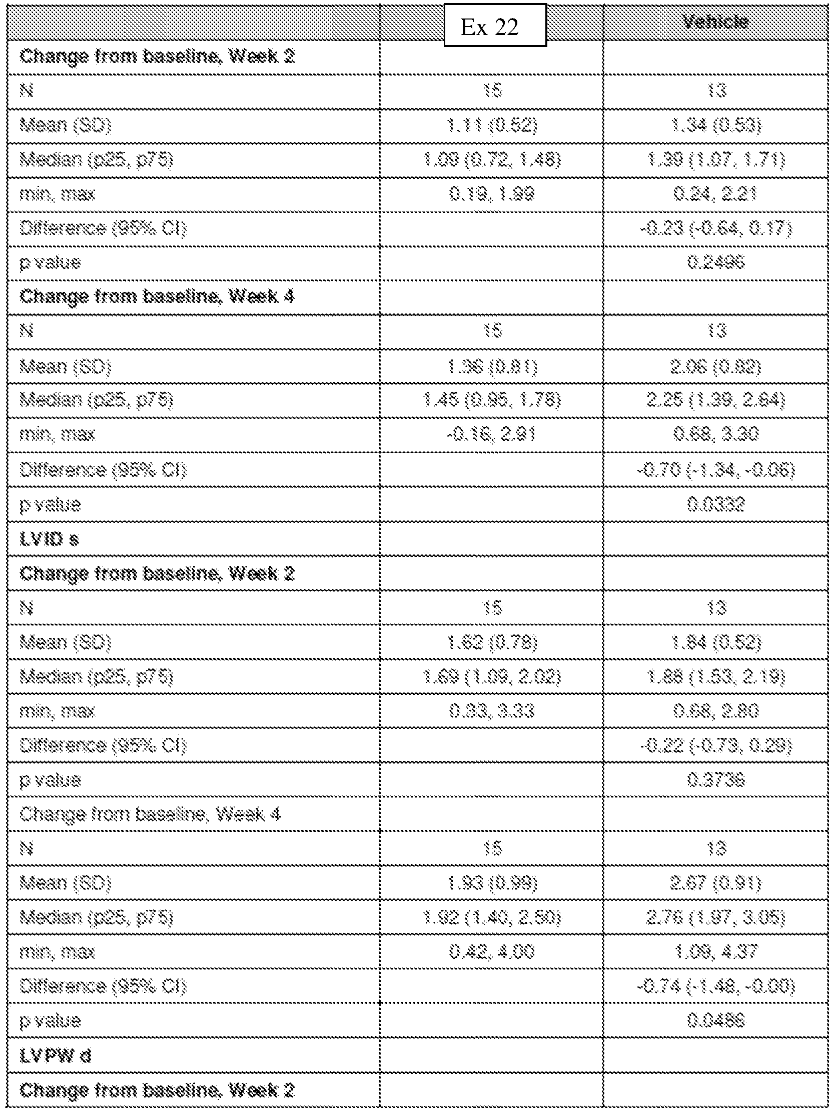

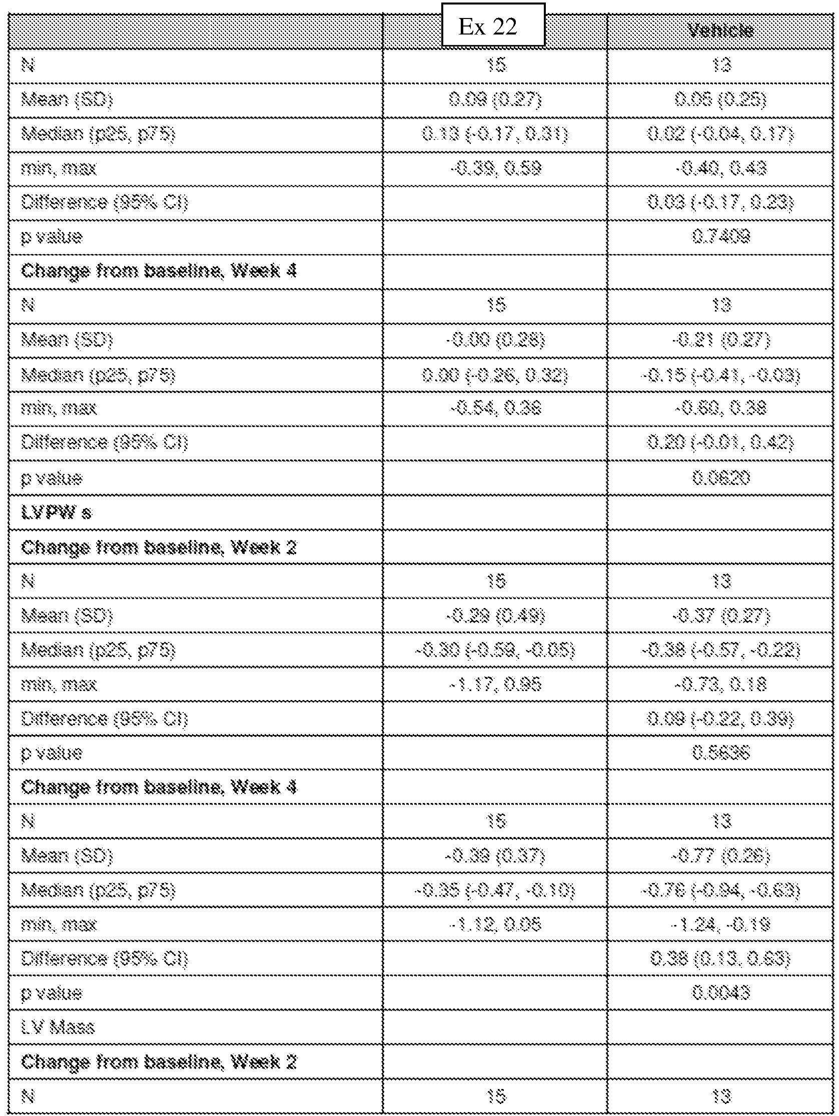

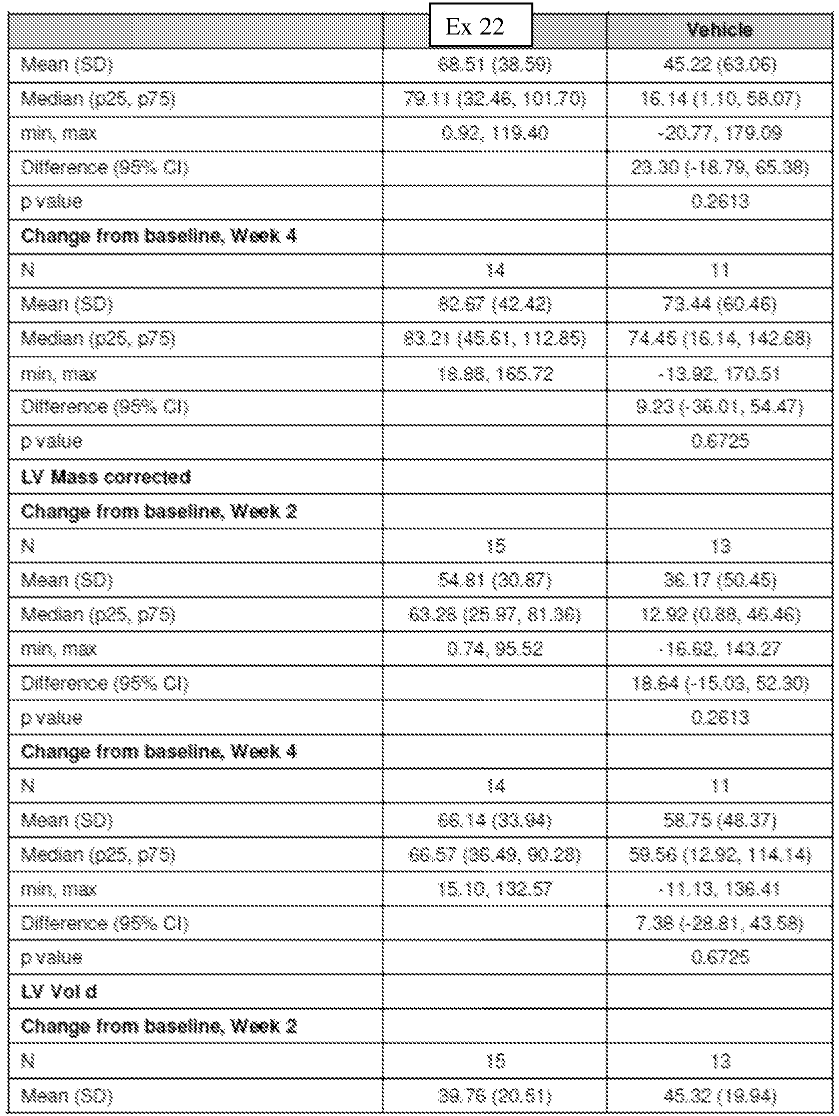

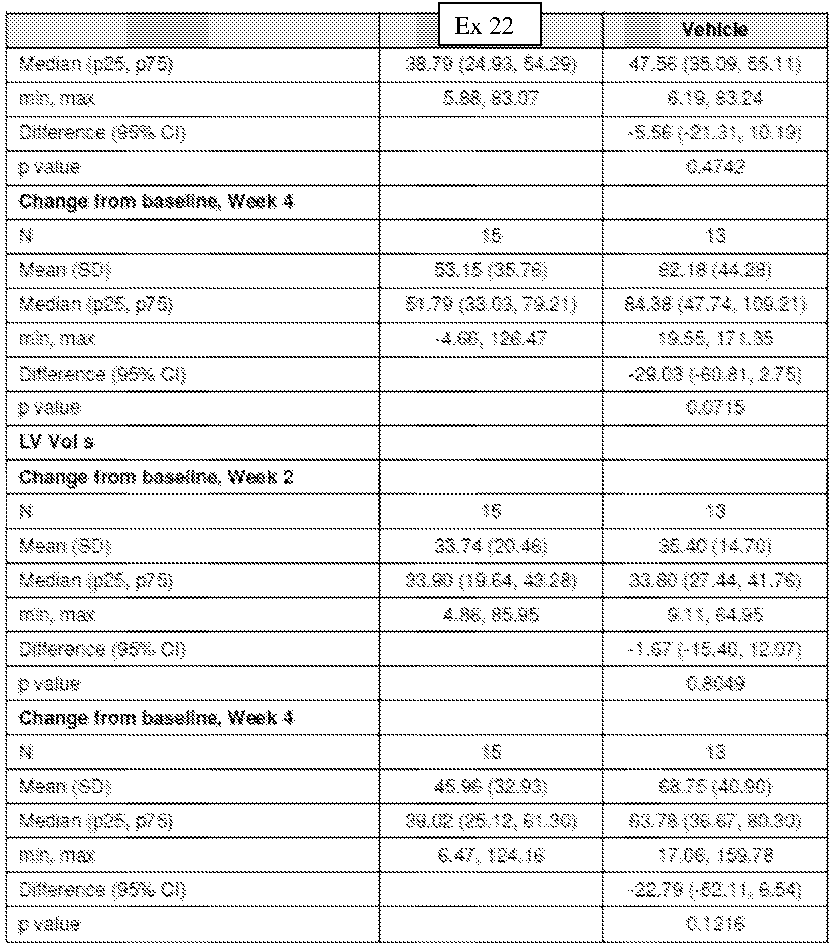

### Assay: Pharmacokinetic Profiling of EXAMPLE 22 Following Oral and Intravenous Administration to Male Sprague-Dawley (SD) Rats

### 1. Test Article:

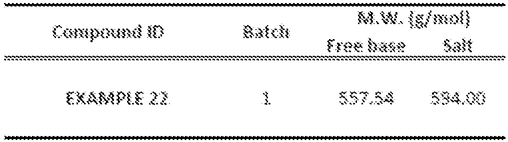

### 2 Dosing vehicle and administration:

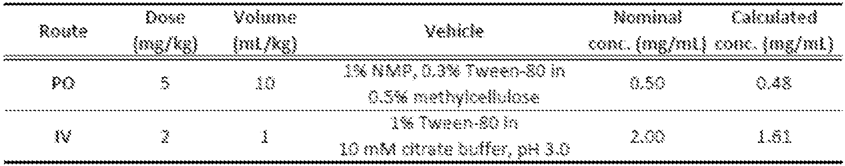

### Preparation details:

Oral: NMP was added to prewwighted powder and manually grinded in a mortar Tween-80 and methylcellulose were then added one after the ther and the mixture was thoroughly grinded between each addition. The resulting mixture was transferred into a 20 ml vial and vortexed to ensure homogeneity. EXAMPLE 22 oral formulation yielded a clear suspension.

### Intravenous:

Tween-80 was added first to the powder followed by the addition of the 10 mM citrate buffer pH 3.0. The formulation was then vortexed gently and soticated 7 minutes. The resulting formulation was a clear solution suitable for iv injection.

### 3. Animals:

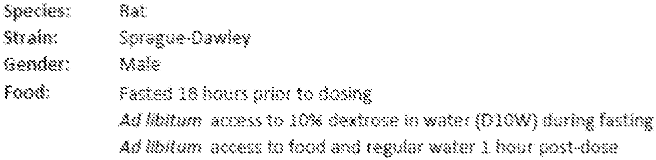

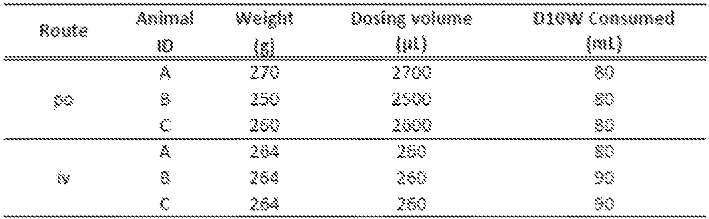

### Analytical Method Summary

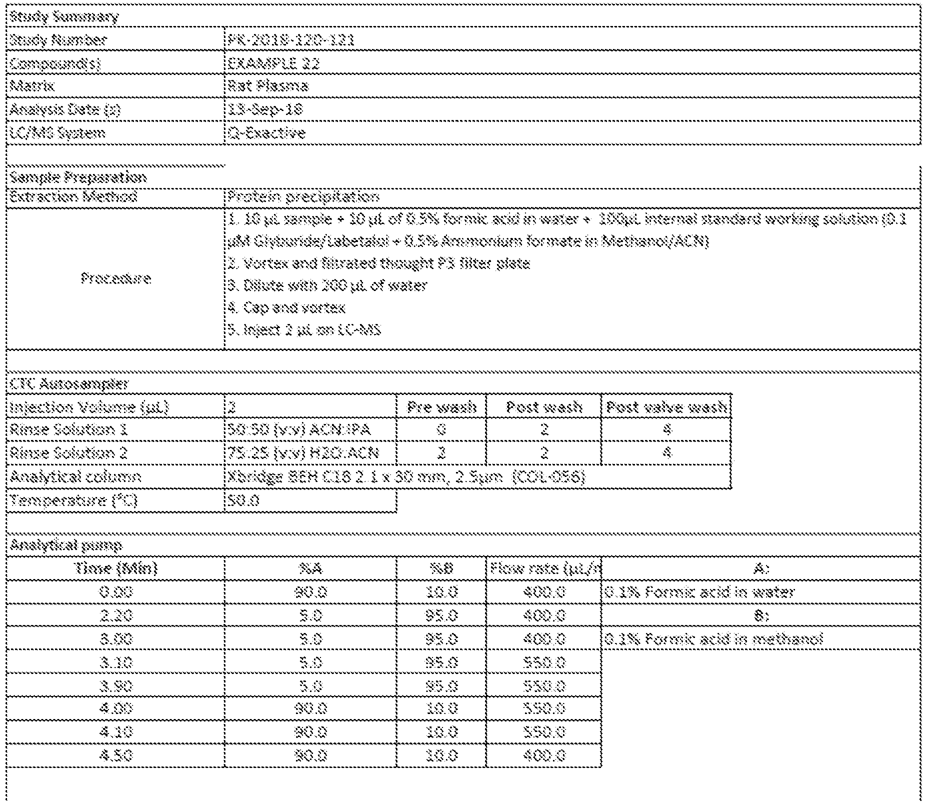

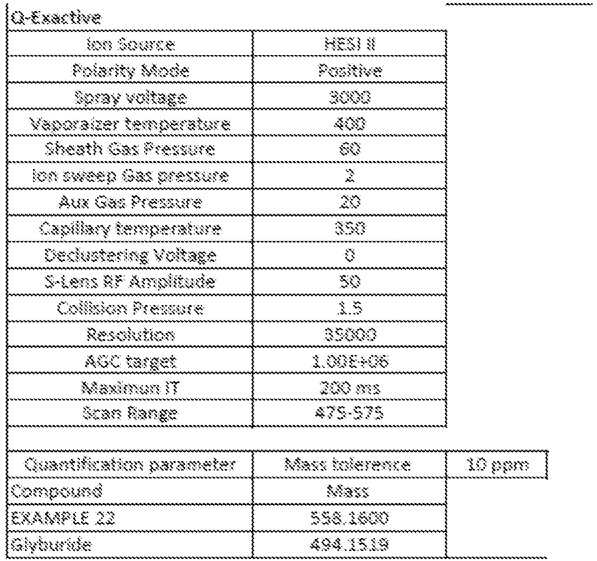

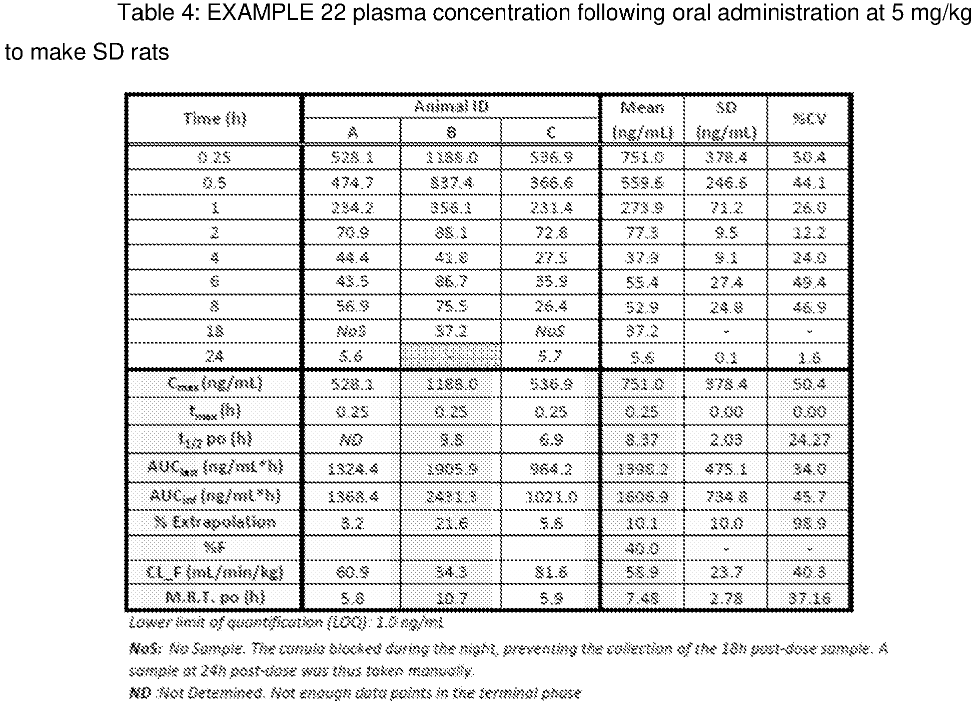

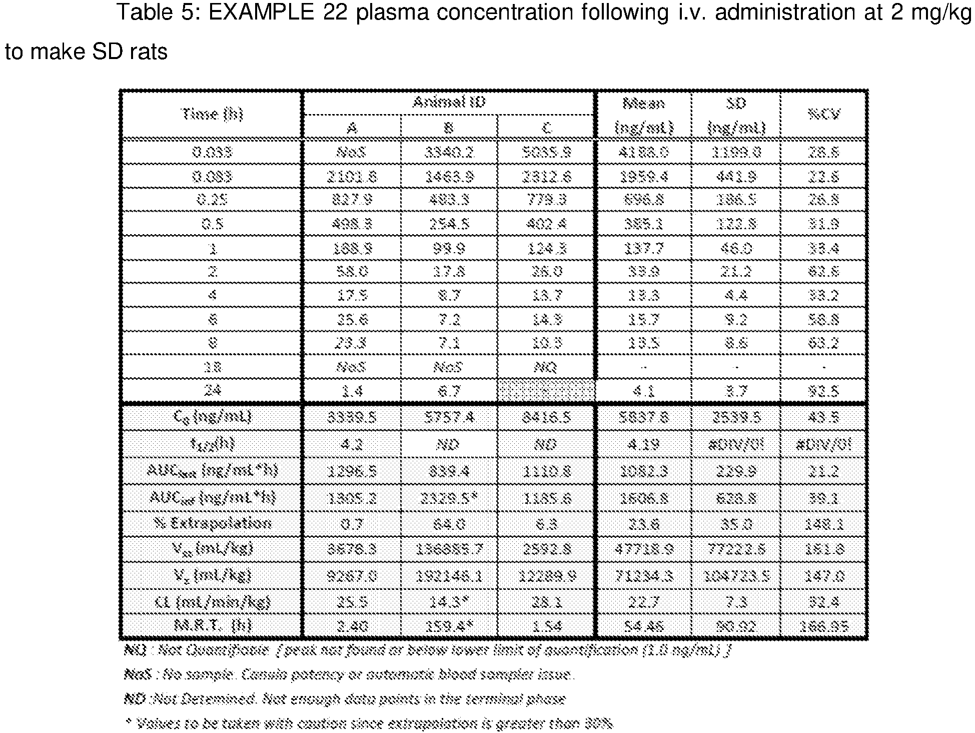

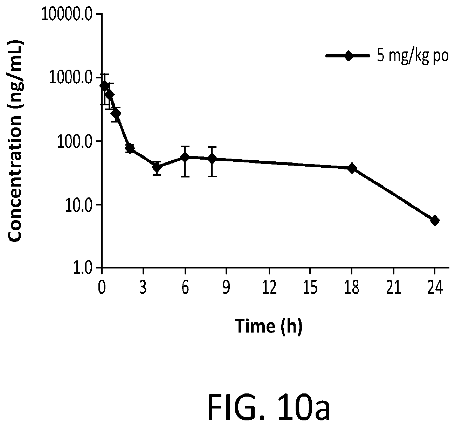

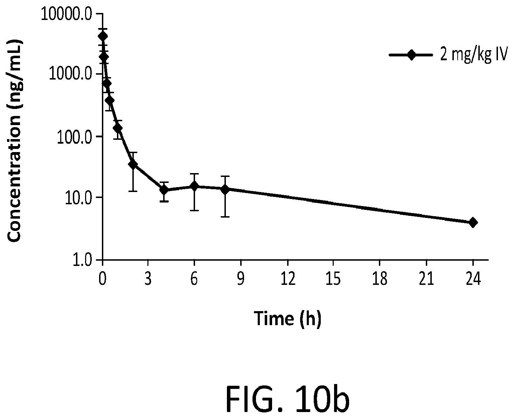

See also, FIGURES 10a and 10b

### Assay: Pharmacokinetic Profiling of EXAMPLE 22 Following Oral and Intravenous Administration to Male C57BI/6 Mice

### 1. Test Article:

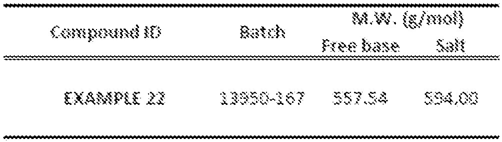

### 2. Dosing vehicle and administration:

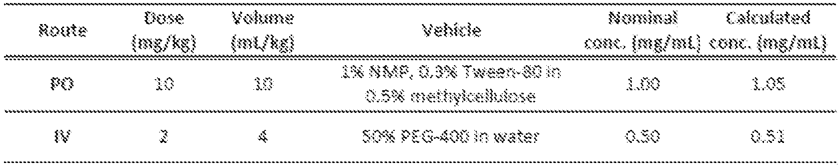

### Preparation details:

Oral: NMP was added to preweighed powder and manually grinded in a mortar. Tween-80 and methylcellulose were then added one after the other and the mixture was thoroughly grinded between each addition. The resulting mixture was transferred into a 4 ml vial and vortexed to ensure homogeneity. EXAMPLE 22 oral formulation yielded a beige suspension.

Intravenous: PEG-400 was added first to the powder and sonicated 20 minutes until clear. Water was then added, the mixture was vortexed and sonicated 1 minute. The resulting formulation was a clear solution.

### 3. Animals:

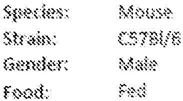

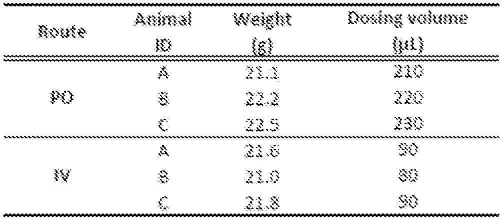

### 7. Analytical Method Summary

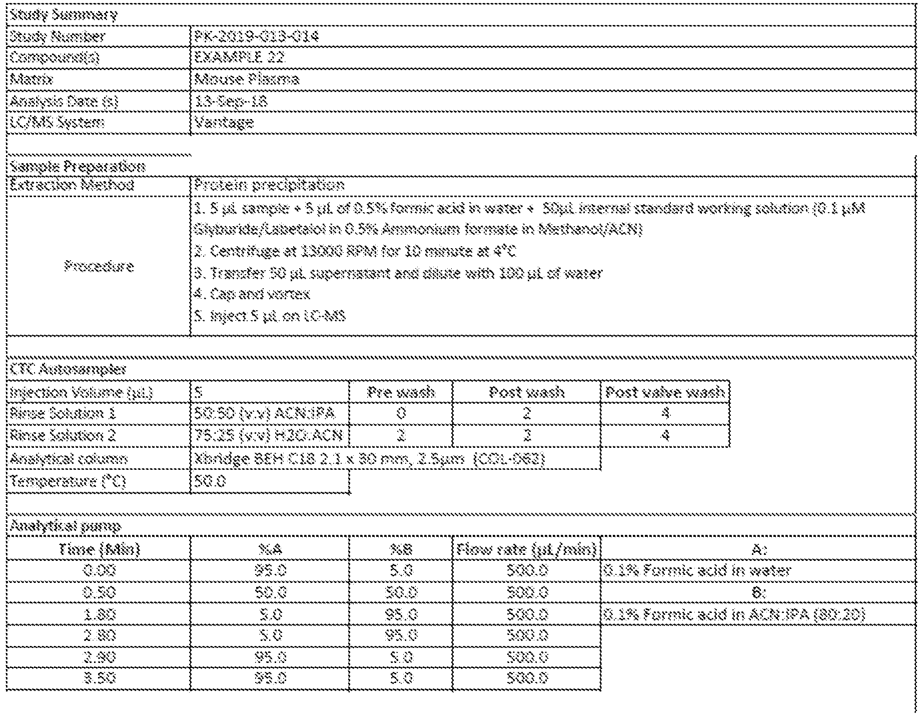

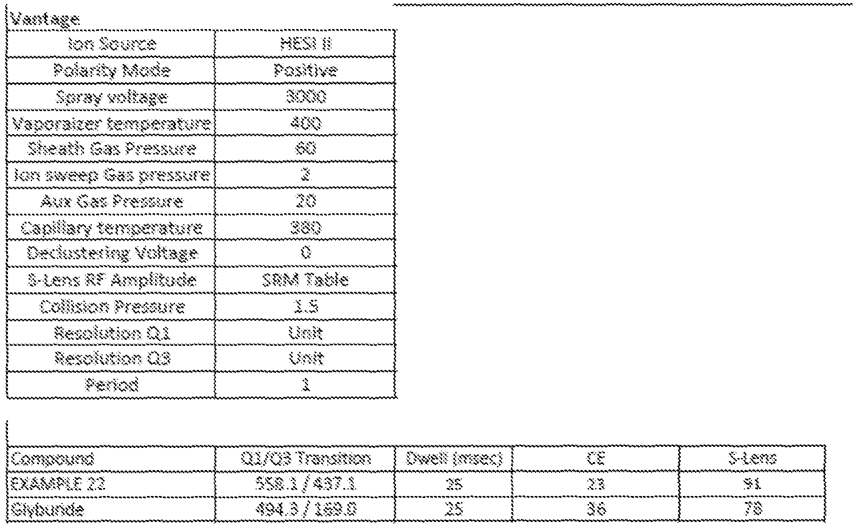

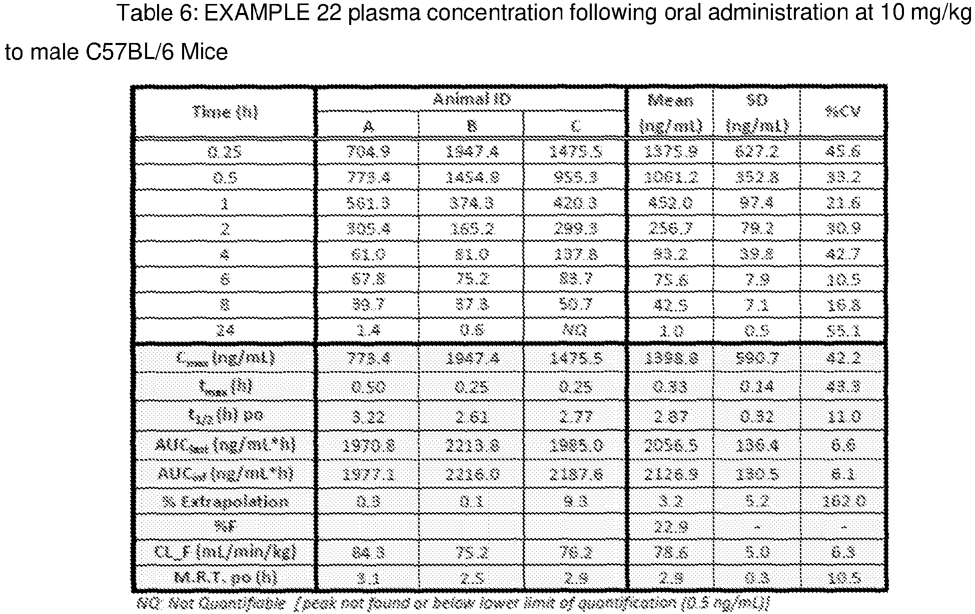

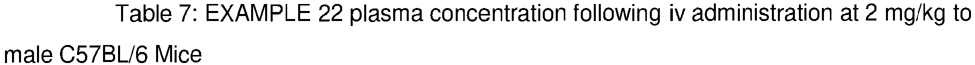

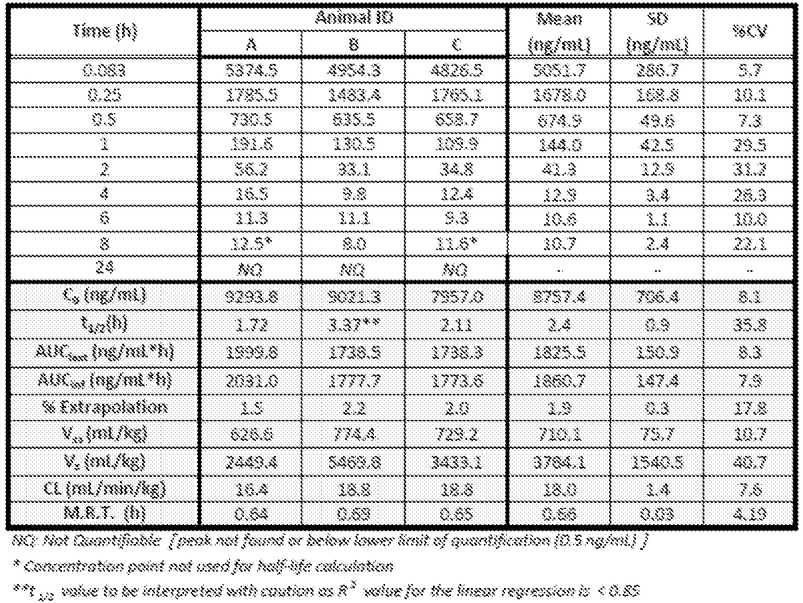

### Assay: Plasma Protein Binding to Various Species Plasma

### 1. QBJECTIVES

The objective of the study was to determine the binding of EXAMPLE 22 to Human, Mouse and rat plasma plasma protein and Comparator B to rat plasma protein using the high throughput dialysis (HTD) device.

### 2. BIOANALYSIS

### 2.1. Analysis and Reporting

Sample were analyzed by LC/MS/MS using a CTC PAL autosampler, Thermo Surveyor MS pupm plus and a Thermo TSQ Vantage triple quadrupole mass spectrometer. The [M+H]+ adducts of the test compounds and internal standard (Glyburide) were monitored using positive mode electrospray ionization in MRM (multiple reaction monitoring mode. The analytes were injected onto a C18 column and chromatographed with a generic reverse phase gradient using 0.1% formic acid in water and 0.1% formic acid in acetonitrile/IPA/Methanol mobile phases.

Peak integrations were performed using Quickquan (v 2.4) and Thermo Xcalibur (v 2.1) software. Resulting data was tabulated and summarized using Microsoft Excel.

### 2.2. MS/MS Conditions Summary

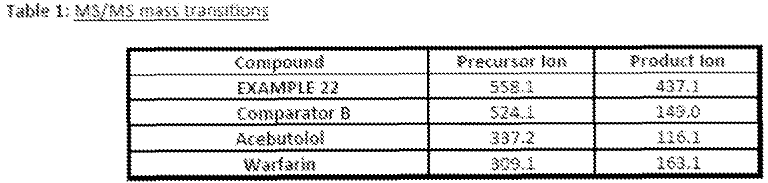

### 3. EXPERIMENTAL CONDITIONS

### 3.1 Equillibrium Dialysis

Test compound and controls (1 µM) were spiked in human (EXAMPLE 22), mouse (EXAMPLE 27) and rat (EXAMPLE 22 and Comparator B) plasma, respectively, and aliquoted in triplicate in a high throughput dialysis (HTD) 96-well plate, where the plasma and dialysate buffer were separated by a semi-permeable cellulose membrane (12-14K MWCO). Once sealed, the HTD plate was incubated at 37°C and kept under light agitation for 6 hours, until equilibrium was reached. Plasma and buffer samples were then extracted along with their corresponding standard curve samples using ice-cold acetonitrile in methanol (1:1). After centrifugation, supernatants from both plasma- and buffer-containing samples were futher diluted prior to be submitted to bioanalysis by LC-MS/MS. Acebutolol and warfarin served as low-bound and highly-bound controls, respectively. Determination of the percentage of plasma protein binding and recovery was achieved by following equations: $% Plasma binding = \frac{\left(Conc.in plasma-Conc.in buffer\right)}{Conc . in plasma} \times 100$ $% Recovery = \frac{\left(Conc. in plasma+Conc. in buffer\right)}{initial conc . in plasma} \times 100$

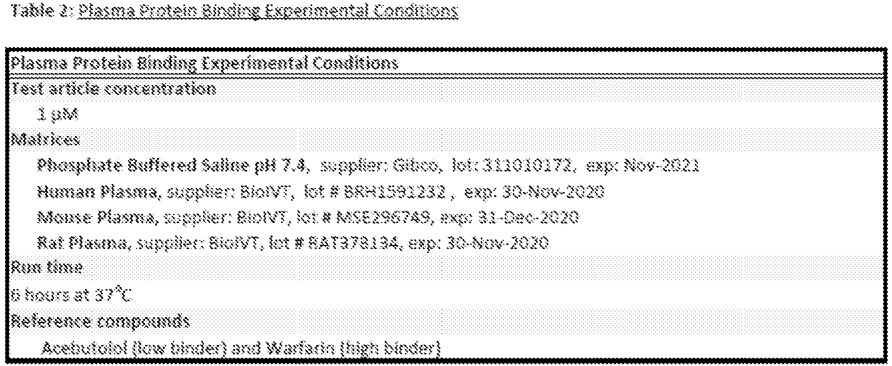

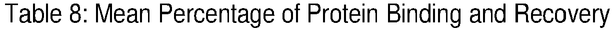

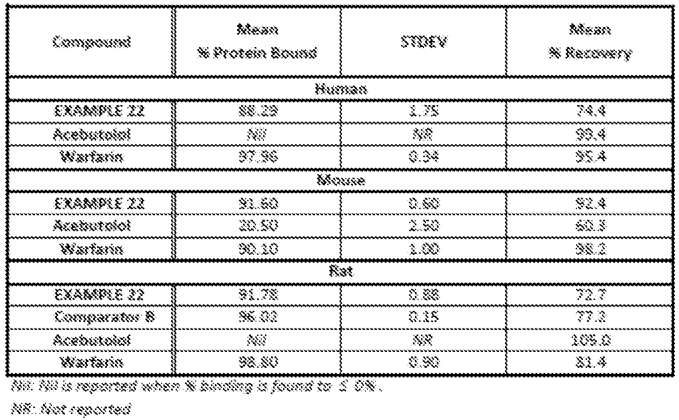

### Tables 9, 10, and 11:

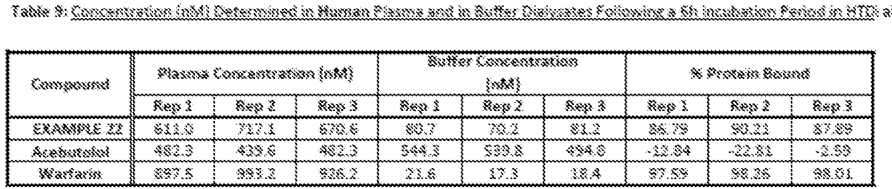

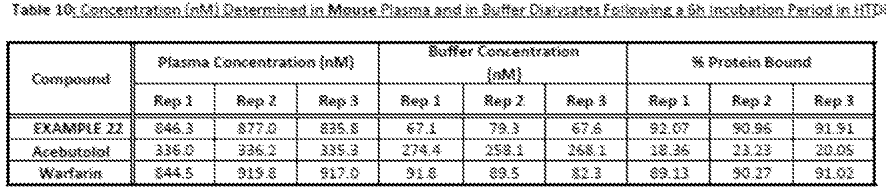

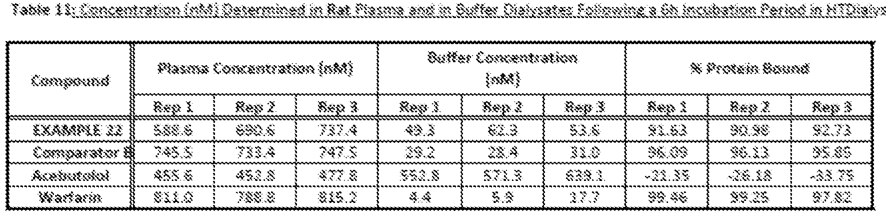

### Assay: Plasma and Tissue Pharmacokinetics of EXAMPLE 22 Following a Single Oral Administration in male SD rat

The study design is summarized as follows:

### Summary

### Compound Info

| Compound #: | EXAMPLE 22 |
|---|---|
| Batch ID: | 14201-116 |
| Salt: | NO |
| Compound MW: | 593.13 |
| Batch FW: | 594.00 |
| Salt Factor: | 1.00 |

### In-life

| | | |
|---|---|---|
| Study Type: | PO | Tissue PK |
| N/ Treatment: | | 5/total 10 |
| Doses: | | 1 mg/kg |
| | PO | 0 |
| Formulations: | 0.5% (w/v) CMC-Na, 0.1 % (v/v) Tween 80 in Milli-Q Water | |
| | PO | |
| Dosing Solution: | | 1 mg/mL |
| | PO | |
| Blood Sampling @: | | Plasma: 4, 24h post dose |
| | PO | |
| Tissue Sampling @: | lung, brain, kidney, muscle/skeletal, spleen, heart and skin: 4, 24h post dose | |
| | PO | |
| Species/strain: | SD Rat | |
| Sex: | Male | |
| In-life Comments: | No abnormal clinical symptoms were observed during the entire experiment. | |

### Bioanalysis

| | | |
|---|---|---|
| Bioanalytical Assay: | HPLC | Instrument: Shimadzu (DGU-20A5R, Serial No: L20705518892 IX; LC-30AD Serial No: L20555510778 AE and L20555510647AE; SIL-30AC, Serial No: L20565504943AE; Rack Changer II Serial No. L20585501071 SS; CTO-30A: Serial No.L20575501294 CD; CBM-20A: Serial No.L20235533959 CD) |
| | MS | AB API 5500 LC/MS/MS instrument (Serial No. EF20351803) |
| | Column | YMC-Triart C18 5 µm (50*2.1 mm) |
| | Mobile Phase | A 95% Water (0.1% Formic Acid) |
| | | B 95% Acetonitrile (0.1% Formic Acid) |
| | Quantification | Internal Standard Method |
| Bioanalysis Comments: | 50 uL of plasma sample + 5 uL of blank solution + 200 uL of ACN for PPE (protein precipitation extraction). | |
| | 50 uL of tissue homogenate samples (tissue) + 5 uL of blank solution + 200 uL of ACN for PPE (protein precipitation extraction). | |

### Formulation

### Compound information

| | | | |
|---|---|---|---|
| **Compound ID** | EXAMPLE 22 | **Batch ID:** | 14201-116 |
| **MW (Free form)** | 593.13 | **FW (Salt form)** | 594.00 |
| **Purity** | >99% | **Appearance** | white powder |

### Preparation of PO (10 mg/kg, 10 mL/kg) Dosing

1 mg/mL solution of 0.5% (w/v) CMC-Na, 0.1 % (v/v) Tween 80 in Milli-Q Water.

Dissolved 29.68 mg of EXAMPLE 22 in 29.637 mL of 0.5% (w/v) CMC-Na, 0.1 % (v/v). Tween 80 in Milli-Q Water with vortexing and sonicate to obtain a solution.

Note: The formulation was freshly made prior to use.

### Dose Formulations Validation

| Route | Sample Name | Dilution Factor | Nominal | Measured (mg/mL) | Mean (mg/mL) | Accuracy (%) | SD (mg/mL) | CV (%) |
|---|---|---|---|---|---|---|---|---|
| PO | DOSE PO1 | 10000 | 1 | 1.01 | 1.01 | 1.1 | 0.02 | 1.74 |
| | DOSE PO2 | 10000 | | 1.02 | | | | |
| | DOSE PO3 | 10000 | | 0.986 | | | | |

### Analytical Method

| | |
|---|---|
| LC-MS/MS System: | Instrument: Shimadzu (DGU-20A5R, Serial No: L20705518892 IX; LC-30AD Serial No: L20555510778 AE and L20555510647AE; SIL-30AC, Serial No: L20565504943AE; Rack Changer |
| HPLC: | II Serial No. L20585501071 SS; CTO-30A: Serial No.L20575501294 CD; CBM-20A: Serial No.L20235533959 CD) |
| Column: | YMC-Triart C18 5 µm (50*2.1 mm) |
| MS: | AB API 5500 LC/MS/MS instrument (Serial No. EF20351803) |
| HPLC Conditions Mobile Phase: | Solution A: 95% Water (0.1 % Formic Acid) |
| | Solution B: 95% Acetonitrile (0.1% Formic Acid) |

### Gradient of Fenofibrate

### Flow rate: 0.5 mL/min

| Time (min) | A (%) | B (%) |
|---|---|---|
| 0.01 | 90.0 | 10.0 |
| 0.30 | 90.0 | 10.0 |
| 1.90 | 10.0 | 90.0 |
| 2.20 | 10.0 | 90.0 |
| 2.21 | 90.0 | 10.0 |
| 2.50 | 90.0 | 10.0 |

### Injection volume: 50 µL for heart, 20 µL for others

### Gradient of Fenofibrate: For Heart

| Flow rate: 0.5 mL/min | | |
|---|---|---|
| Time (min) | A (%) | B (%) |
| 0.01 | 90.0 | 10.0 |
| 0.30 | 90.0 | 10.0 |
| 4.20 | 10.0 | 90.0 |
| 4.70 | 10.0 | 90.0 |
| 4.75 | 90.0 | 10.0 |
| 5.00 | 90.0 | 10.0 |

### Sample Preparation of Plasma

The desired serial concentrations of working solutions were achieved by diluting stock solution of analyte with 50% acetonitrile in water solution. 5 µL of working solutions (10, 20, 50, 100, 500, 1000, 5000, 10000 ng/mL) were added to 50 µL of the blank SD Rat plasma to achieve calibration standards of 1~1000 ng/mL ( 1, 2, 5, 10, 50, 100, 500, 1000 ng/mL) in a total volume of 55 µL. Four quality control samples at 2 ng/mL, 5 ng/mL, 50 ng/mL and 800 ng/mL for plasma were prepared independently of those used for the calibration curves. These QC samples were prepared on the day of analysis in the same way as calibration standards.

55 µL standards, 55 µL QC samples and 55 µL unknown samples (50 µL plasma with 5 µL blank solution) were added to 200 µL of acetonitrile containing IS mixture for precipitating protein respectively. Then the samples were vortexed for 30 s. After centrifugation at 4 degree Celsius, 3900 rpm for 15 min, the supernatant was diluted 3 times with water. 20 µL of diluted supernatant was injected into the LC/MS/MS system for quantitative analysis.

### Sample Preparation of Tissue

The desired serial concentrations of working solutions were achieved by diluting stock solution of analyte with 50% acetonitrile in water solution. 5 µL of working solutions ( 10, 20, 50, 100, 500, 1000, 5000, 10000 ng/mL) were added to 50 µL of the blank SD Rat tissue( lung, brain, kidney, muscle/skeletal, spleen, heart and skin) homogenous to achieve calibration standards of 1~1000 ng/mL ( 1, 2, 5, 10, 50, 100, 500, 1000 ng/mL) in a total volume of 55 µL. Four quality control samples at 2 ng/mL, 5 ng/mL, 50 ng/mL and 800 ng/mL for tissue were prepared independently of those used for the calibration curves. These QC samples were prepared on the day of analysis in the same way as calibration standards.

50 µL standards, 50 µL QC samples and 50 µL unknown samples (50 µL Tissue with 5 µL blank solution) were added to 200 µL of acetonitrile containing IS mixture for precipitating protein respectively. Then the samples were vortexed for 30 s. After centrifugation at 4 degree Celsius, 3900 rpm for 15 min, the supernatant was diluted 3 times with water. 50 µL of heart diluted supernatant and 20 µL of other tissue diluted supernatant was injected into the LC/MS/MS system for quantitative analysis.

**Table 13: Example 22 Mean Plasma and Tissue Concentrations and Mean Tissue to Plasma Ratios**

| Example 22 | **Plasma** | **Lung** | **Brain** | **Kidney** | **Muscle** | **Spleen** | **Heart** | **Skin** |
|---|---|---|---|---|---|---|---|---|
| 4h Conc. (ng/g) | 102 ng/mL | 40 | BLQ | 305 | 7.3 | 14 | 28.8 | 19.3 |
| 4h | N/A | 0.39 | N/C | 3.11 | 0.07 | 0.14 | 0.28 | 0.19 |
| Tissue:Plasma Ratio | | | | | | | | |
| 24h Conc. (ng/g) | 6.6 ng/mL | N/C | BLQ | 22.9 | BLQ | BLQ | BLQ | BLQ |
| 24h | N/A | N/C | N/C | 3.47 | N/C | N/C | N/C | N/C |
| Tissue:Plasma Ratio | | | | | | | | |

| | | | | | | | | |
|---|---|---|---|---|---|---|---|---|
| N/A: not applicable; BLQ: below the limit of quantitation; N/C: not calculated due to insufficient data. | | | | | | | | |

### Assay: Mini-Ames Assay in Salmonella Typhimurium and Escherichia Coli

The objective of this study was to evaluate the test articles (including EXAMPLE 22), for their ability to induce reverse mutations at the histidine locus of strains of *Salmonella typhimurium* (TA98, TA100, TA1535 and TA1537), and the tryptophan auxotrophic strain of *Escherichia coli* WP2 *uvr*A (pKM101) in the presence and absence of exogenous metabolic activation (β-naphthoflavone and phenobarbital induced rat liver S9).

The assay was conducted in the presence or absence of the S9 mix along with concurrent negative/solvent control and positive controls. Unlimited by the test article solubility in DMSO, the dose levels tested in the Mini-Ames assay were 1.5, 4, 10, 25, 64, 160, 400 and 1000 µg/well.

No cytotoxicity was observed at any dose levels tested either in the presence or absence of S9 mix in each tester strain. No precipitate was observed at any dose levels tested.

The compounds did not induce ≥ 2-fold increases (for TA98, TA100 and WP2 *uvr*A (pKM101)) or ≥ 3-fold increases (for TA1535 and TA1537) at any dose levels tested in the mean number of revertant colonies compared to the concurrent negative/solvent control. No dose related increase of revertant colonies was observed either.

All positive controls used induced the expected increases (three-fold or greater) in the mean number of revertant colonies, in the presence or absence of the S9 mix, when compared to the concurrent negative/solvent control. All of the negative/solvent control data were comparable with historical data.

The genotypes of the tester strains used in this assay were confirmed.

It is concluded that this Mini-Ames assay was valid and the test articles were **negative** under the conditions of this study.

The test system was exposed to the test article via the plate incorporation methodology described by Ames et al. (1975) and developed by N. Flamand et al. (2000). This methodology of Mini-Ames assay was developed from the standard Ames test and can provide a quick screen to evaluate the mutagenic potential of a test article using a relatively small amount of test article.

The revertant colony counts and cytotoxicity results were presented in **Table 14** for EXAMPLE 22.

No cytotoxicity was observed at any dose levels tested either in the presence or absence of S9 mix in each tester strain. No precipitate was observed at any dose levels tested.

EXAMPLE 22 did not induce ≥ 2-fold increases (for TA98, TA100 and WP2 *uvr*A (pKM101)) or ≥ 3-fold increases (for TA1535 and TA1537) at any dose levels tested in the mean number of revertant colonies compared to the concurrent negative/solvent control. No dose related increase of revertant colonies was observed either.

For the entire tester strains used, the negative/solvent control exhibited a characteristic number of spontaneous revertants per well. The positive controls induced the expected increase (three-fold or greater) in the mean number of revertant colonies when compared to the concurrent solvent control. Therefore, the performance of the solvent and positive controls was consistent with a valid assay.

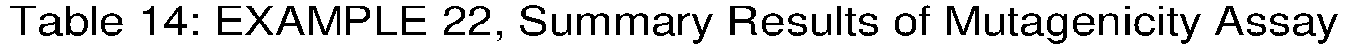

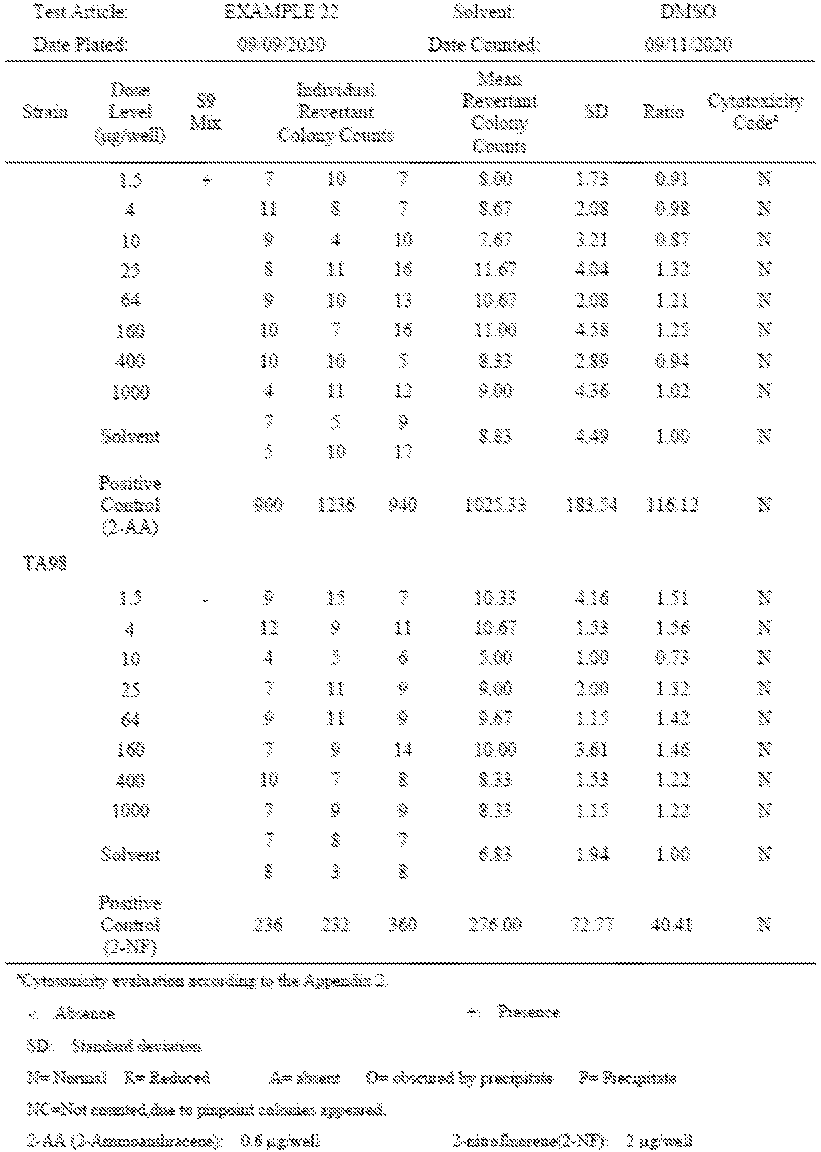

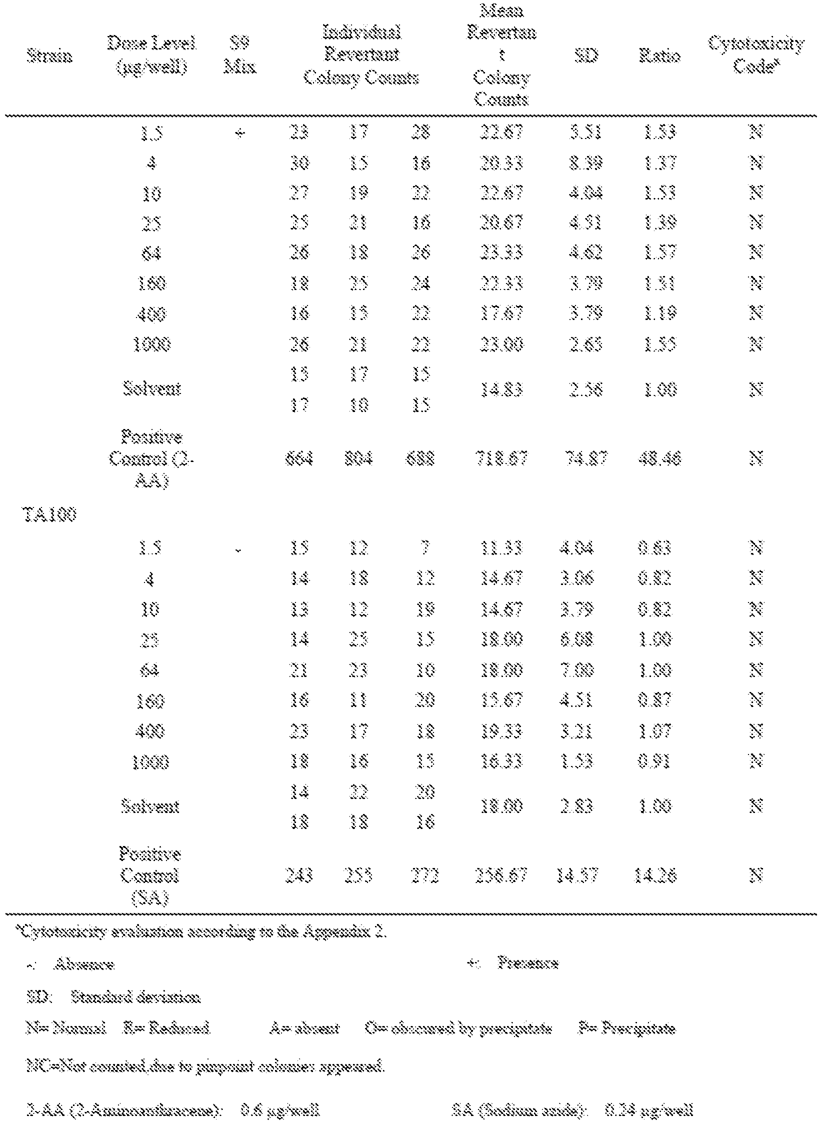

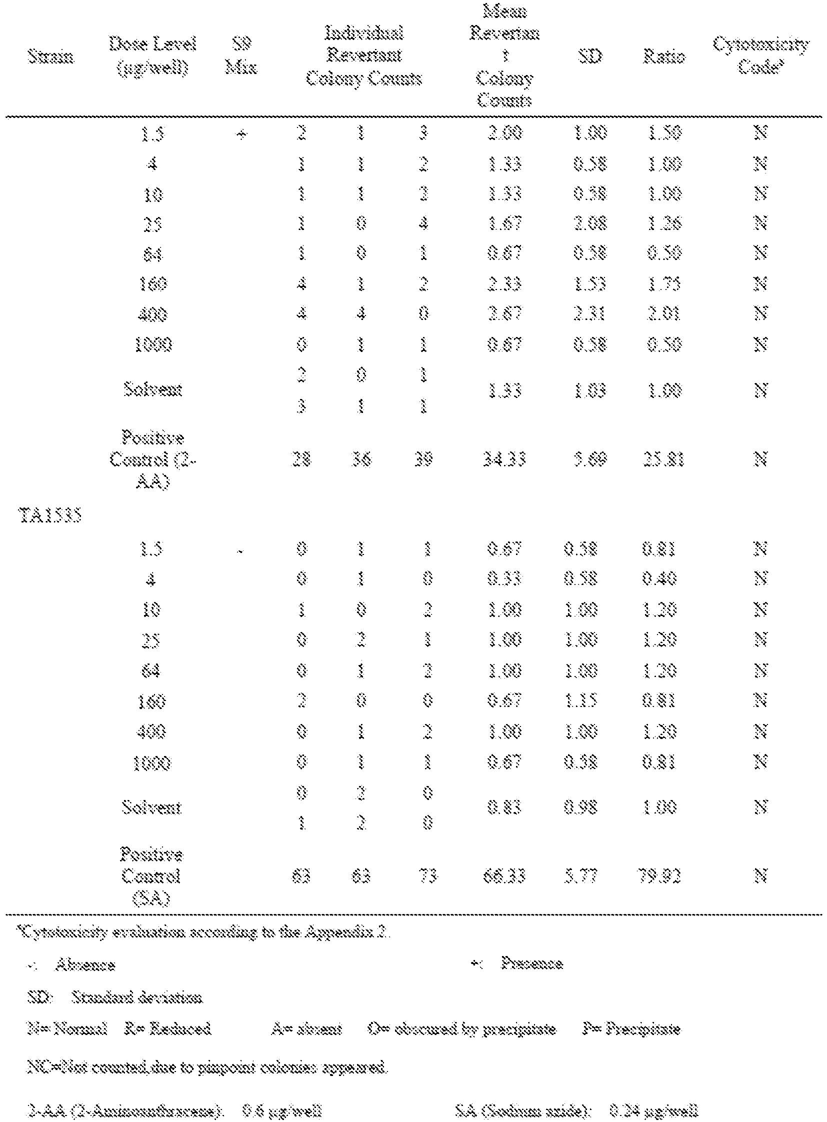

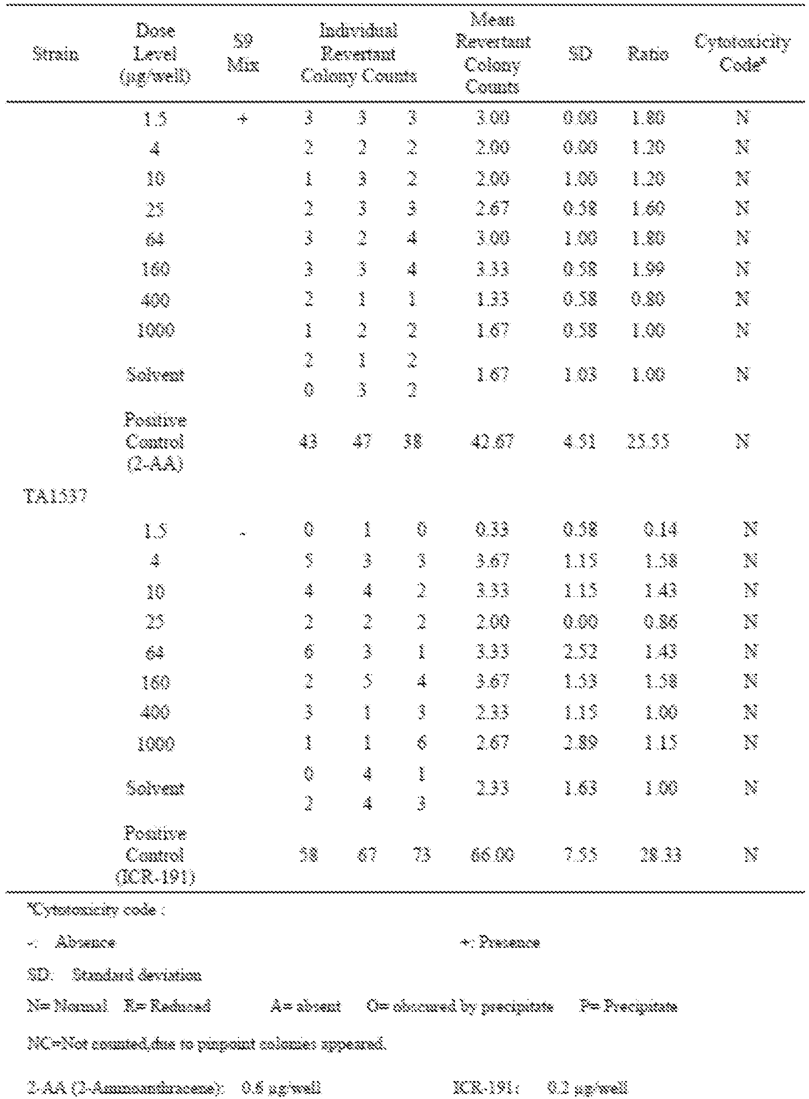

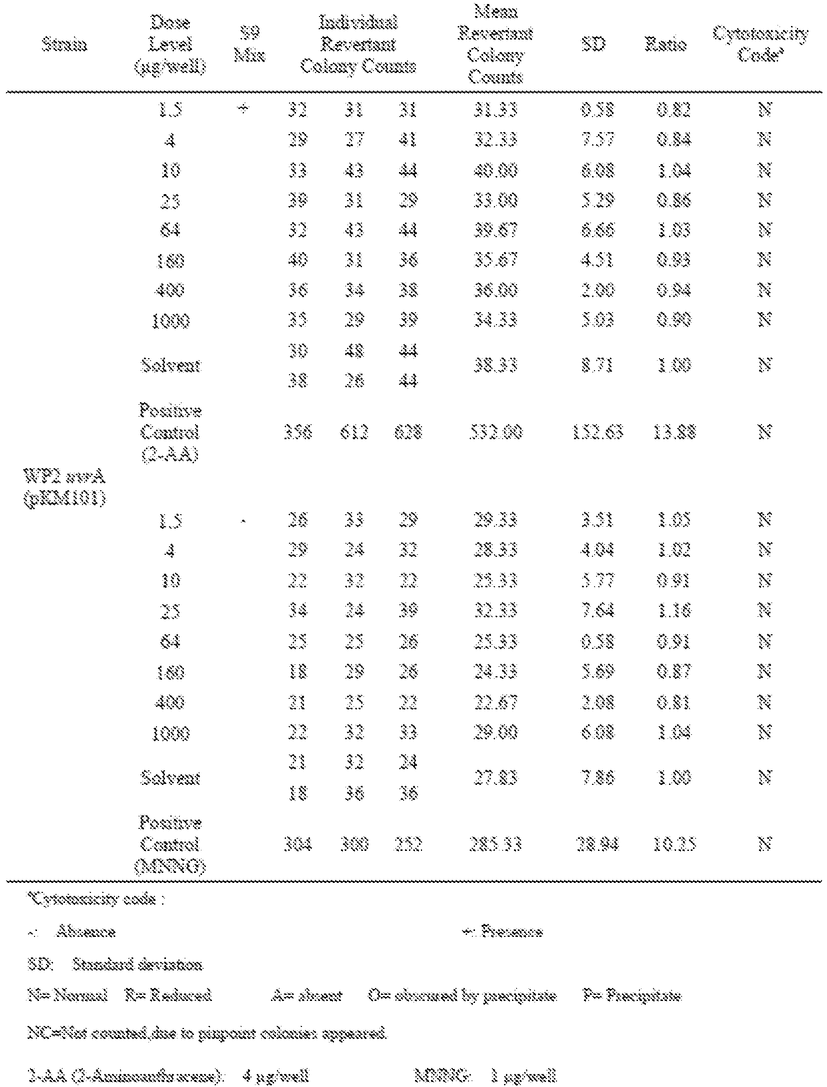

## Claims

1. A compound represented by the Formula I: 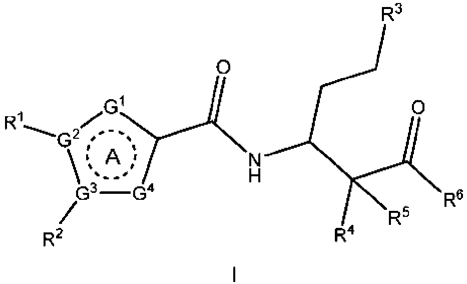
or a pharmaceutically acceptable salt thereof,
wherein
each of G¹, G², G³, and G⁴ is C, CH, or N, wherein:
(a) G¹ is CH, G² is C, G³ is N, and G⁴ is N;
(b) G¹ is N, G² is N, G³ is C, and G⁴ is CH; or
the dashed ring A is heteroaromatic;
R¹ is 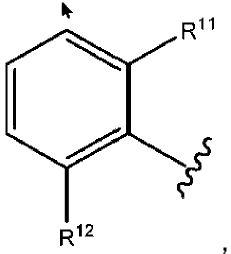 wherein
R¹¹ is halogen, C₁₋₆ alkoxy, or C₁₋₆ haloalkyl;
R¹² is H, halogen, C₁₋₆ alkoxy, or C₁₋₆ haloalkyl;
R² is thiazole, wherein R² may be optionally substituted with one or more R²¹, wherein each R²¹ is halogen, C₁₋₆ alkyl, C₁₋₆ alkoxy, or C₁₋₆ haloalkyl;
R³ is 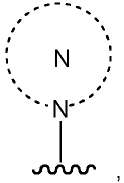 wherein
ring B is a 5- or 6-membered heterocyclic ring, optionally containing one or more degrees of unsaturation, and substituted with one or more R³¹,
wherein each R³¹ is halogen, C₁₋₆ alkyl, or oxo;
each of R⁴ and R⁵ is H, CH₃, or R⁴ and R⁵ combine with the atom to which they are attached to form a 3- to 6-membered cycloalkyl ring; and
R⁶ is OH or NH-R⁶¹, wherein R⁶¹ is a 3- to 6-membered cycloalkyl ring.

2. The compound of claim 1, wherein R¹¹ is C₁₋₆ alkoxy or C₁₋₆ haloalkyl.

3. The compound of claim 2, wherein R¹¹ is methoxy or trifluoromethyl.

4. The compound of one of claims 1 to 3, wherein R¹² is H, halogen, or C₁₋₆ alkoxy.

5. The compound of any one of claims 1 to 4, wherein R²¹ is substituted with halogen or C₁₋₆ alkyl.

6. The compound of any one of claims 1 to 5, wherein ring B is piperidine, pyrrolidine, or azetidine.

7. The compound of claim 1, wherein each R³¹ is halogen.

8. The compound of any one of claims 1 to 7, wherein each of R⁴ and R⁵ is H and R⁶ is OH.

9. The compound of any one of claims 1 to 8, wherein G¹ is CH, G² is C, G³ is N, and G⁴ is N.

10. The compound of any one of claims 1 to 9, wherein the compound is: 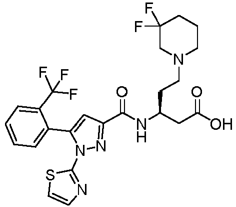 or a pharmaceutically acceptable salt thereof.

11. The compound of any one of claims 1 to 10 for use in a method of treating an apelin receptor related disorder, wherein the apelin receptor related disorder is selected from one or more of asthma, cardiomyopathy, diabetes, dyslipidemia, hypertension, inflammation, liver disease, metabolic disorder, neurodegenerative disease, obesity, preeclampsia, and renal dysfunction.

12. The compound of any one of claims 1 to 10 for use in a method for treating idiopathic pulmonary fibrosis in a patient in need thereof.

13. The compound of any one of claims 1 to 10 for use in a method for treating nephrotic syndrome in a patient in need thereof, optionally wherein:
the nephrotic syndrome is one or more of a glomerular disease or a chronic kidney disease.

14. The compound of any one of claims 1 to 10 for use in a method for promoting neovascularization through endothelial cell signaling or preservation of endothelial cell population in a patient in need thereof.

15. The compound for use in a method according to any one of claims 11 to 14, wherein the compound is used to treat one or more of asthma, chronic obstructive pulmonary disease (COPD), bronchitis, emphysema, pulmonary edema, acute respiratory disease syndrome (ARDS), interstitial lung disease, sarcoidosis, co-morbid pulmonary disorder, an autoimmune condition, rheumatoid arthritis, and scleroderma.

## Patentansprüche

1. Verbindung der Formel I: 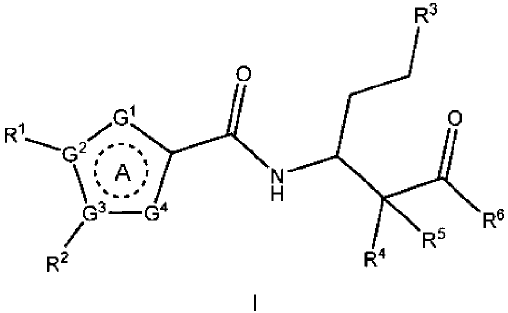
oder ein pharmazeutisch akzeptables Salz davon,
wobei
jedes von G¹, G², G³, und G⁴ C, CH oder N ist, wobei:
(a) G¹ CH, G² C, G³ N und G⁴ N ist;
(b) G¹ N, G² N, G³ C und G⁴ CH ist; oder
der gestrichelt dargestellte Ring A heteroaromatisch ist;
R¹ ist 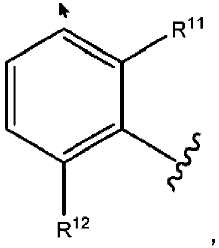 wobei
R¹¹ Halogen, C₁₋₆-Alkoxy oder C₁₋₆-Haloalkyl ist;
R¹² H, Halogen, C₁₋₆-Alkoxy oder C₁₋₆-Haloalkyl ist;
R² Thiazol ist, wobei R² optional mit einem oder mehreren R²¹ substituiert sein kann, wobei jedes R²¹ Halogen, C₁₋₆-Alkyl, C₁₋₆-Alkoxy oder C₁₋₆-Haloalkyl ist;
R³ ist 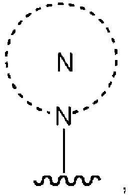
wobei
Ring B ein 5- oder 6-gliedriger heterocyclischer Ring ist, der optional einen oder mehrere Ungesättigungsgrade enthält und mit einem oder mehreren R³¹ substituiert ist,
wobei jedes R³¹ Halogen, C₁₋₆-Alkyl oder Oxo ist;
jedes von R⁴ und R⁵ H oder CH₃ ist oder R⁴ und R⁵ zusammen mit dem Atom, an das sie gebunden sind, einen 3- bis 6-gliedrigen Cycloalkylring bilden; und
R⁶ OH oder NH-R⁶¹ ist, wobei R⁶¹ ein 3- bis 6-gliedriger Cycloalkylring ist.

2. Verbindung nach Anspruch 1, wobei R¹¹ C₁₋₆-Alkoxy oder C₁₋₆-Haloalkyl ist.

3. Verbindung nach Anspruch 2, wobei R¹¹ Methoxy oder Trifluormethyl ist.

4. Verbindung nach einem der Ansprüche 1 bis 3, wobei R¹² H, Halogen oder C₁₋₆-Alkoxy ist.

5. Verbindung nach einem der Ansprüche 1 bis 4, wobei R²¹ mit Halogen oder C₁₋₆-Alkyl substituiert ist.

6. Verbindung nach einem der Ansprüche 1 bis 5, wobei Ring B Piperidin, Pyrrolidin oder Azetidin ist.

7. Verbindung nach Anspruch 1, wobei jedes R³¹ Halogen ist.

8. Verbindung nach einem der Ansprüche 1 bis 7, wobei R⁴ und R⁵ jeweils H sind und R⁶ OH ist.

9. Verbindung nach einem der Ansprüche 1 bis 8, wobei G¹ CH, G² C, G³ N und G⁴ N ist.

10. Verbindung nach einem der Ansprüche 1 bis 9, wobei die Verbindung die folgende Struktur aufweist: 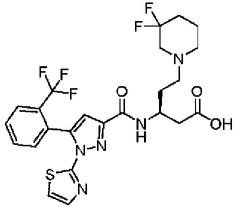 oder ein pharmazeutisch akzeptables Salz davon.

11. Verbindung nach einem der Ansprüche 1 bis 10 zur Verwendung in einem Verfahren zur Behandlung einer Apelinrezeptor-assoziierten Erkrankung, wobei die Apelinrezeptor-assoziierten Erkrankung ausgewählt ist aus einer oder mehreren von Asthma, Kardiomyopathie, Diabetes, Dyslipidämie, Bluthochdruck, Entzündung, Lebererkrankung, Stoffwechselstörung, neurodegenerativer Erkrankung, Adipositas, Präeklampsie und Nierenfunktionsstörung.

12. Verbindung nach einem der Ansprüche 1 bis 10 zur Verwendung in einem Verfahren zur Behandlung einer idiopathischen Lungenfibrose bei einem Patienten, der einer solchen Behandlung bedarf.

13. Verbindung nach einem der Ansprüche 1 bis 10 zur Verwendung in einem Verfahren zur Behandlung eines nephrotischen Syndroms der einer solchen Behandlung bedarf, optional wobei:
das nephrotische Syndrom eine oder mehrere von einer glomerulären Erkrankung oder einer chronischen Nierenerkrankung ist.

14. Verbindung nach einem der Ansprüche 1 bis 10 zur Verwendung in einem Verfahren zur Förderung der Neovaskularisation durch Endothelzell-Signalübertragung oder zur Erhaltung der Endothelzellpopulation bei einem Patienten, der einer solchen Behandlung bedarf.

15. Verbindung zur Verwendung in einem Verfahren nach einem der Ansprüche 11 bis 14, wobei die Verbindung zur Behandlung von einem oder mehreren von Asthma, chronisch obstruktiver Lungenerkrankung (COPD), Bronchitis, Emphysem, Lungenödem, akutem Atemnotsyndrom (ARDS), interstitieller Lungenerkrankung, Sarkoidose, komorbider Lungenerkrankung, einer Autoimmunerkrankung, rheumatoider Arthritis und Sklerodermie verwendet wird.

## Revendications

1. Composé représenté par la formule I : 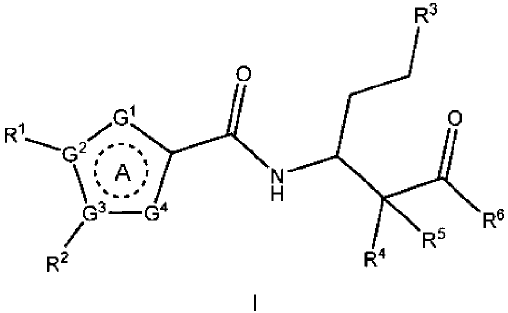
ou un sel pharmaceutiquement acceptable de celui-ci,
dans lequel
chacun parmi G¹, G², G³ et G⁴ est C, CH ou N, où :
(a) G¹ est CH, G² est C, G³ est N et G⁴ est N ;
(b) G¹ est N, G² est N, G³ est C et G⁴ est CH ; ou
le cycle A représenté par des traits pointillés est hétéroaromatique ;
R¹ est 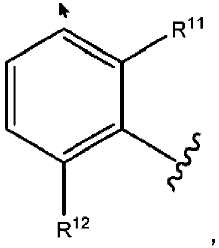 où
R¹¹ est un halogène, un alcoxy en C₁₋₆ ou un haloalkyle en C₁₋₆ ;
R¹² est H, un halogène, un alcoxy en C₁₋₆ ou un haloalkyle en C₁₋₆ ;
R² est un thiazole, où R² peut être éventuellement substitué par un ou plusieurs R²¹, où chaque R²¹ est un halogène, un alkyle en C₁₋₆, un alcoxy en C₁₋₆ ou un haloalkyle en C₁₋₆ ;
R³ est 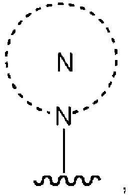
où
le cycle B est un cycle hétérocyclique à 5 ou 6 chaînons, pouvant éventuellement comporter un ou plusieurs degrés d'insaturation, et substitué par un ou plusieurs R³¹,
où chaque R³¹ est un halogène, un alkyle en C₁₋₆ ou un oxo ;
chacun parmi R⁴ et R⁵ est H ou CH₃, ou bien R⁴ et R⁵ se combinent avec l'atome auquel ils sont liés pour former un cycle cycloalkyle de 3 à 6 chaînons ; et
R⁶ est OH ou NH-R⁶¹, où R⁶¹ est un cycle cycloalkyle de 3 à 6 chaînons.

2. Composé selon la revendication 1, dans lequel R¹¹ est un alcoxy en C₁₋₆ ou un haloalkyle en C₁₋₆.

3. Composé selon la revendication 2, dans lequel R¹¹ est un méthoxy ou un trifluorométhyle.

4. Composé selon l'une des revendications 1 à 3, dans lequel R¹² est H, un halogène ou un alcoxy en C₁₋₆.

5. Composé selon l'une quelconque des revendications 1 à 4, dans lequel R²¹ est substitué par un halogène ou un alkyle en C₁₋₆.

6. Composé selon l'une quelconque des revendications 1 à 5, dans lequel le cycle B est une pipéridine, une pyrrolidine ou une azétidine.

7. Composé selon la revendication 1, dans lequel chaque R³¹ est un halogène.

8. Composé selon l'une quelconque des revendications 1 à 7, dans lequel chacun de R⁴ et R⁵ est H et R⁶ est OH.

9. Composé selon l'une quelconque des revendications 1 à 8, dans lequel G¹ est CH, G² est C, G³ est N et G⁴ est N.

10. Composé selon l'une quelconque des revendications 1 à 9, **caractérisé en ce que** ledit composé est : 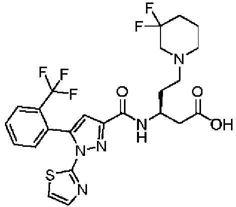 ou un sel pharmaceutiquement acceptable de celui-ci.

11. Composé selon l'une quelconque des revendications 1 à 10, destiné à être utilisé dans un procédé de traitement d'un trouble lié au récepteur de l'apéline, dans lequel le trouble lié au récepteur de l'apéline est choisi parmi un ou plusieurs des troubles suivants : asthme, cardiomyopathie, diabète, dyslipidémie, hypertension, inflammation, maladie hépatique, trouble métabolique, maladie neurodégénérative, obésité, prééclampsie et insuffisance rénale.

12. Composé selon l'une quelconque des revendications 1 à 10, destiné à être utilisé dans un procédé de traitement de la fibrose pulmonaire idiopathique chez un patient qui en a besoin.

13. Composé selon l'une quelconque des revendications 1 à 10, destiné à être utilisé dans un procédé de traitement du syndrome néphrotique chez un patient qui en a besoin, dans lequel, éventuellement :
le syndrome néphrotique est l'une ou plusieurs parmi une maladie glomérulaire ou une insuffisance rénale chronique.

14. Composé selon l'une quelconque des revendications 1 à 10, destiné à être utilisé dans un procédé visant à favoriser la néovascularisation par le biais de la signalisation des cellules endothéliales ou la préservation de la population de cellules endothéliales chez un patient qui en a besoin.

15. Composé destiné à être utilisé dans un procédé selon l'une quelconque des revendications 11 à 14, dans lequel le composé est utilisé pour traiter une ou plusieurs des affections suivantes : asthme, bronchopneumopathie chronique obstructive (BPCO), bronchite, emphysème, œdème pulmonaire, syndrome de détresse respiratoire aiguë (SDRA), pneumopathie interstitielle, sarcoïdose, trouble pulmonaire comorbide, affection auto-immune, polyarthrite rhumatoïde et sclérodermie.
